(19) **Europäisches Patentamt**

**European Patent Office**

**Office européen des brevets**

(11) **EP 1 132 405 A1**

(12) **EUROPEAN PATENT APPLICATION**
published in accordance with Art. 158(3) EPC

(43) Date of publication:
**12.09.2001 Bulletin 2001/37**

(21) Application number: **99972224.2**

(22) Date of filing: **11.11.1999**

(51) Int Cl.$^7$: **C07K 14/705**, C12N 15/12,
C12P 21/02, C07K 16/28,
A61K 39/395, G01N 33/50,
C07K 2/00, A61K 38/00

(86) International application number:
**PCT/JP99/06283**

(87) International publication number:
**WO 00/29441 (25.05.2000 Gazette 2000/21)**

(84) Designated Contracting States:
**AT BE CH CY DE DK ES FI FR GB GR IE IT LI LU
MC NL PT SE**
Designated Extension States:
**AL LT LV MK RO SI**

(30) Priority: **13.11.1998 JP 32375998**
**08.03.1999 JP 6003099**
**14.04.1999 JP 10681299**
**14.06.1999 JP 16667299**
**04.08.1999 JP 22164099**
**14.09.1999 JP 25981899**

(71) Applicant: **Takeda Chemical Industries, Ltd.
Osaka-shi, Osaka 541-0045 (JP)**

(72) Inventors:
• **WATANABE, Takuya
Osaka-shi, Osaka 532-0033 (JP)**
• **KIKUCHI, Kuniko
Toride-shi, Ibaraki 302-0024 (JP)**

• **TERAO, Yasuko
Tsukuba-shi, Ibaraki 305-0034 (JP)**
• **SHINTANI, Yasushi
Tsukuba-shi, Ibaraki 305-0821 (JP)**
• **HINUMA, Shuji
Tsukuba-shi, Ibaraki 305-0821 (JP)**
• **FUKUSUMI, Shoji
Tsukuba-shi, Ibaraki 305-0044 (JP)**
• **FUJII, Ryo
Tsukuba-shi, Ibaraki 305-0821 (JP)**
• **HOSOYA, Masaki
Tsuchiura-shi, Ibaraki 300-0007 (JP)**
• **KITADA, Chieko
Sakai-shi, Osaka 590-0073 (JP)**

(74) Representative: **Keller, Günter, Dr. et al
Lederer, Keller & Riederer
Patentanwälte
Prinzregentenstrasse 16
80538 München (DE)**

(54) **NOVEL G PROTEIN-COUPLED RECEPTOR PROTEIN, ITS DNA AND LIGAND THEREOF**

(57)    The present invention relates to a novel polypeptide, its partial peptide or salt thereof, a method for manufacturing the polypeptide, a receptor of the polypeptide, a pharmaceutical composition comprising the polypeptide or the like, an antibody to the polypeptide, a method/kit for screening a compound that promotes or inhibits an activity of the polypeptide or salt thereof, a compound that can be obtained by the screening, a pharmaceutical composition comprising the compound, and the like.

The polypeptide, its partial peptide or the like of the present invention can be used, for example, as a therapeutic agent for nervous disease, a promoting agent for somatostatin secretion and the like. Further, the antibody of the present invention can be used for a quantification of the polypeptide of the present invention in a sample solution. Furthermore, the polypeptide of the present invention is useful for a reagent for screening a compound that promotes or inhibits the activity of the polypeptide of the present invention.

**Description**

FIELD OF THE INVENTION

The present invention relates to a novel polypeptide (hereinafter sometimes referred to as a novel physiologically active polypeptide throughout the specification), its partial peptide, DNA encoding the same, as well as a receptor protein capable of recognizing the polypeptide as a ligand, its partial peptide and DNA encoding the same, and so on. In particular, the present invention relates to a novel polypeptide characterized by containing an RF amide-like structure and its partial peptide.

BACKGROUND ART

Peptides play pivotal roles as the molecules for regulating various functions in vivo such as metabolism, growth, reproduction, maintenance of homeostasis, mental activities, biological protection or the like. These peptides are coupled to specific receptors on the cell membrane to transduce their information. So far, most of these physiologically active peptides have been isolated from tissue extracts, etc. based on their physiological activities followed by determination of their structures. Using these receptors, physiologically active peptides have recently been isolated from tissue extracts, etc.

On the other hand, the latest rapid progress of sequencing of genome or cDNA has made accessible to enormous information on DNAs. It is assumed that these DNAs would comprise the DNA encoding for physiologically active peptides hitherto unknown. However, most physiologically active peptides have only very short amino acid sequences. Therefore, even if one attempts to explore, from genomic DNA sequences or expressed sequence tag (EST), such unknown physiologically active peptides bearing a sequence in part similar to or a common motif to known physiologically active peptides, desired sequences similar to these known peptides are frequently found only in protein genes not at all associated with physiologically active peptides or in DNA sequences of non-translational region. It was thus extremely difficult to ascertain which the objective physiologically active peptide is in these peptides.

FMRF amide, one of physiologically active peptides, is a peptide isolated from the ganglia of bivalve, which structure was determined for the first time (Price, D.A. & Greenberg, M.J., Science, 197 670-671, 1977). Since then it has turned out that peptides having an RF amide structure at the C terminus and peptides having a structure similar to the RF amide structure are present over many species of the invertebrate animal. Many peptides having the RF amide structure are reported to be present especially in the nematodes. It is also known that most of these peptides are borne on one gene in such a state that a plurality of peptides is contiguous (Nelson, L.S., et al., Molecular Brain Research, 58, 103-111, 1998).

Turning to the vertebrate animal, LPLRF amide was isolated from the brain of chicken and identified to be an FMRF amide-like peptide having the RF amide structure. However, its gene structure remains yet unknown (Dockray, G.J., et al., Nature, 305, 328-330, 1983). In fish, C-RFa was recently reported to be a peptide with the RF amide structure. As peptides containing the RF amide structure in mammal, there are known two peptides purified and isolated from bovine (Yang, H.-Y. T., et al., Proc. Natl. Acad. Sci. USA, 82, 7757-7761, 1985) and neuropeptide SF (NSF) and neuropeptide AF(NAF) isolated from human cDNA, which are considered to correspond to the two peptides above. Recently, the present inventors identified prolactin-releasing peptides (PrRP) containing the RF amide structure in human, bovine and rats (Hinuma, S., et al., Nature, 393, 272-276, 1998).

Various reports have been published on the physiological activities of the FMRF amide peptides, which include, for example, acceleration or suppression of heartbeats, contraction or relaxation of various radular muscle, visceral muscle and retractor muscle, and hyperpolarization or depolarization of nerve cells. With respect to PrRP and LPLRF amides, prolactin-releasing stimulation activity, and nerve cell-stimulating effects or hypertension effects are reported, respectively.

As stated above, many important physiological activities have been reported on the RF amide structure-bearing peptides. However, it is totally unknown if there is any other peptide containing the RF amide or the like structure in mammals, except NSF, NAF or PrRP.

On the other hand, a variety of physiologically active substances such as hormones, neurotransmitters, etc. regulate the functions in vivo through specific receptor proteins located in a cell membrane. Many of these receptor proteins are coupled with guanine nucleotide-binding protein (hereinafter sometimes referred to as G protein) and mediate the intracellular signal transduction via activation of G protein. These receptor proteins possess the common structure, i.e. seven transmembrane domains and are thus collectively referred to as G protein-coupled receptors or seven-transmembrane receptors (7TMR).

G protein-coupled receptor proteins present on the cell surface of each functional cells and organs in the body, and play important physiological roles as the targets of molecules that regulate the functions of the cells and organs, e.g., hormones, neurotransmitters, physiologically active substances and the like. Receptors transmit signals into cells via binding with physiologically active substances, and the signals induce various reactions such as activation and inhibition of the cells.

To clarify the relationship between substances that regulate complex biological functions in various cells and organs and their specific receptor proteins, in particular, G protein-coupled receptor proteins, would elucidate the functional mechanisms in various cells and organs in the body to provide a very important means for development of drugs closely associated with the functions.

For example, in various organs, their physiological functions are controlled in vivo through regulation by many hormones, hormone-like substances, neurotransmitters or physiologically active substances. In particular, physiologically active substances are found in numerous sites of the body and regulate the physiological functions through their corresponding receptor proteins. However, it is supposed that many unknown hormones, neurotransmitters or other physiologically active substances still exist in the body and, as for their receptor proteins, many of such proteins have not yet been reported. In addition, it is still unknown if there are subtypes of known receptor proteins.

It is also very important for development of drugs to clarify the relationship between substances that regulate elaborate functions in vivo and their specific receptor proteins. Furthermore, for efficient screening of agonists and antagonists to receptor proteins in development of drugs, it is required to clarify functional mechanisms of receptor protein genes expressed in vivo and express the genes in an appropriate expression system.

In recent years, random analysis of cDNA sequences has been actively studied as a means for analyzing genes expressed in vivo. The sequences of cDNA fragments thus obtained have been registered on and published to databases as Expressed Sequence Tag (EST). However, since many ESTs contain sequence information only, it is difficult to deduce their functions from the information.

It has thus been desired to find an unknown polypeptide (peptide) having RF amide-like structure or an unknown G protein-coupled receptor protein and using these peptides to develop a drug for the prevention, treatment or diagnosis for disease, comprising a novel physiologically active peptide.

DISCLOSURE OF THE INVENTION

In order to solve the foregoing problems, the present inventors have made extensive studies and as a result, succeeded in preparing primers based on the sequence information such as EST and cloning cDNA having a novel base sequence by RT-PCR using poly(A)$^+$ RNA of human fetal brain as a template. The present inventors have thus found that polypeptides encoded by the thus obtained cDNA are useful peptides in which the C terminal structure is LPL RF amide-, LPL RS amide-, LPQ RF amide- or LPLRL amide-like.

Based on the EST information prepared by the degenerated PCR technique, the present inventors have succeeded in isolating cDNAs encoding novel G protein-coupled receptor proteins derived from rat cerebellum and from human hypothalamus and in sequencing their full base sequences. When the base sequences were translated into the amino acid sequences, 1 to 7 transmembrane domains were found to be on the hydrophobic plot, verifying that the proteins encoded by these cDNAs are seven-transmembrane type G protein-coupled receptor proteins.

The present inventors have made further extensive investigations and found that the RF amide-like polypeptides show a ligand activity for the G protein-coupled receptor proteins.

Based on these findings, the present inventors have continued extensive studies and as a result, have come to accomplish the present invention.

Thus, the present invention relates to the following features.

(1) A polypeptide containing the same or substantially the same amino acid sequence as that represented by SEQ ID NO:1, its amide or ester, or a salt thereof.

(2) A polypeptide or its amide or ester, or a salt thereof, according to (1), wherein substantially the same amino acid sequence is represented by SEQ ID NO:8, SEQ ID NO:14, SEQ ID NO:18, SEQ ID NO:33 or SEQ ID NO:50.

(3) A partial peptide of the polypeptide according to (1), or its amide or ester, or a salt thereof.

(4) A partial peptide or its amide or ester, or a salt thereof, according to (3), comprising amino acid residues 81 (Met) to 92 (Phe) of SEQ ID NO:1.

(5) A partial peptide or its amide or ester, or a salt thereof, according to (3), comprising amino acid residues 101 (Ser) to 112 (Ser) of SEQ ID NO:1.

(6) A partial peptide or its amide or ester, or a salt thereof, according to (3), comprising amino acid residues 124 (Val) to 131 (Phe) of SEQ ID NO:1.

(7) An amide of the partial peptide of the polypeptide according to (1), or a salt thereof.

(8) A DNA containing a DNA bearing a base sequence encoding the polypeptide according to (1).

(9) A DNA according to (8) having a base sequence represented by SEQ ID NO:2, SEQ ID NO:9, SEQ ID NO:15, SEQ ID NO:19, SEQ ID NO:34 or SEQ ID NO:51.

(10) A DNA containing a DNA encoding the partial peptide according to (3).

(11) A DNA according to (10), comprising bases 241 to 276 of the base sequence represented by SEQ ID NO:2.

(12) A DNA according to (10), comprising bases 301 to 336 of the base sequence represented by SEQ ID NO:2.

(13) A DNA according to (10), comprising bases 370 to 393 of the base sequence represented by SEQ ID NO:2.

(14) A recombinant vector containing the DNA according to (8) or (10).

(15) A transformant transformed with the recombinant vector according to (14).

(16) A method for manufacturing the polypeptide or its amide or ester, or a salt thereof, according to (1) or the partial peptide or its amide or ester, or a salt thereof, according to (3), which comprises culturing said transformant according to (15) and producing and accumulating the polypeptide according to (1) or the partial peptide according to (3).

(17) An antibody to the polypeptide or its amide or ester, or a salt thereof, according to (1) or the partial peptide or its amide or ester, or a salt thereof according to (3).

(18) A diagnostic composition comprising the DNA according to (8) or (10) or the antibody according to (17).

(19) An antisense DNA having a complementary or substantially complementary base sequence to the DNA according to (8) or (10) and capable of suppressing expression of said DNA.

(20) A composition comprising the polypeptide or its amide or ester, or a salt thereof, according to (1) or the partial peptide, or its amide or ester, or a salt thereof, according to (3).

(21) A pharmaceutical composition comprising the polypeptide or its amide or ester, or a salt thereof, according to (1) or the partial peptide or its amide or ester, or a salt thereof, according to (3).

(22) A method for screening a compound that accelerates or inhibits the activity of the polypeptide or its amide or ester, or a salt thereof, according to (1) or the partial peptide or its amide or ester, or a salt thereof, according to (3), which comprises using the polypeptide or its amide or ester, or a salt thereof, according to (1) or the partial peptide or its amide or ester, or a salt thereof, according to (3).

(23) A method for screening according to (22), wherein the polypeptide or its amide or ester, or a salt thereof, according to (1) or the partial peptide or its amide or ester, or a salt thereof, according to (3) and a protein containing the same or substantially the same amino acid sequence as that represented by SEQ ID NO:37, or a salt thereof, or the partial peptide or its amide or ester, or a salt thereof are used. (24) A kit for screening a compound that accelerates or inhibits the activity of the polypeptide or its amide or ester, or a salt thereof, according to (1) or the partial peptide or its amide or ester, or a salt thereof, according to (3), comprising the polypeptide or its amide or ester, or a salt thereof, according to (1) or the partial peptide or its amide or ester, or a salt thereof, according to (3).

(25) A kit for screening according to (24), comprising the polypeptide or its amide or ester, or a salt thereof, according to (1) or the partial peptide or its amide or ester, or a salt thereof, according to (3) and a protein containing the same or substantially the same amino acid sequence as that represented by SEQ ID NO:37 or the partial peptide or its amide or ester, or a salt thereof.

(26) A compound that accelerates or inhibits the polypeptide, or its amide or ester, or a salt thereof, according to (1) or the partial peptide, or its amide or ester, or a salt thereof, according to (3), which is obtainable using the screening method according to (22) or the screening kit according to (24).

(27) A pharmaceutical composition comprising a compound that accelerates or inhibits the polypeptide, or its amide or ester, or a salt thereof, according to (1) or the partial peptide, or its amide or ester, or a salt thereof, according to (3), which is obtainable using the screening method according to (22) or the screening kit according to (24).

(28) A protein or a salt thereof containing the same or substantially the same amino acid sequence as the amino acid sequence represented by SEQ ID NO:37.

(29) A protein or its salt according to (28), wherein substantially the same amino acid sequence as the amino acid sequence represented by SEQ ID NO:37 is the amino acid sequence represented by SEQ ID NO:54.

(30) A partial peptide or its amide or ester, or a salt thereof, according to (28).

(31) A DNA containing a DNA having a base sequence encoding the protein according to (28) or the partial peptide according to (30).

(32) A DNA according to (31) having the base sequence represented by SEQ ID NO:38, SEQ ID NO:55 or SEQ ID NO:56.

(33) A recombinant vector containing the DNA according to (31) .

(34) A transformant transformed with the recombinant vector according to (33).

(35) A method for manufacturing the protein or its salt according to (28) or the partial peptide or its amide or ester, or a salt thereof, according to (30), which comprises culturing the transformant according to (34) and producing and accumulating the protein according to (28) or the partial peptide according to (30) .

(36) An antibody to the protein or its salt according to (28) or the partial peptide or its amide or ester, or a salt thereof, according to (30).

(37) A diagnostic composition comprising the DNA according to (31) or the antibody according to (36).

(38) A ligand to the protein or its salt according to (28), which is obtainable by using the protein or its salt according to (28) or the partial peptide or its amide or ester or, a salt thereof, according to (30).

(39) A method for determination of a ligand to the protein or its salt according to (28), characterized by using the protein or its salt according to (28) or the partial peptide or its amide or ester, or a salt thereof, according to (30).

(40) A method for screening a compound that alters the binding property between a ligand and the protein or its salt according to (28), which comprises using the protein or its salt according to (28) or the partial peptide or its amide or ester, or a salt thereof, according to (30).

(41) A kit for screening a compound that alters the binding property between a ligand and the protein or its salt according to (28), comprising the protein or its salt according to (28) or the partial peptide or its amide or ester, or a salt thereof, according to (30).

(42) A compound that alters the binding property between a ligand and the protein or its salt according to (28), which is obtainable by using the screening method according to (40) or the screening kit according to (41).

(43) A pharmaceutical composition comprising a compound that alters the binding property between a ligand and the protein or its salt according to (28), which is obtainable by using the screening method according to (40) or the screening kit according to (41).

(44) A method for quantifying the protein or its salt according to (28), which comprises using the antibody of (36).

The present invention further relates to the following:

(45) A polypeptide, its amide or ester, or a salt thereof, according to (1), wherein substantially the same amino acid sequence as the amino acid sequence shown by SEQ ID NO:1 is an amino acid sequence possessing homology of at least about 70%, preferably at least about 80%, more preferably at least about 90% and most preferably about 95%, to the amino acid sequence shown by SEQ ID NO:1.

(46) A polypeptide, its amide or ester, or a salt thereof, according to (1), wherein substantially the same amino acid sequence as the amino acid sequence shown by SEQ ID NO:1 is (i) an amino acid sequence represented by SEQ ID NO:1, SEQ ID NO:8, SEQ ID NO:14, SEQ ID NO:18, SEQ ID NO:33 or SEQ ID NO:50, of which 1 to 20 (preferably 1 to 15, more preferably 1 to 5 and most preferably 1 to 3) amino acids are deleted; (ii) an amino acid sequence represented by SEQ ID NO:1, SEQ ID NO:8, SEQ ID NO:14, SEQ ID NO:18, SEQ ID NO:33 or SEQ ID NO:50, to which 1 to 20 (preferably 1 to 15, more preferably 1 to 5 and most preferably 1 to 3) amino acids are added; (iii) an amino acid sequence represented by SEQ ID NO:1, SEQ ID NO:8, SEQ ID NO:14, SEQ ID NO:18, SEQ ID NO:33 or SEQ ID NO:50, into which 1 to 20 (preferably 1 to 15, more preferably 1 to 5 and most preferably 1 to 3) amino acids are inserted, (iv) an amino acid sequence represented by SEQ ID NO:1, SEQ ID NO:8, SEQ ID NO:14, SEQ ID NO:18, SEQ ID NO:33 or SEQ ID NO:50, in which 1 to 20 (preferably 1 to 15, more preferably 1 to 5 and most preferably 1 to 3) amino acids are substituted by other amino acids; and (v) a combination of the above amino acid sequences.

(47) DNA containing DNA having a base sequence which is hybridizable with the base sequence encoding DNA according to (8) or (10) under highly stringent conditions.

(48) A recombinant vector containing DNA according to (47).

(49) A transformant transformed with the recombinant vector according to (48).

(50) A method for manufacturing the polypeptide, it amide or ester, or a salt thereof, encoded by DNA according to (47), which comprises culturing the transformant according to (49), producing and accumulating the polypeptide encoded by DNA according to (47) and harvesting the polypeptide.

(51) A polypeptide, its amide or ester, or a salt thereof, encoded by DNA according to (47), which is manufactured by the method according to (50).

(52) A protein or its salt according to (28), wherein substantially the same amino acid sequence as the amino acid sequence shown by SEQ ID NO:37 is an amino acid sequence possessing homology of at least about 50%, preferably at least about 70%, more preferably at least about 80%, further more preferably at least about 90% and most preferably about 95%, to the amino acid sequence shown by SEQ ID NO:37.

(53) A protein or its salt according to (28), wherein substantially the same amino acid sequence as the amino acid sequence shown by SEQ ID NO:37 is (i) an amino acid sequence represented by SEQ ID NO:37, of which 1 to 20 (preferably 1 to 15, more preferably 1 to 5 and most preferably 1 to 3) amino acids are deleted; (ii) an amino acid sequence represented by SEQ ID NO:37, to which 1 to 20 (preferably 1 to 15, more preferably 1 to 5 and most preferably 1 to 3) amino acids are added; (iii) an amino acid sequence represented by SEQ ID NO:37, in which 1 to 20 (preferably 1 to 15, more preferably 1 to 5 and most preferably 1 to 3) amino acids are substituted by other amino acids; or (iv) a combination of the above amino acid sequences.

(54) DNA containing DNA having a base sequence which is hybridizable with the base sequence encoding DNA according to (31) under highly stringent conditions.

(55) A recombinant vector containing DNA according to (54).

(56) A transformant transformed with the recombinant vector according to (55).

(57) A method for manufacturing the polypeptide, it amide or ester, or a salt thereof, encoded by DNA according to (54), which comprises culturing the transformant according to (56), producing and accumulating the polypeptide encoded by DNA according to (54) and harvesting the polypeptide.

(58) A polypeptide, its amide or ester, or a salt thereof, encoded by DNA according to (54), which is manufactured by the method according to (57).

(59) A method for screening according to (22), which comprises measuring and comparing (i) the activity of the polypeptide, its amide or ester, or a salt thereof, according to (1), or the partial peptide, its amide or ester, or a salt thereof, according to (3), where its receptor is brought in contact with the polypeptide, its amide or ester, or a salt thereof, according to (1) or the partial peptide, its amide or ester, or a salt thereof, according to (3) and (ii) the activity of the polypeptide, its amide or ester, or a salt thereof; according to (1) or the partial peptide, its amide or ester, or a salt thereof, according to (3), where its receptor and a test compound are brought in contact with polypeptide, its amide or ester, or a salt thereof, according to (1) or the partial peptide, its amide or ester, or a salt thereof, according to (3).

(60) A method for screening according to (59), wherein the receptor is a protein containing the same or substantially the same amino acid sequence as the amino acid sequence shown by SEQ ID NO:37, or a salt thereof, or its partial peptide, its amide or ester or a salt thereof.

(61) A pharmaceutical composition comprising a compound that accelerates the activity of the polypeptide or its amide or ester, or a salt thereof, according to (1) or the partial peptide or its amide or ester, or a salt thereof, according to (3), which is obtainable using the screening method according to (22) or the screening kit according to (24).

(62) A pharmaceutical composition comprising a compound that inhibits the activity of the polypeptide or its amide or ester, or a salt thereof, according to (1) or the partial peptide or its amide or ester, or a salt thereof, according to (3), which is obtainable using the screening method according to (22) or the screening kit according to (24).

(63) A method for quantifying the polypeptide, its amide or ester, or a salt thereof, according to (1) or the partial peptide, its amide or ester, or a salt thereof, according to (3) in a test sample fluid, which comprises competitively reacting the antibody according to (17) with a test sample fluid and the polypeptide, its amide or ester, or a salt thereof, according to (1) or the partial peptide, its amide or ester, or a salt thereof, according to (3), which is labeled, and measuring the ratio of the labeled polypeptide, amide or ester or salt according to (1) or the labeled partial peptide, amide or ester or salt according to (3) in the test sample fluid.

(64) A method for quantifying the polypeptide, its amide or ester or, a salt thereof, according to (1) or the partial peptide, its amide or ester or, a salt thereof, according to (3) in a test sample fluid, which comprises reacting a test sample fluid simultaneously or sequentially with the antibody of (17) immobilized on a carrier and labeled antibody of (17) and then measuring the activity of a labeling agent on the immobilized carrier.

(65) A method for quantifying the protein or its salt according to (28) or the partial peptide, its amide or ester, or a salt thereof, according to (30) in a test sample fluid, which comprises competitively reacting the antibody according to (36) with a test sample fluid and the protein or its salt according to (28) or the partial peptide, its amide or ester, or a salt thereof, according to (30), which is labeled, and measuring the ratio of the antibody-bound labeled protein, amide or ester or salt according to (1) or the antibody-bound labeled partial peptide, amide or ester or salt according to (30) in the test sample fluid. And,

(66) A method for quantifying the polypeptide, its amide or ester, or a salt thereof, according to (28) or the partial peptide, its amide or ester, or a salt thereof, according to (30) in a test sample fluid, which comprises reacting a test sample fluid simultaneously or sequentially with the antibody of (36) immobilized on a carrier and labeled antibody of (36) and then measuring the activity of a labeling agent on the immobilized carrier.

BRIEF DESCRIPTION OF THE DRAWINGS

FIG. 1 shows the base sequence of DNA encoding the polypeptide (human type) of the present invention obtained in Example 2, and the amino acid sequence deduced from the base sequence.

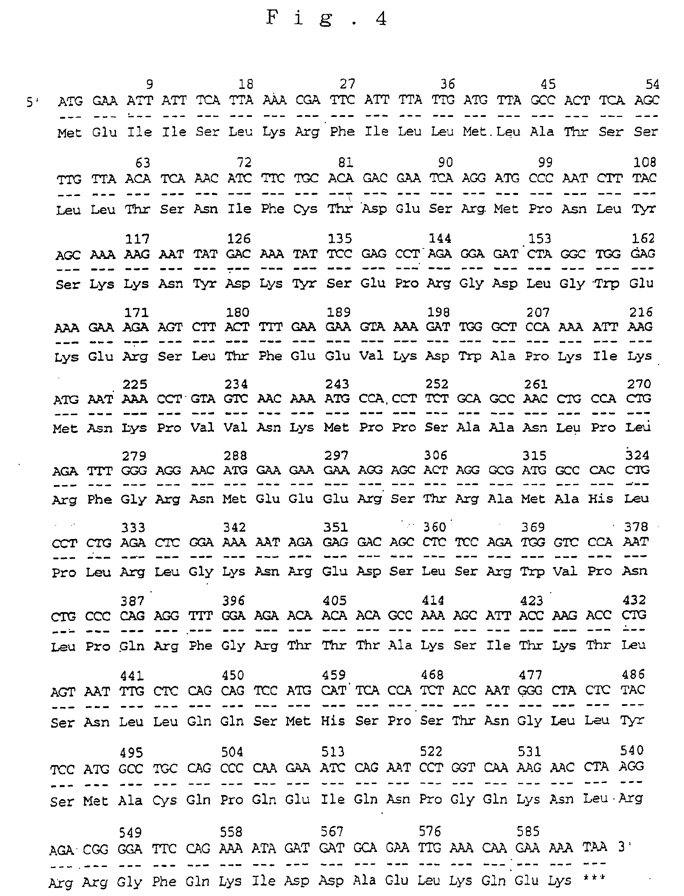

FIG. 2 shows the hydrophobic plotting of the polypeptide of the present invention.

FIG. 3 shows the base sequence of DNA encoding the polypeptide (human type) of the present invention obtained in Example 3, and the amino acid sequence deduced from the base sequence.

FIG. 4 shows the base sequence of DNA encoding the polypeptide (bovine type) of the present invention obtained in Example 4, and the amino acid sequence deduced from the base sequence.

FIG. 5 shows the base sequence of DNA encoding the polypeptide (rat type) of the present invention obtained in Example 5, and the amino acid sequence deduced from the base sequence.

FIG. 6 shows comparison of the amino acid sequences of the polypeptides of the present invention obtained in Examples 3, 4 and 5.

FIG. 7 shows the base sequence of DNA encoding the polypeptide (mouse type) of the present invention obtained in Example 6, and the amino acid sequence deduced from the base sequence.

FIG. 8 shows the reactivity of peptides to r0T7T022L receptor-expressed CHO cells, assayed by Cytosensor in Example 7, in which ●-● and Δ-Δ denote MPHSFANLPLRF amide (SEQ ID NO:39) and VPNLPQRF amide (SEQ ID NO:40), respectively.

FIG. 9 shows the activity of MPHSFANLPLRF amide (SEQ ID NO:39) and VPNLPQRF amide (SEQ ID NO: 40) for suppressing cAMP production against the r0T7T022L-expressed CHO cells,- assayed in Example 10, in which □-□ and ●-● denote MPHSFANLPLRF amide (SEQ ID NO:39) and VPNLPQRF amide (SEQ ID NO:40), respectively.

BEST MODE OF EMBODIMENT OF THE INVENTION

The polypeptide of the present invention having the same or substantially the same amino acid sequence as the amino acid sequence shown by SEQ ID NO:1 (hereinafter referred to as the polypeptide of the present invention) may be any polypeptide derived from any cells of human and other warm-blooded animals (e.g. guinea pig, rat, mouse, chicken, rabbit, swine, sheep, bovine, monkey, etc.) such as retina cell, liver cell, splenocyte, nerve cell, glial cell, β cell of pancreas, bone marrow cell, mesangial cell, Langerhans' cell, epidermic cell, epithelial cell, endothelial cell, fibroblast, fibrocyte, myocyte, fat cell, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte, interstitial cell, etc., the corresponding precursor cells, stem cells, cancer cells, etc., or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; polypeptides derived from hemocyte type cells or their cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the polypeptides may also be synthetic polypeptides.

The amino acid sequence which has substantially the same amino acid sequence as that represented by SEQ ID NO:1 includes an amino acid sequence having at least about 70% homology, preferably at least about 80% homology, more preferably at least about 90% homology, and most preferably at least about 95% homology, to the amino acid sequence represented by SEQ ID NO:1.

Examples of the polypeptide which has substantially the same amino acid sequence as that shown by SEQ ID NO:1 include a polypeptide containing the 22-180 amino acid sequence of the amino acid sequence represented by SEQ ID NO:1, etc.

Preferred examples of the polypeptide which has substantially the same amino acid sequence as that represented by SEQ ID NO:1 include a polypeptide having substantially the same amino acid sequence as that represented by SEQ ID NO:1 (e.g., amino acid sequence shown by SEQ ID NO:8, SEQ ID NO:14, SEQ ID NO:18, SEQ ID NO:33 or SEQ ID NO:50) and having the activity substantially equivalent to that of the amino acid sequence represented by SEQ ID NO:1.

Examples of the substantially equivalent activity include a cell-stimulating activity caused by adding such a polypeptide (specifically, a protein containing the same or substantially the same amino acid sequence as that shown by SEQ ID NO:37, or its salts) to the polypeptide receptor-expressing cells (hereinafter simply referred to as a cell stimulating activity) such as arachidonic acid release, acetylcholine release, intracellular $Ca^{2+}$ release, intracellular CAMP formation, intracellular cGMP formation, inositol phosphate production, change in cell membrane potential, phosphorylation of intracellular proteins, activation of c-fos and change in extracellular pH, etc.), a somatostatin secretion regulating activity and the like.

The term "substantially equivalent" is used to mean that the nature of these activities is equivalent (for example, biochemically or pharmacologically). Therefore, it is preferred that the these activities such as a cell-stimulating activity, a somatostatin secretion regulating activity, etc. are equivalent in strength (e.g., about 0.1 to about 100 times, preferably about 0.5 to about 10 times, more preferably about 0.5 to about 2 times), and it is allowable that even differences among grades such as the strength of these activities and molecular weight of the polypeptide are present.

The activities such as a cell stimulating activity or the like can be determined according to a publicly known method, for example, by means of screening which will be later described.

The polypeptides of the present invention include so-called muteins such as polypeptides comprising (i) an amino acid sequence represented by SEQ ID NO:1, SEQ ID NO:8, SEQ ID NO:14, SEQ ID NO:18, SEQ ID NO:33 or SEQ ID NO:50, of which 1 to 20 (preferably 1 to 15, more preferably 1 to 5 and most preferably 1 to 3) amino acids are deleted; (ii) an amino acid sequence represented by SEQ ID NO:1, SEQ ID NO:8, SEQ ID NO:14, SEQ ID NO:18, SEQ ID NO:33 or SEQ ID NO:50, to which 1 to 20 (preferably 1 to 15, more preferably 1 to 5 and most preferably 1 to 3) amino acids are added; (iii) an amino acid sequence represented by SEQ ID NO:1, SEQ ID NO:8, SEQ ID NO:14, SEQ ID NO:18, SEQ ID NO:33 or SEQ ID NO:50, into which 1 to 20 (preferably 1 to 15, more preferably 1 to 5 and most preferably 1 to 3) amino acids are inserted, (iv) an amino acid sequence represented by SEQ ID NO:1, SEQ ID NO:8, SEQ ID NO:14, SEQ ID NO:18, SEQ ID NO:33 or SEQ ID NO:50, in which 1 to 20 (preferably 1 to 15, more preferably 1 to 5 and most preferably 1 to 3) amino acids are substituted by other amino acids; and (v) a combination of the above amino acid sequences.

When an amino acid sequence(s) are inserted, deleted or substituted as described above, the positions of such insertion, deletion or substitution are not particularly limited.

Specific examples of the polypeptide which contains substantially the same amino acid sequence as that shown by SEQ ID NO:1 are a polypeptide containing substantially the same amino acid sequence as that shown by SEQ ID NO:8, a polypeptide containing substantially the same amino acid sequence as that shown by SEQ ID NO: 14, a polypeptide containing substantially the same amino acid sequence as that shown by SEQ ID NO:18, a polypeptide containing substantially the same amino acid sequence as that shown by SEQ ID NO:33, a polypeptide containing substantially the same amino acid sequence as that shown by SEQ ID NO:50 and so on.

Throughout the present specification, the polypeptides are represented in accordance with the conventional way of describing polypeptides, that is, the N-terminus (amino terminus) at the left hand and the C-terminus (carboxyl terminus) at the right hand. In the polypeptides of the present invention including the polypeptide containing the amino acid sequence shown by SEQ ID NO:1, the C-terminus is usually in the form of a carboxyl group (-COOH) or a carboxylate (-COO$^-$) but may be in the form of an amide (-CONH$_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl such as a phenyl-$C_{1-2}$ alkyl group, e.g., benzyl, phenethyl, etc.; an $\alpha$-naphthyl-$C_{1-2}$ alkyl group such as $\alpha$-naphthylmethyl, etc.; and the like. In addition, pivaloyloxymethyl or the like which is used widely as an ester for oral administration may also be used.

Where the polypeptide of the present invention contains a carboxyl group (or a carboxylate) at a position other than the C-terminus, it may be amidated or esterified and such an amide or ester is also included within the polypeptide of the present invention. The ester group may be the same group as that described with respect to the above C-terminal.

Furthermore, examples of the polypeptide of the present invention include variants of the above polypeptides, wherein the amino group at the N-terminus (e.g., methionine residue) of the polypeptide is protected with a protecting group (e.g., a $C_{1-6}$ acyl group such as a $C_{1-6}$ alkanoyl group e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains.

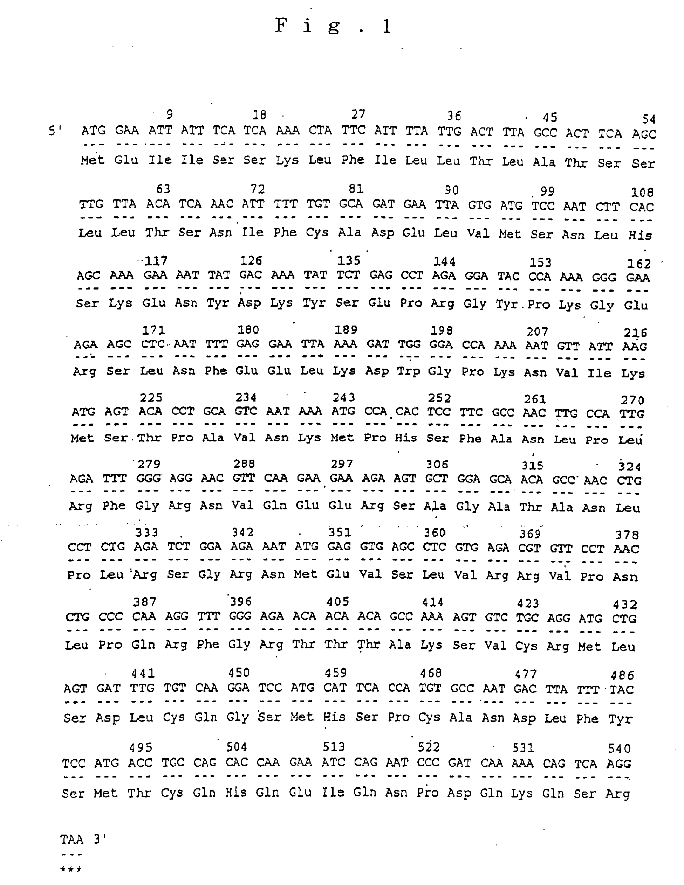

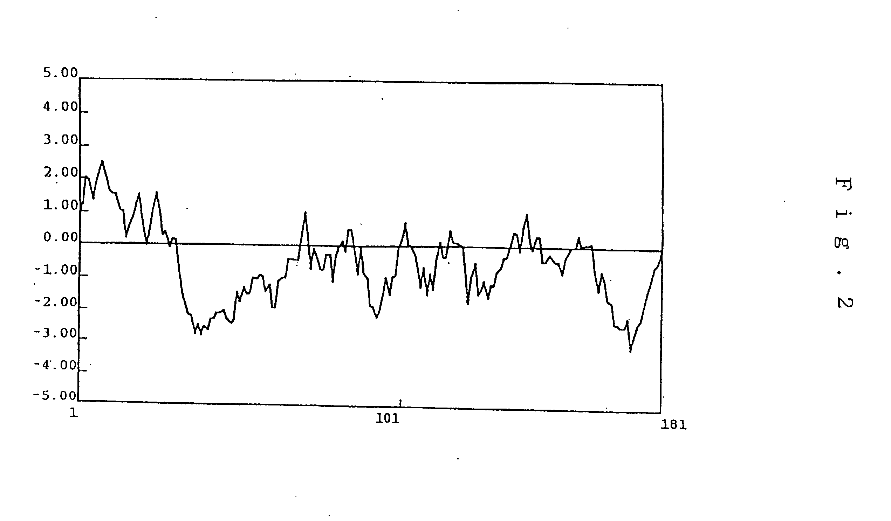

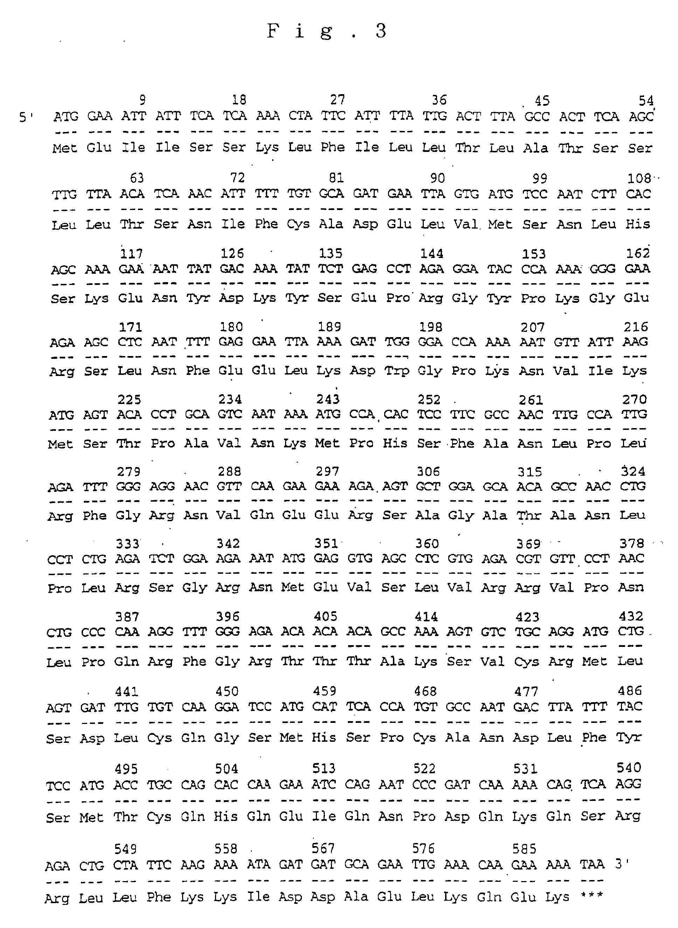

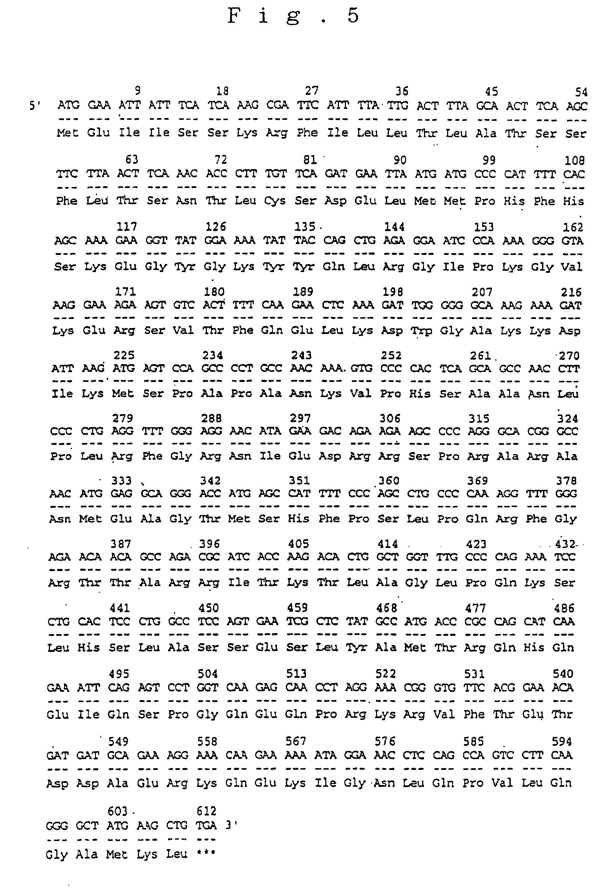

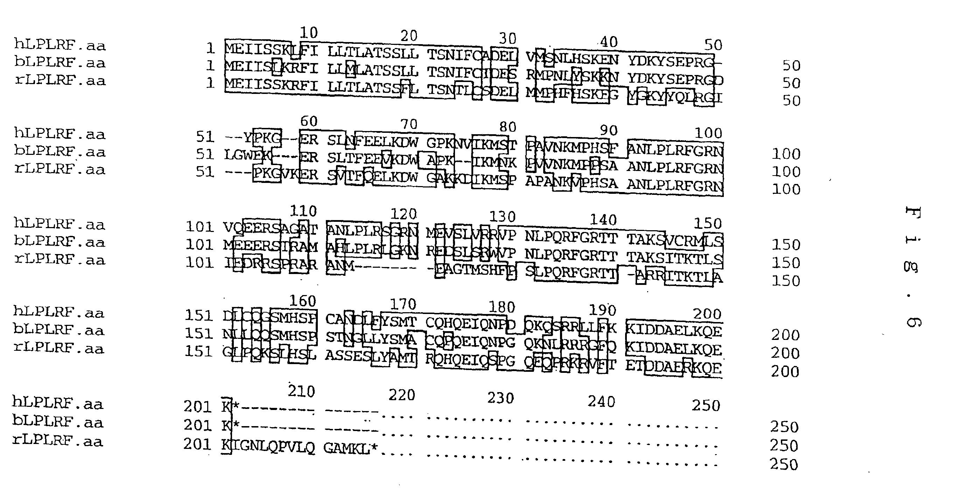

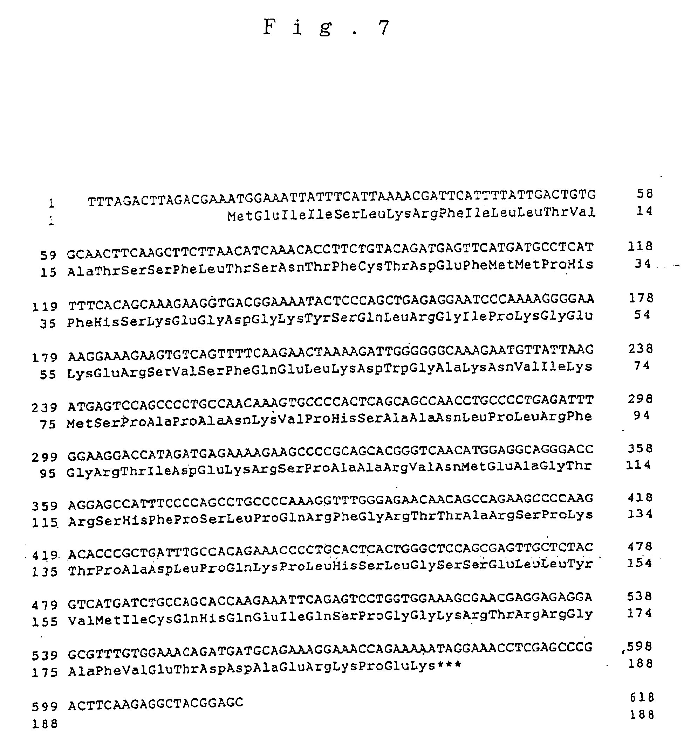

Specific examples of the polypeptide of the present invention include a human-derived polypeptide containing the amino acid sequence represented by SEQ ID NO:1 (FIG. 1), a human-derived polypeptide containing the amino acid sequence represented by SEQ ID NO:8 (FIG. 3), a bovine-derived polypeptide containing the amino acid sequence represented by SEQ ID NO:14 (FIG. 4), a rat-derived polypeptide containing the amino acid sequence represented by SEQ ID NO:18 (FIG. 5), a mouse-derived polypeptide containing the amino acid sequence represented by SEQ ID NO:33 (FIG.7), a rat-derived polypeptide containing the amino acid sequence represented by SEQ ID NO:50, etc.

The polypeptides of the present invention may also be the precursors of the partial peptides below and in this case, may not necessarily require the activities (e.g., a cell stimulating activity or the like) of the following partial peptides.

The partial peptides of the polypeptides of the present invention may be any partial peptides of the polypeptides of the present invention described above, preferably those having a cell stimulating activity caused by adding the partial peptides to cells capable of expressing the partial peptide precursor of the polypeptide of the present invention (specifically, a protein containing the same or substantially the same amino acid sequence as that shown by SEQ ID NO:37, or its salts) (hereinafter simply referred to as a cell-stimulating activity) such as arachidonic acid release, acetylcholine release, intracellular Ca$^{2+}$ release, intracellular CAMP formation, intracellular cGMP formation, inositol phosphate production, change in cell membrane potential, phosphorylation of intracellular proteins, activation of c-fos and change in extracellular pH, etc.), or a somatostatin secretion regulating activity, etc.

As the partial peptide of the polypeptide of the present invention, preferred are peptides containing the RF amide, RS amide or RL amide structure.

The RF amide structure refers to the peptide structure, the C-terminus of which is argininephenylalanine-NH$_2$. The RS amide structure is used to mean the peptide structure, the C-terminus of which is arginine-serine-NH$_2$. In the RL amide structure, the C-terminus of the peptide is arginine-leucine-NH$_2$.

Among these peptides, preferred examples include:

(1) a peptide containing the amino acid sequence of 81 (Met) - 92 (Phe) , 101 (Ser) - 112(Ser) , 124 (Val) - 131 (Phe), 1(Met) - 92(Phe), 1(Met) - 112(Ser) or 1 (Met) - 131(Phe) in the amino acid sequence shown by SEQ ID NO:1;

(2) a peptide containing the amino acid sequence of 81(Met) - 92(Phe), 101(Ser) - 112(Ser), 124(Val)-131(Phe), 1(Met) - 92(Phe), 1(Met) - 112(Ser) or 1(Met) - 131(Phe) in the amino acid sequence shown by SEQ ID NO:8;

(3) a peptide containing the amino acid sequence of 81(Met)-92(Phe), 124(Val)-131(Phe), 1(Met) - 92(Phe) or 1 (Met) - 131(Phe) in the amino acid sequence shown by SEQ ID NO:14;

(4) a peptide containing the amino acid sequence of 84(Pro)-94(Phe) in the amino acid sequence shown by SEQ ID NO:33;

(5) a peptide containing the amino acid sequence of 84(Pro)-94(Phe) in the amino acid sequence shown by SEQ ID NO:50.

In particular, the amide derivatives of these peptides are preferred.

Specific examples include a peptide having the amino acid sequence shown by 81(Met)-92(Phe) in SEQ ID NO:1 wherein the C-terminus is amidated ($-CONH_2$)(SEQ ID NO:39), a peptide having the amino acid sequence shown by 101(Ser)-112(Ser) in SEQ ID NO:1 wherein the C-terminus is amidated ($-CONH_2$)(SEQ ID NO:41) and a peptide having the amino acid sequence shown by 124(Val)-131(Phe) in SEQ ID NO:1 wherein the C-terminus is amidated ($-CONH_2$)(SEQ ID NO:40).

The partial peptide of the present invention may contain an amino acid sequence wherein 1 to 5 (preferably 1 to 3) amino acids are deleted, an amino acid sequence to which 1 to 5 (preferably 1 to 3) amino acids are added, an amino acid sequence wherein 1 to 5 (preferably 1 to 3) amino acids are inserted, or an amino acid sequence wherein 1 to 5 (preferably 1 to 3) amino acids are substituted by other amino acids. The partial peptide may contain a combination of the above amino acid sequences.

In the partial peptide of the present invention, the C-terminus is normally a carboxyl group (-COOH) or carboxylate ($-COO^-$) but the C-terminus may be in the form of an amide ($-CONH_2$) or an ester (-COOR) (wherein R has the same significance as defined above), as has been described with the polypeptide of the present invention. In particular, preferred are the partial peptides having an amide ($-CONH_2$) at the C-terminus.

As in the polypeptide of the present invention described above, the partial peptide of the present invention further includes conjugated peptides such as those in which the amino group of the N-terminal amino acid residue (e. g., methionine residue) is protected by a protecting group, those in which the N-terminal residue is cleaved in vivo and the produced glutamine residue is pyroglutaminated, those in which substituents on the side chains of amino acids in the molecule are protected by appropriate protecting groups and conjugated proteins such as so-called glycoproteins having sugar chains.

The partial peptide of the present invention can be employed as an antigen for producing an antibody and therefore, does not necessarily require the cell stimulating activity, the somatostatin secretion regulating activity, etc.

The polypeptide, amides or esters of the present invention or the partial peptide, amides or esters of the present invention may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptide of the present invention or salts thereof may be manufactured by a publicly known method used to purify a polypeptide from human or other warm-blooded animal cells or tissues described above. Alternatively, the polypeptide of the present invention or salts thereof may also be manufactured by culturing a transformant containing DNA encoding the polypeptide of the present invention, as will be later described. Furthermore, the polypeptide of the present invention or salts thereof may also be manufactured by the methods for synthesizing proteins, which will also be described hereinafter, or by modified methods.

Where the polypeptide or salts thereof are manufactured from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

To synthesize the polypeptide of the present invention, its partial peptide or its salts or amides, commercially available resins that are used for polypeptide synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective polypeptide according to various condensation methods publicly known in the art. At the end of the reaction, the polypeptide is excised from the resin and at the same time, the protecting groups are removed. Then, intramolecular disulfide bond-forming reaction is performed in a highly diluted solution to obtain the objective polypeptide, partial peptide or amides thereof.

For condensation of the protected amino acids described above, a variety of activation reagents for polypeptide synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for polypeptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to polypeptide binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower $C_{1-6}$ alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the polypeptide or partial peptide of the present invention, for example, the α-carboxyl group of the carboxy terminal amino acid is first protected by amidation; the peptide (polypeptide) chain is then extended from the amino group side to a desired length. Thereafter, a polypeptide in which only the protecting group of the N-terminal α-amino group has been eliminated from the polypeptide and a polypeptide in which only the protecting group of the C-terminal carboxyl group has been eliminated are manufactured. The two polypeptides are condensed in a mixture of the solvents described above. The details of the condensation reaction are the same as described above. After the protected polypeptide obtained by the condensation is purified, all the protecting groups are eliminated by the method described above to give the desired crude polypeptide. This crude polypeptide is purified by various known purification means. Lyophilization of the major fraction gives the amide of the desired polypeptide or partial peptide.

To prepare the esterified polypeptide or partial peptide of the present invention, for example, the α-carboxyl group of the carboxy terminal amino acid is condensed with a desired alcohol to prepare the amino acid ester, which is followed by procedure similar to the preparation of the amidated polypeptide above to give the desired esterified polypeptide or partial peptide.

The partial peptide or salts of the present invention can be manufactured by publicly known methods for peptide synthesis, or by cleaving the polypeptide of the present invention with an appropriate peptidase. For the methods for peptide synthesis, for example, either solid phase synthesis or liquid phase synthesis may be used. That is, the partial peptide or amino acids that can construct the polypeptide of the present invention are condensed with the remaining part of the partial peptide of the present invention. Where the product contains protecting groups, these protecting groups are removed to give the desired peptide. Publicly known methods for condensation and elimination of the protecting groups are described in 1) - 5) below.

1) M. Bodanszky & M.A. Ondetti: Peptide Synthesis, Interscience Publishers, New York (1966)

2) Schroeder & Luebke: The Peptide, Academic Press, New York (1965)

3) Nobuo Izumiya, et al.: *Peptide Gosei-no-Kiso to Jikken* (Basics and experiments of peptide synthesis), published by Maruzen Co. (1975)

4) Haruaki Yajima & Shunpei Sakakibara: *Seikagaku Jikken Koza* (Biochemical Experiment) 1, *Tanpakushitsu* no *Kagaku* (Chemistry of Proteins) IV, 205 (1977)

5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form or a different salt form by a publicly known method.

The DNA encoding the polypeptide of the present invention may be any DNA so long as it contains the base sequence encoding the polypeptide of the present invention described above. Such a DNA may also be any one of genomic DNA, genomic DNA library, cDNA derived from the cells or tissues described above, cDNA library derived from the cells or tissues described above and synthetic DNA.

The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Specifically, the DNA encoding the polypeptide of the present invention may be any one of, for example, DNA having the base sequence represented by SEQ ID NO:2, SEQ ID NO:9, SEQ ID NO:15, SEQ ID NO:19, SEQ ID NO: 34 or SEQ ID NO:51 or any DNA having a base sequence hybridizable to the base sequence represented by SEQ ID NO:2, SEQ ID NO:9, SEQ ID NO:15, SEQ ID NO:19, SEQ ID NO:34 or SEQ ID NO:51 under high stringent conditions and encoding a polypeptide which has the activities substantially equivalent to those of the polypeptide of the present invention (e.g., a cell stimulating activity, a somatostatin secretion regulating activity, etc.).

Specific examples of the DNA that is hybridizable to the base sequence represented by SEQ ID NO:2, SEQ ID NO:9, SEQ ID NO:15, SEQ ID NO:19, SEQ ID NO:34 or SEQ ID NO:51 under high stringent conditions include DNA having at least about 70% homology, preferably at least about 80% homology, more preferably at least about 90% homology and most preferably at least about 95% homology, to the base sequence represented by SEQ ID NO:2.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration at about 19 mM to about 40 mM, preferably about 19 mM to about 20 mM at a temperature of about 50°C to about 70°C, preferably about 60'C to about 65°C. In particular, hybridization conditions in a sodium concentration at about 19 mM at a temperature of about 65°C are most preferred.

More specifically, for the DNA encoding the polypeptide having the amino acid sequence represented by SEQ ID NO:1, there may be employed DNA having the base sequence represented by SEQ ID NO:2 and, DNA having the base sequence represented by SEQ ID NO:9 may be used for the DNA encoding the polypeptide having the amino acid sequence represented by SEQ ID NO:8. For the DNA encoding the polypeptide having the amino acid sequence represented by SEQ ID NO:14, DNA having the base sequence represented by SEQ ID NO:15 may be employed and, DNA having the base sequence represented by SEQ ID NO:19 may be used as the DNA encoding the polypeptide having the amino acid sequence represented by SEQ ID NO:18. As the DNA encoding the polypeptide having the amino acid sequence represented by SEQ ID NO:33, there may be employed DNA having the base sequence represented by SEQ ID NO:34 and, DNA having the base sequence represented by SEQ ID NO:51 may be used for the DNA encoding the polypeptide having the amino acid sequence represented by SEQ ID NO:50.

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA.

Specifically, the DNA encoding the partial peptide of the present invention may be any one of, for example, DNA having a partial base sequence of the DNA having the base sequence represented by SEQ ID NO:2, SEQ ID NO:9, SEQ ID NO:15, SEQ ID NO:19, SEQ ID NO:34 or SEQ ID NO:51 or any DNA having a partial base sequence of the DNA having a base sequence hybridizable to the base sequence represented by SEQ ID NO:2, SEQ ID NO:9, SEQ ID NO:15, SEQ ID NO:19, SEQ ID NO:34 or SEQ ID NO:51 under high stringent conditions and encoding a polypeptide which has the activities substantially equivalent to those of the polypeptide of the present invention.

Specific examples of the DNA that is hybridizable to the base sequence represented by SEQ ID NO:2, SEQ ID NO:9, SEQ ID NO:15, SEQ ID NO:19, SEQ ID NO:34 or SEQ ID NO:51 are the same as described above.

Methods for the hybridization and the high stringent conditions that can be used are also the same as described above.

The polypeptide encoded by the DNA that is hybridizable to the base sequence represented by SEQ ID NO: 2, SEQ ID NO:9, SEQ ID NO:15, SEQ ID NO:19, SEQ ID NO:34 or SEQ ID NO:51 can be manufactured in a manner similar to the method for manufacturing the polypeptide of the present invention, which will be described hereinafter. Examples of the amides, esters and salts of the polypeptide include those as described for the amides, esters and salts of the polypeptide of the present invention described above.

Specifically, the DNA encoding the partial peptide of the present invention includes:

(1) a DNA containing a DNA having the base sequence encoding the peptide containing the amino acid sequence of 81(Met) - 92 (Phe), 101(Ser) - 112(Ser), 124(Val) - 131(Phe), 1(Met) - 92 (Phe), 1(Met) - 112(Ser) or 1(Met)- 131(Phe) in the amino acid sequence shown by SEQ ID NO:1, or a DNA containing a DNA having the base sequence hybridizable thereto under high stringent conditions;

(2) a DNA containing a DNA having the base sequence encoding the peptide containing the amino acid sequence of 81 (Met) - 92 (Phe), 101(Ser) - 112(Ser), 124 (Val) - 131(Phe), 1 (Met) - 92 (Phe), 1 (Met) - 112 (Ser) or 1(Met) - 131(Phe) in the amino acid sequence shown by SEQ ID NO:8, or a DNA containing a DNA having the base sequence hybridizable thereto under high stringent conditions;

(3) a DNA containing a DNA having the base sequence encoding the peptide containing the amino acid sequence of 81(Met)-92(Phe), 124(Val)-131(Phe), 1(Met)-92(Phe) or 1(Met)-131(Phe) in the amino acid sequence shown by SEQ ID NO:14, or a DNA containing a DNA having the base sequence hybridizable thereto under high stringent conditions;

(4) a DNA containing a DNA having the base sequence encoding the peptide containing the amino acid sequence of 84(Pro) - 94(Phe) in the amino acid sequence shown by SEQ ID NO:33, or a DNA containing a DNA having the base sequence hybridizable thereto under high stringent conditions;

(5) a DNA containing a DNA having the base sequence encoding the peptide containing the amino acid sequence of 84(Pro) - 94(Phe) in the amino acid sequence shown by SEQ ID NO:50, or a DNA containing the DNA having the base sequence hybridizable thereto under high stringent conditions, and the like.

More specifically, the DNA includes:

a DNA containing a DNA (DNA having 241-276 bases of the base sequence represented by SEQ ID NO:2) having the base sequence represented by SEQ ID NO:42 as the DNA having the base sequence encoding the peptide containing the 81(Met) - 92(Phe) amino acid sequence in the amino acid sequence represented by SEQ ID NO:1; DNA containing the DNA (DNA having 301-336 bases of the base sequence represented by SEQ ID NO:2) having the base sequence represented by SEQ ID NO:43 as the DNA having the base sequence encoding the peptide containing the 101(Ser)-112(Ser) amino acid sequence in the amino acid sequence represented by SEQ ID NO:1; DNA containing the DNA (DNA having 370-393 bases of the base sequence represented by SEQ ID NO:2) having the base sequence represented by SEQ ID NO:44 as the DNA having the base sequence encoding the peptide containing the 124(Val)-131(Phe) amino acid sequence in the amino acid sequence represented by SEQ ID NO:1; DNA containing the DNA (DNA having 1-276 bases of the base sequence represented by SEQ ID NO:2) having the base sequence represented by SEQ ID NO:45 as the DNA having the base sequence encoding the peptide containing the 1(Met)-92(Phe) amino acid sequence in the amino acid sequence represented by SEQ ID NO:1; DNA containing the DNA (DNA having 1-336 bases of the base sequence represented by SEQ ID NO:2) having the base sequence represented by SEQ ID NO:46 as the DNA having the base sequence encoding the peptide containing the 1(Met)-112(Ser) amino acid sequence in the amino acid sequence represented by SEQ ID NO:1; and,

DNA containing the DNA (DNA having 1-393 bases of the base sequence represented by SEQ ID NO:2) having the base sequence represented by SEQ ID NO:47 as the DNA having the base sequence encoding the peptide containing the 1(Met)-131(Phe) amino acid sequence in the amino acid sequence represented by SEQ ID NO:1.

The polypeptide of the present invention or its partial peptide, the receptor protein of the present invention or its partial peptide, which will be described hereinafter, and DNA encoding these proteins or peptides may be labeled in a publicly known manner. Specific examples include those labeled with an isotope, those labeled with fluorescence (labeling with, e.g., fluorescein, etc.), those biotinated and those labeled with enzyme.

For cloning of the DNA that completely encodes the polypeptide or its partial peptide of the present invention (hereinafter sometimes collectively referred to as the polypeptide of the present invention in the following description of cloning and expression of the DNA encoding these polypeptides or the like), the DNA may be either amplified by publicly known PCR using synthetic DNA primers containing a part of the base sequence of the polypeptide of the present invention, or the DNA inserted into an appropriate vector can be selected by hybridization with a labeled DNA fragment or synthetic DNA that encodes a part or entire region of the polypeptide of the present invention. The hybridization can be carried out, for example, according to the method described in Molecular Cloning, 2nd (J. Sambrook et al., Cold Spring Harbor Lab. Press, 1989). The hybridization may also be performed using commercially available library in accordance with the protocol described in the attached instructions.

Conversion of the base sequence of DNA can be effected by publicly known methods such as the Gupped duplex method or the Kunkel method or its modification by using a publicly known kit available as Mutan™-G or Mutan™-K (both manufactured by Takara Shuzo Co., Ltd., trademark).

The cloned DNA encoding the polypeptide of the present invention can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the polypeptide of the present invention can be manufactured, for example, by (a) excising the desired DNA fragment from the DNA encoding the polypeptide of the present invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, HIV·LTR promoter, CMV promoter, HSV-TK promoter, etc.

Among them, CMV (cytomegalovirus) promoter or SR α promoter is preferably used. Where the host is bacteria of the genus Escherichia, preferred examples of the promoter include trp promoter, lac promoter, recA promoter, λPL promoter, 1pp promoter, T7 promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp$^r$), neomycin resistant gene (hereinafter sometimes abbreviated as Neo, G418 resistance), etc. In particular, when dhfr gene is used as the selection marker together with dhfr gene, selection can also be made on thymidine free media.

If necessary and desired, a signal sequence that matches with a host is added to the N-terminus of the polypeptide of the present invention. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector comprising the DNA encoding the polypeptide of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711) and Sf21 cell (both cells are described in Vaughn, J. L. et al., In Vivo, 13, 213-217 (1977).

As the insect, for example, a larva of Bombyx mori can be used (Maeda et al., Nature, 315, 592 (1985)).

Examples of animal cells include monkey cell COS-7, Vero, Chinese hamster cell CHO (hereinafter referred to as CHO cell), dhfr gene deficient Chinese hamster cell CHO (hereinafter simply referred to as CHO(dhfr⁻) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991) or Proc. Natl. Acad. Sci. U.S.A., 75, 1929 (1978).

Insect cells or insects can be transformed, for example, according to the method described in Bio/Technology, 6, 47-55(1988).

Animal cells can be transformed, for example, according to the method described in *Saibo Kogaku* (Cell Engineering), extra issue 8, *Shin Saibo Kogaku Jikken* Protocol (New Cell Engineering Experimental Protocol), 263-267 (1995), published by Shujunsha, or Virology, 52, 456 (1973).

Thus, the transformant transformed with the expression vector containing the DNA encoding the polypeptide can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the polypeptide of the present invention can be produced in the cell membrane of the transformant, etc.

The polypeptide of the present invention can be separated and purified from the culture described above by the following procedures.

When the polypeptide of the present invention is extracted from the culture or cells, after cultivation the transformant or cell is collected by a publicly known method and suspended in a appropriate buffer. The transformant or cell is then disrupted by publicly known methods such as ultrasonication, a treatment with lysozyme and/or freeze-thaw cycling, followed by centrifugation, filtration, etc. Thus, the crude extract of the polypeptide or its partial peptide of the present invention can be obtained. The buffer used for the procedures may contain a protein modifier such as urea or guanidine hydrochloride, or a surfactant such as Triton X-100™, etc. When the polypeptide or its partial peptide of the present invention is secreted in the culture broth, after completion of the cultivation the supernatant can be separated from the transformant or cell to collect the supernatant by a publicly known method.

The supernatant or the polypeptide of the present invention contained in the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the polypeptide of the present invention thus obtained is in a free form, it can be converted into the salt by publicly known methods or modifications thereof. On the other hand, when the polypeptide is obtained in the form of a salt, it can be converted into the free form or in the form of a different salt by publicly known methods or modifications thereof.

The polypeptide of the present invention produced by the recombinant can be treated, prior to or after the purification, with an appropriate protein modifying enzyme so that the protein or partial peptide can be appropriately modified to partially remove a polypeptide. Examples of the protein-modifying enzyme include trypsin, chymotrypsin, arginyl endopeptidase, protein kinase, glycosidase and the like.

The activity of the thus produced polypeptide of the present invention or salts thereof can be determined by a binding test to a labeled ligand and by an enzyme immunoassay using a specific antibody.

Specific examples of the receptor for the polypeptide, its amides or esters or salts of the present invention or the partial peptide or its esters, amides or salts (hereinafter sometimes collectively referred to as the receptor protein) include receptor proteins which possess the same or substantially the same amino acid sequence as the amino acid sequence shown by SEQ ID NO:37.

The receptor protein of the present invention may be any polypeptide derived from any cells of human and other mammals (e.g. guinea pig, rat, mouse, rabbit, swine, sheep, bovine, monkey, etc.) such as splenocyte, nerve cell, glial cell, β cell of pancreas, bone marrow cell, mesangial cell, Langerhans' cell, epidermic cell, epithelial cell, endothelial cell, fibroblast, fibrocyte, myocyte, fat cell, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, etc.; the corresponding precursor cells, stem cells, cancer cells, etc.) or hemocyte type cells; or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testis, ovary, placenta, uterus, bone, joint, skeletal muscle, etc. (especially brain or any of brain regions). The receptor protein may also be a synthetic protein.

The amino acid sequence which has substantially the same amino acid sequence as that represented by SEQ ID NO:37 includes an amino acid sequence having at least about 50% homology, preferably at least about 70% homology, more preferably at least about 80% homology, much more preferably at least about 90% homology and most preferably at least about 95% homology, to the amino acid sequence represented by SEQ ID NO:37.

The protein which has substantially the same amino acid sequence as that shown by SEQ ID NO:37 is preferably a protein having substantially the same amino acid sequence shown by SEQ ID NO:37 and having the activities substantially equivalent to the amino acid sequence shown by SEQ ID NO:37. A specific example of such protein is a protein containing the amino acid sequence represented by SEQ ID NO:54.

The substantially equivalent activities are, for example, a ligand binding activity, a signal transduction activity, a somatostatin secretion regulating activity, etc. The term "substantially equivalent" is used to mean that the nature of these activities is equivalent. Therefore, it is preferred that these activities such as ligand binding activity, a signal transduction activity, a somatostatin secretion regulating activity, etc. are equivalent in strength (e.g., about 0.1 to about 100 times, preferably about 0.5 to about 20 times, more preferably about 0.5 to about 2 times), and it is allowable that even differences among grades such as the strength of these activities and molecular weight of the polypeptide are present.

The activities such as a ligand binding activity, a signal transduction activity, a somatostatin secretion regulating activity or the like can be assayed according to a publicly known method, for example, by means of ligand determination or screening, which will be later described.

The receptor protein of the present invention which can be employed include proteins comprising (i) an amino acid sequence represented by SEQ ID NO:37 or SEQ ID NO:54, of which at least 1 or 2 (preferably 1 to 30, more preferably 1 to 10 and most preferably several (1 or 2)) amino acids are deleted; (ii) an amino acid sequence represented by SEQ ID NO:37 or SEQ ID NO:54, to which at least 1 or 2 (preferably 1 to 30, more preferably 1 to 10 and most preferably several (1 or 2)) amino acids are added; (iii) an amino acid sequence represented by SEQ ID NO:37 or SEQ ID NO:54, in which at least 1 or 2 (preferably 1 to 30, more preferably 1 to 10 and most preferably several (1 or 2)) amino acids are substituted by other amino acids; and (v) a combination of the above amino acid sequences.

Throughout the present specification, the receptor proteins are represented in accordance with the conventional way of describing peptides, that is, the N-terminus (amino terminus) at the left hand and the C-terminus (carboxyl terminus) at the right hand. In the receptor proteins of the present invention including the receptor proteins containing the amino acid sequence shown by SEQ ID NO:37, the C-terminus is usually in the form of a carboxyl group (-COOH) or a carboxylate (-COO⁻) but may be in the form of an amide (-CONH$_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; an aralkyl having 7 to 14 carbon atoms such as a phenyl-$C_{1-2}$ alkyl group, e.g., benzyl, phenethyl, etc.; an α-naphthyl-$C_{1-2}$ alkyl group such as α-naphthylmethyl, etc.; and the like. In addition, pivaloyloxymethyl or the like which is used widely as an ester for oral administration may also be used.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the above C-terminal.

Furthermore, examples of the receptor protein of the present invention include variants of the above receptor protein, wherein the amino group at the N-terminus (e.g., methionine residue) of the peptide is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins having sugar chains.

Specific examples of the receptor protein of the present invention include a rat-derived receptor protein containing the amino acid sequence represented by SEQ ID NO:37, a human-derived receptor protein containing the amino acid sequence represented by SEQ ID NO:54, etc.

As the partial peptide of the receptor protein of the present invention, any partial peptide described for the receptor protein can be used. For example, a part of the receptor protein molecule of the present invention which is exposed to outside of a cell membrane or the like can be used so long as it has a receptor binding activity.

Specifically, the partial peptide of the receptor protein having the amino acid sequence represented by SEQ ID NO:37 or SEQ ID NO:54 is a peptide containing the parts, which have been analyzed to be extracellular domains (hydrophilic domains) in the hydrophobic plotting analysis. A peptide containing a hydrophobic domain part can be used as well. In addition, the peptide may contain each domain separately or plural domains together.

In the receptor protein of the present invention, the partial peptide is a peptide having at least 20, preferably at least 50 and more preferably at least 100 amino acids, in the amino acid sequence, which constitutes the receptor protein of the present invention.

The substantially the same amino acid sequence includes an amino acid sequence having at least about 50% homology, preferably at least about 70% homology, more preferably at least about 80% homology, much more preferably at least about 90% homology and most preferably at least about 95% homology, to the amino acid sequence represented.

Herein the term "substantially equivalent activities" refers to the same significance as defined hereinabove. The "substantially equivalent activities" can be assayed by the same method as described above.

In the partial peptide of the receptor protein of the present invention, at least 1 or 2 (preferably 1 to 10, more preferably several (1 or 2)) amino acids may be deleted; at least 1 or 2 (preferably 1 to 20, more preferably 1 to 10 and most preferably several (1 or 2)) amino acids may be added; or at least 1 or 2 (preferably 1 to 10, more preferably several (1 or 2)), amino acids may be substituted by other amino acids.

In the partial peptide in the receptor protein of the present invention, the C-terminus is usually in the form of a carboxyl group (-COOH) or a carboxylate (-COO⁻) but may be in the form of an amide (-CONH$_2$) or an ester (-COOR), as in the polypeptide of the present invention described above.

Furthermore, examples of the partial peptide of the receptor protein in the present invention include variants of the above peptides, wherein the amino group at the N-terminal methionine residue is protected with a protecting group, those wherein the N-terminal region is cleaved in vivo and the Gln formed is pyroglutaminated, those wherein a substituent on the side chain of an amino acid in the molecule is protected with a suitable protecting group, or conjugated proteins such as glycoproteins having sugar chains, as in the receptor protein of the present invention described above.

As the salts of the receptor protein or its partial peptide in the present invention, physiologically acceptable acid addition salts are particularly preferred. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The receptor protein or salts of the present invention may be manufactured by a publicly known method used to purify a receptor protein from human or other mammalian cells or tissues described above, or by preparing a transformant containing the DNA encoding the receptor protein of the present invention (a host similar to the host of the transformant containing the DNA encoding the polypeptide of the present invention described above may be used) in a manner similar to the aforesaid method for preparing the transformant containing the DNA encoding the polypeptide of the present invention, culturing the resulting transformant in a manner similar to the aforesaid method for preparing the transformant containing the DNA encoding the polypeptide of the present invention. Furthermore, the receptor protein or salts of the present invention may also be manufactured by the aforesaid methods for synthesizing polypeptides or by modified methods.

Where the receptor protein or salts thereof are manufactured from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

The partial peptide of the receptor protein or salts thereof in the present invention can be manufactured by a publicly known method used to synthesize a peptide or, by cleaving the receptor protein of the present invention with an appropriate peptidase.

The receptor protein or salts of the present invention, its partial peptide, amides, esters or salts can be synthesized by the aforesaid method for synthesizing the polypeptide, amides, esters or salts of the present invention.

For the polynucleotide encoding the receptor protein of the present invention, any polynucleotide can be used as long as it contains the base sequence (DNA or RNA, preferably DNA) encoding the receptor protein of the present invention. Such a polynucleotide may be DNA and RNA including mRNA encoding the receptor protein of the present invention. The polynucleotide may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the receptor protein of the present invention, mRNA of the receptor protein of the present invention can be quantified by, for example, the publicly known method published in separate volume of *Jikken Igaku* 15 (7) "New PCR and its application" (1997) or the modified method.

The DNA encoding the receptor protein of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the receptor protein of the present invention may be any DNA having the base sequence shown by SEQ ID NO:38, SEQ ID NO:55 or SEQ ID NO:56, or the base sequence hybridizable to the base sequence represented by SEQ ID NO:38, SEQ ID NO:55 or SEQ ID NO:56 under high stringent conditions and encoding a polypeptide which has the activities substantially equivalent to those of the receptor protein of the present invention (e.g., a ligand binding activity, a signal transduction activity or a somatostatin secretion regulating activity, etc.).

Specific examples of the DNA that is hybridizable to the base sequence represented by SEQ ID NO:38, SEQ ID NO:55 or SEQ ID NO:56 include DNA having at least about 70% homology, preferably at least about 80% homology, more preferably at least about 90% homology and most preferably at least about 95% homology, to the base sequence represented by SEQ ID NO:38, SEQ ID NO:55 or SEQ ID NO:56.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein refer to the conditions, for example, in a sodium concentration of about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

The polypeptide encoded by the DNA, which is hybridizable to the base sequence shown by SEQ ID NO:38, SEQ ID NO:55 or SEQ ID NO:56 can be manufactured by methods similar to those for manufacturing the polypeptide of the present invention, described above. Examples of the amides, esters or salts of the polypeptide are the same as those for the amides, esters or salts of the polypeptide of the present invention described above. More specifically, for the DNA encoding the receptor protein having the amino acid sequence represented by SEQ ID NO:37, DNA having the base sequence represented by SEQ ID NO:38 may be employed; and DNA having the base sequence represented by SEQ ID NO:55 or SEQ ID NO:56 may be used for the DNA encoding the receptor protein having the amino acid sequence represented by SEQ ID NO:54.

The polypeptide containing a part of the base sequence of DNA encoding the receptor protein of the present invention or a part of the base sequence complementary to the DNA is used to mean that not only the DNA encoding the partial peptide of the present invention described below but also RNA are embraced.

According to the present invention, antisense polynucleotides (nucleic acids) that can inhibit replication or expression of the G protein-coupled receptor protein gene can be designed and synthesized based on the cloned or determined base sequence information of the DNA encoding the G protein-coupled receptor protein. Such a polynucleotide (nucleic acid) is capable of hybridizing with RNA of G protein coupled receptor protein gene to inhibit the synthesis or function of said RNA or capable of modulating the expression of a G protein-coupled receptor protein gene via interaction with G protein coupled receptor protein-associated RNA. Polynucleotides complementary to selected sequences of RNA associated with G protein-coupled receptor protein and polynucleotides specifically hybridizable with the selected sequences of RNA associated with G protein-coupled receptor protein are useful in modulating or controlling the expression of a G protein coupled receptor protein gene in vivo and in vitro, and in treating or diagnosing disease later described. The term "corresponding" is used to mean homologous to or complementary to a particular sequence of the base sequence or nucleic acid including the gene. The term "corresponding" between nucleotides, base sequences or nucleic acids and peptides (proteins) usually refers to amino acids of a peptide (protein) under the order derived from the sequence of nucleotides (nucleic acids) or their complements. 5' end hairpin loop, 5' end 6-base-pair repeats, 5' end untranslated region, polypeptide translation initiation codon, protein coding region, ORF translation initiation codon, 3' untranslated region, 3' end palindrome region, and 3' end hairpin loop in the G protein-coupled receptor protein gene may be selected as preferred target regions, though any other region may be selected as a target in G protein coupled receptor protein genes.

Any DNA can be used as the DNA encoding the partial peptide of the receptor protein of the present invention so long as DNA contains the base sequence encoding the partial peptide of the present invention described above. The DNA may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any DNA having the base sequence shown by SEQ ID NO:38, SEQ ID NO:55 or SEQ ID NO:56, or the base sequence hybridizable to the base sequence represented by SEQ ID NO:38, SEQ ID NO:55 or SEQ ID NO:56 under high stringent conditions and encoding a polypeptide which has the activities substantially equivalent to those of the receptor protein of the present invention (e.g., a ligand binding activity, a signal transduction activity or a somatostatin secretion regulating activity, etc.).

Antibodies to the polypeptide of the present invention, partial peptide or esters or amides, or salts thereof, or antibodies to the receptor protein of the present invention or its salts or the partial peptide, its amides or esters may be any of polyclonal antibodies and monoclonal antibodies, as long as they are capable of recognizing the polypeptide of the present invention, partial peptide or esters or amides, of salts thereof, or antibodies to the receptor protein of the present invention or its salts or the partial peptide, its amides or esters.

The antibodies to the polypeptide of the present invention, partial peptide or esters or amides, or salts thereof, or antibodies to the receptor protein of the present invention or its salts or the partial peptide, its amides or esters (hereinafter in the description of antibodies sometimes merely referred to as the receptor protein of the present invention) may be manufactured by publicly known methods for manufacturing antibodies or antisera, using as antigens the polypeptide of the present invention or the receptor protein of the present invention.

[Preparation of monoclonal antibody]

(a) Preparation of monoclonal antibody-producing cells

The polypeptide or receptor protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and two to ten times in total. Examples of the applicable warm-blooded animals are monkeys, rabbits, dogs, guinea pigs, mice, rats, sheep, goats and chickens, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled polypeptide, which will be described later, with the antiserum followed by assaying the binding activity of the labeling agent bound to the antibody. The fusion may be carried out, for example, by the known method by Koehler and Milstein (Nature, 256, 495, 1975). Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the polypeptide (protein) as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the polypeptide labeled with a radioactive substance or an enzyme and detecting the monoclonal antibody bound to the solid phase.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins (for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.

[Preparation of polyclonal antibody]

The polyclonal antibody of the present invention can be manufactured by publicly known methods or modifications thereof. For example, a warm-blooded animal is immunized with an immunogen (polypeptide antigen) per se, or a complex of immunogen and a carrier protein is formed and a warm-blooded animal is immunized with the complex in a manner similar to the method described above for the manufacture of monoclonal antibodies. The product containing the antibody to the polypeptide of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every 2 to 6 weeks and 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense DNA having a complementary or substantial complementary base sequence to the DNA coding for the polypeptide of the present invention or its partial peptide or the DNA coding for the receptor protein of the present invention or its partial peptide (hereinafter these DNAs are collectively referred to as the DNA of the present invention in the following description of antisense DNA) can be any antisense DNA so long as it possesses a base sequence complementary or substantially complementary to that of the DNA of the present invention and capable of suppressing expression of the DNA.

The base sequence substantially complementary to the DNA of the present invention may, for example, be a base sequence having at least about 70% homology, preferably at least about 80% homology, more preferably at least about 90% homology and most preferably at least about 95% homology, to the full-length base sequence or partial base sequence of the base sequence complementary to the DNA of the present invention (i.e., complementary strand to the DNA of the present invention). In the entire base sequence of the complementary strand to the DNA of the present invention, an antisense DNA having at least about 70% homology, preferably at least about 80% homology, more preferably at least about 90% homology and most preferably at least about 95% homology, to the complementary strand of the base sequence which encodes the N-terminal region of the polypeptide of the present invention or the receptor protein of the present invention (e.g., the base sequence around the initiation codon). These antisense DNAs can be synthesized using a publicly known DNA synthesizer, etc.

Hereinafter the utilities of the following substances (1) through (3) are described: (1) the polypeptide of the present invention, its amides or esters, or its partial peptide or its amides or esters, or salts thereof (hereinafter sometimes merely referred to as the polypeptide of the present invention); (2) the receptor protein of the present invention or its salts, or its partial peptide or its amides or esters or salts thereof (hereinafter sometimes merely referred to as the receptor protein of the present invention); and (3) DNA encoding the polypeptide of the present invention or its partial peptide or the receptor protein of the polypeptide or its partial peptide (hereinafter sometimes merely referred to as the DNA of the present invention), antibodies to the polypeptide or the present invention, its amides or esters, or its partial peptide or its amides or esters, or salts thereof (hereinafter sometimes merely referred to as the antibody of the present invention) and the antisense DNA.

(1) Therapeutic and prophylactic agent for the diseases with which the polypeptide of the present invention or the receptor protein of the present invention is associated

Since the polypeptide of the present invention has a cell stimulating activity to the receptor protein of the present invention, any abnormality or deficiency in the DNA encoding the polypeptide of the present invention or any abnormality or deficiency in the receptor protein of the present invention would cause a variety of diseases such as hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer, rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet' s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc. Therefore, the polypeptide of the present invention, the receptor protein of the present invention and the DNA of the present invention can be used as a pharmaceutical composition for the treatment and prevention of various diseases as described above.

The polypeptide of the present invention, the receptor protein of the present invention and the DNA of the present invention can also be used as the therapeutic/prophylactic agent for macular edema cystoid.

Moreover, since the polypeptide of the present invention, the receptor protein of the present invention and the DNA of the present invention are associated with secretion control (also termed secretion regulation; hereinafter the same) of somatostatin, they are useful as:

(1) therapeutic agents for tumors such as acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor, etc.;

(2) therapeutic agents for insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) agents for improving hyperinsulinism or for the treatment of obesity, bulimia, etc. caused by the suppression of appetite;

(4) therapeutic agents for acute pancreatitis, chronic pancreatitis, pancreatic/intestinal fistula, hemorrhagic ulcer, peptic ulcer, gastritis, hyperchylia, reflux esophagitis, etc.;

(5) agents for alleviating various conditions accompanied by Helicobacter pylori bacterial infections (e.g., an agent for suppressing accentuated gastrin secretion, etc.);

(6) agents for suppressing secretion of amylase accompanied by endoscopic cholangio pancreatography and for the postoperative treatment in pancreas surgery;

(7) agents for the treatment of diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro - endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia, etc.;

(8) agents for the treatment of Dumping syndrome, hypersensitive colitis, Crohn's disease, inflammatory bowel disease, etc.;

(9) agents for the treatment of tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-α, β and γ, interleukin-2, etc.);

(10) agents for the prevention and treatment of hypertrophic cardiomyopathy, arteriosclerosis, valvular disease, myocardial infarction (especially myocardial infarction after percutaneous transluminal angioplasty) or regeneration of blood vessels;

(11) agents for the treatment of hemorrhage in esophagal venous cancer, cirrhosis or peripheral vessel disease;

(12) agents for the treatment of disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.), etc.;

(13) agents for the treatment of, for example, dementia (e.g., Alzheimer's disease, Alzheimer's senile dementia, vascular/multiple dementia, etc.), schizophrenia, epilepsy, depression, general anxiety disorder, sleeping disorder, multiple sclerosis, etc.;

(14) agents for the treatment of eye disease (e.g., glaucoma, etc.), etc.;

(15) agents for the prevention and treatment of acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet' s disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis, etc.

(16) Furthermore, the polypeptide of the present invention, receptor protein of the present invention and the DNA of the present invention are also used for healing organ transplantation, burn, wound, alopecia, etc.

(17) These substances of the present invention are also useful as analgesics for suppression or alleviation of chronic or acute pains (pains accompanied by, e.g., postoperative pain, inflammatory pain, toothache, bone disease (e.g., arthritis, rheumatoid, osteoporosis, etc.)).

When a patient has a reduced level of, or deficient in the polypeptide of the present invention or the receptor protein of the present invention in his or her body, the DNA of the present invention can provide its role sufficiently or properly for the patient, (a) by administering the DNA of the present invention to the patient to express the polypeptide of the present invention or the receptor protein of the present invention in vivo, (b) by inserting the DNA of the present invention into a cell, expressing the polypeptide of the present invention or the receptor protein of the present invention and then transplanting the cell to the patient, or (c) by administering the polypeptide of the present invention or the receptor protein of the present invention to the patient.

Where the DNA of the present invention is used as the prophylactic/therapeutic agents described above, the DNA per se is administered directly to human or other warm-blooded animal; alternatively, the DNA is inserted into an appropriate vector such as retrovirus vector, adenovirus vector, adenovirus-associated virus vector, etc. and then administered to human or other warm-blooded animal in a conventional manner. The DNA of the present invention may also be administered as naked DNA, or with adjuvants to assist its uptake by gene gun or through a catheter such as a catheter with a hydrogel. Where the polypeptide of the present invention or the receptor protein of the present invention is used as the aforesaid therapeutic/prophylactic agents, the polypeptide or the protein is advantageously used on a purified level of at least 90%, preferably at least 95%, more preferably at least 98% and most preferably at least 99%.

The polypeptide of the present invention or the receptor protein of the present invention can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing the polypeptide of the present invention or the receptor protein of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol. The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

The vector in which the DNA of the present invention is inserted may also be prepared into pharmaceutical preparations in a manner similar to the procedures above. Such preparations are generally used parenterally.

Since the thus obtained pharmaceutical preparation is safe and low toxic, the preparation can be administered to human or other warm-blooded animal (e.g., rat, mouse, guinea pig, rabbit, chicken, sheep, swine, bovine, horse, cat, dog, monkey, etc.).

The dose of the polypeptide of the present invention or the receptor protein of the present invention varies depending on target disease, subject to be administered, route for administration, etc.; for example, in oral administration for the treatment of nerve disease, the dose is normally about 0.1 mg to about 100 mg, preferably about 1.0 to about 50 mg, and more preferably about 1.0 to about 20 mg per day for adult (as 60 kg body weight). In parenteral administration, the single dose varies depending on subject to be administered, target disease, etc. but it is advantageous for the treatment of nerve disease to administer the active ingredient intravenously at a daily dose of about 0.01 to about 30 mg, preferably about 0.1 to about 20 mg, and more preferably about 0.1 to about 10 mg for adult (as 60 kg body weight). For other animal species, the corresponding dose as converted per 60 kg body weight can be administered.

(2) Screening of drug candidate compounds for disease

Because of the cell stimulating activity or the like by the polypeptide of the present invention to the receptor protein of the present invention, a compound that accelerates or inhibits the functions (e.g., the cell stimulating activity, etc.) of the polypeptide of the present invention or the receptor protein of the present invention, or its salts (these compounds are also referred to as a compound that alter the binding property between the polypeptide of the present invention and the receptor protein of the present invention, or its salts; hereinafter the same) can be used as drugs for the treatment/prevention of diseases such as hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer, rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc.

The compound or its salts that accelerate or inhibit the functions (e.g., the cell stimulating activity or the like) of the polypeptide of the present invention or the receptor protein of the present invention can also be used as the therapeutic and prophylactic agent for macular edema cystoid.

Furthermore, since the polypeptide of the present invention or the receptor protein of the present invention are associated with secretion control of somatostatin, the compound or its salts that accelerate or inhibit the functions (e.g., the cell stimulating activity or the like) of the polypeptide of the present invention or the receptor protein of the present invention are useful as:

(1) therapeutic agents for tumors such as acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor, etc.;

(2) therapeutic agents for insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) agents for improving hyperinsulinism or for the treatment of obesity, bulimia, etc. caused by the suppression of appetite;

(4) therapeutic agents for acute pancreatitis, chronic pancreatitis, pancreatic/intestinal fistula, hemorrhagic ulcer, peptic ulcer, gastritis, hyperchylia, reflux esophagitis, etc.;

(5) agents for alleviating various conditions accompanied by Helicobacter pylori bacterial infections (e.g., an agent for suppressing accentuated gastrin secretion, etc.);

(6) agents for suppressing secretion of amylase accompanied by endoscopic cholangio pancreatography and for the postoperative treatment in pancreas surgery;

(7) agents for the treatment of diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro-endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia, etc.;

(8) agents for the treatment of Dumping syndrome, hypersensitive colitis, Crohn's disease, inflammatory bowel disease, etc.;

(9) agents for the treatment of tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-$\alpha$, $\beta$ and $\gamma$, interleukin-2, etc.);

(10) agents for the prevention and treatment of hypertrophic cardiomyopathy, arteriosclerosis, valvular disease, myocardial infarction (especially myocardial infarction after percutaneous transluminal angioplasty) or regeneration of blood vessels;

(11) agents for the treatment of hemorrhage in esophagal venous cancer, cirrhosis or peripheral vessel disease;

(12) agents for the treatment of disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.), etc.;

(13) agents for the treatment of, for example, dementia (e.g., Alzheimer's disease, Alzheimer's senile dementia, vascular/multiple dementia, etc.), schizophrenia, epilepsy, depression, general anxiety disorder, sleeping disorder, multiple sclerosis, etc.;

(14) agents for the treatment of eye disease (e.g., glaucoma, etc.), etc.;

(15) agents for the prevention and treatment of acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet's disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis, etc.

(16) The compound or its salts are also used for healing organ transplantation, burn, wound, alopecia, etc.

(17) The compound or its salts are also useful as analgesics for suppression or alleviation of chronic or acute pains (pains accompanied by, e.g., postoperative pain, inflammatory pain, toothache, bone disease (e.g., arthritis, rheumatoid, osteoporosis, etc.)).

Therefore, the polypeptide of the present invention or the receptor protein of the present invention is useful as reagents for screening the compound or its salts that accelerate or inhibit the functions of the polypeptide of the present invention or the receptor protein of the present invention.

That is, the present invention provides:

(1) a method for screening the compound or its salts that accelerate the functions (e.g., a cell stimulating activity, a somatostatin secretion regulating activity, etc.) of the polypeptide of the present invention, its amides or esters, or salts thereof, the partial peptide, its amides or esters, or salts thereof (hereinafter sometimes merely referred to as the accelerator), or the compound or its salts that inhibit the functions of the polypeptide of the present invention, its amides or esters, or salts thereof, the partial peptide, its amides or esters, or salts thereof (hereinafter sometimes merely referred to as the inhibitor), which comprises using the polypeptide of the present invention, its amides or esters, or salts thereof, or the partial peptide, its amides or esters, or salts thereof.

The present invention further provides:

(2) a method for screening the compound or its salts that accelerate the functions (e.g., a cell stimulating activity, a somatostatin secretion regulating activity, etc.) of the receptor protein of the present invention or its salts, or the partial peptide, its amides or esters, or salts thereof (hereinafter sometimes merely referred to as the accelerator), or the compound or its salts that inhibit the functions of the receptor protein of the present invention or its salts, the partial peptide, its amides or esters, or salts thereof (hereinafter sometimes merely referred to as the inhibitor), which comprises using the receptor protein of the present invention or its salts, or the partial peptide, its amides or esters, or salts thereof.

More specifically, the present invention provides:

(3) a method for screening the compound or its salts that accelerate the functions (e.g., a cell stimulating activity, a somatostatin secretion regulating activity, etc.) of the polypeptide of the present invention, its amides or esters, or salts thereof, the partial peptide, its amides or esters, or salts thereof, or the receptor protein of the present invention or its salts, or the partial peptide, its amides or esters, or salts thereof (hereinafter sometimes merely referred to as the accelerator), or, the compound or its salts that inhibits the functions (e.g., a cell stimulating activity, a somatostatin secretion regulating activity, etc.) of the polypeptide of the present invention, its amides or esters, or salts thereof, the partial peptide, its amides or esters, or salts thereof, or the receptor protein of the present invention or its salts, or the partial peptide, its amides or esters, or salts thereof (hereinafter sometimes merely referred to as the inhibitor), which comprises using the polypeptide of the present invention, its amides or esters, or salts thereof, or the partial peptide, its amides or esters, or salts thereof, or the receptor protein of the present invention or its salts, or the partial peptide, its amides or esters, or salts thereof (specifically the protein containing the same or substantially the same amino acid sequence as the amino acid sequence shown by SEQ ID NO:37, or salts thereof, or the partial peptide, its amides or esters, or salts thereof).

The present invention further provides:

(3) a method for screening the compound or its salts that accelerate the functions (e.g., a cell stimulating activity, a somatostatin secretion regulating activity, etc.) of the polypeptide of the present invention, its amides or esters, or salts thereof, the partial peptide, its amides or esters, or salts thereof, or the receptor protein of the present invention or its salts, or the partial peptide, its amides or esters, or salts thereof (hereinafter sometimes merely referred to as the accelerator), or, the compound or its salts that inhibits the functions (e.g., a cell stimulating activity, a somatostatin secretion regulating activity, etc.) of the polypeptide of the present invention, its amides or esters, or salts thereof, the partial peptide, its amides or esters, or salts thereof, or the receptor protein of the present invention or its salts, or the partial peptide, its amides or esters, or salts thereof (hereinafter sometimes merely referred to as the inhibitor), which comprises measuring

(i) the activity of the polypeptide of the present invention, its amides or esters, or salts thereof, or the partial peptide, its amides or esters, or salts thereof, when the receptor protein of the present invention or its salts, or the partial peptide, its amides or esters, or salts thereof (specifically the protein containing the same or substantially the same amino acid sequence as the amino acid sequence shown by SEQ ID NO:37, or salts thereof, or the partial peptide, its amides or esters, or salts thereof) is brought into contact with the polypeptide of the present invention, its amides or esters, or salts thereof, or the partial peptide, its amides or esters, or salts thereof, and
(ii) the activity of the polypeptide of the present invention, its amides or esters, or salts thereof, or the partial peptide, its amides or esters, or salts thereof, when the receptor protein of the present invention or its salts, or the partial peptide, its amides or esters, or salts thereof (specifically the protein containing the same or substantially the same amino acid sequence as the amino acid sequence shown by SEQ ID NO:37, or salts thereof, or the partial peptide, its amides or esters, or salts thereof) and a test compound are brought into contact with the polypeptide of the present invention, its amides or esters, or salts thereof, or the partial peptide, its amides or esters, or salts thereof;

and comparing the activities;
and the like.

Specifically, the screening method described above is characterized by measuring the cell stimulating activities of the polypeptide of the present invention and a test compound or the binding amount of the polypeptide of the present invention and a test compound to the receptor protein of the present invention, in the cases (i) and (ii), and comparing these activities.

The activities of the polypeptide in the present invention such as the cell stimulating activity, etc. can be measured in accordance with publicly known methods, for example, Dockray, G.J., et al., Nature, 305, 328-330, 1983, Fukusumi, S., et al., Biochem. Biophys. Res. Commun., 232, 157-163, 1997, Hinuma, S., et al., Nature, 393, 272-276, 1998, Tatemoto, K., et al., Biochem. Biophys. Res. Commun., 251, 471-476, 1998, etc., or modifications thereof.

The binding amount of the polypeptide of the present invention and a test compound to the receptor protein of the present invention can be measured by a modification of the methods for "determination of a ligand (agonist) to the receptor protein of the present invention" which will be described hereinafter.

Examples of such a test compound are a peptide, a protein, a non-peptide compound, a synthetic compound, a fermentation product, a cell extract, a plant extract, an animal tissue extract and the like. These compounds may be novel compounds or publicly known compounds.

To perform the screening method described above, the polypeptide of the present invention is suspended in a buffer suitable for screening to prepare a specimen of the polypeptide of the present invention. Any buffer having pH of approximately 4 to 10 (desirably pH of approximately 6 to 8) such as a phosphate buffer, Tris-hydrochloride buffer, etc. may be used so long as it does not interfere the reaction between the polypeptide of the present invention and the receptor protein of the present invention.

For example, when a test compound increases the cell stimulating activity, etc. in (ii) described above by at least about 20%, preferably at least 30%, more preferably at least about 50% as compared to the case of (i) above, the test compound can be selected to be a compound that accelerates the cell stimulating activity, etc. of the polypeptide of the present invention. On the other hand, a test compound can be selected to be a compound that inhibits the cell stimulating activity, etc. of the polypeptide of the present invention, when the test compound inhibits the cell stimulating activity, etc. in (ii) described above by at least about 20%, preferably at least 30%, more preferably at least about 50% as compared to the case of (i) above.

It is desirable, before conducting these tests, to examine the binding ability of a test compound to the receptor protein of the present invention to see if the test compound is capable of binding to the receptor protein of the present invention, which is effected by the methods (1) to (3) later described for the "determination of ligand (agonist) to the receptor protein of the present invention".

As an index that the test compound described above is judged to be the compound or its salts that accelerate or inhibit the activities of the polypeptide of the present invention, there is such an activity that inhibit the binding between the receptor protein of the present invention and the labeled polypeptide of the present invention or its partial peptide. According to the binding test system described, e.g., in Hosoya, M. et al., Biochem. Biophys. Res. Commun., 194 (1), 133-134, 1993, a test compound that inhibits the binding of the labeled compound by at least 10% in a concentration of $1 \times 10^{-2}$ M or less is highly likely to be the compound or salts that accelerate or inhibit the activities of the polypeptide of the present invention. However, since the binding inhibition activity is a relative value based on the binding of the labeled compound, the activity is not essential for judging the test compound to be a compound or salts that accelerate or inhibit the activities of the polypeptide of the present invention.

The kit for screening of the present invention comprises the polypeptide of the present invention, its amides or esters, or salts thereof, the partial peptide, its amides or esters, or salts thereof. Preferably, the kit for screening of the present invention further comprises the receptor to the polypeptide of the present invention, its amides or esters, or salts thereof, the partial peptide, its amides or esters, or salts thereof, that is, the receptor protein of the present invention or its salts, the partial peptide, its amides or esters, or salts thereof (specifically, the protein containing the same or substantially the same amino acid sequence as that shown by SEQ ID NO:37).

Examples of the screening kit according to the present invention include the following:

1. Reagent for screening

(1) Buffers for assay and washing

Hanks' Balanced Salt Solution (manufactured by Gibco) supplemented with 0.05% of bovine serum albumin (manufactured by Sigma).

The buffers may be sterilized by filtration through a membrane filter with a 0.45 μm pore size and stored at 4°C, or may be prepared at use.

(2) A receptor preparation

CHO cells in which the receptor protein of the present invention is expressed are subcultured at $5 \times 10^5$ cells/well on a 12-well plate followed by culturing at 37°C under a 5% $CO_2$ and 95% air for 2 days.

(3) Labeled ligand

The polypeptide of the present invention, its amides or esters, or the partial peptide, its amides or esters are labeled with commercially available [$^3$H], [$^{125}$I], [$^{14}$C], [$^{35}$S], etc. The product in the form of an aqueous solution is stored at 4°C or at -20°C, which will be diluted at use to 1 µM with a buffer for the assay.

(4) Standard ligand solution

The polypeptide of the present invention, its amides or esters, or the partial peptide, its amides or esters, are dissolved in PBS containing 0.1% of bovine serum albumin (manufactured by Sigma) to make the volume 1 mM and then stored at -20°C.

2. Method for assay

(1) CHO cells are cultured in a 12-well tissue culture plate to express the receptor protein of the present invention. After washing the CHO cells twice with 1 ml of buffer for the assay, 490 µl of the buffer for assay is added to each well.
(2) After 5 µl of a test compound solution of $10^{-3}$ to $10^{-10}$ M is added, 5 µl of a labeled ligand is added to the system followed by incubating at room temperature for an hour. To determine the amount of the non-specific binding, 5 µl of the ligand of $10^{-3}$ M is added to the system, instead of the test compound.
(3) The reaction mixture is removed from the well, which is washed three times with 1 ml each of the buffer for assay. The labeled ligand bound to the cells is dissolved in 0.2N NaOH-1% SDS and mixed with 4 ml of a liquid scintillator A (manufactured by Wako Pure Chemical).
(4) Radioactivity is measured using a liquid scintillation counter (manufactured by Beckman) and PMB (percent of the maximum binding) is calculated in accordance with the following equation:

$$PMB = [(B-NSB)/(B_0 - NSB)] \times 100$$

wherein:

PMB:percent of the maximum binding
B: value when a sample is added
NSB:non-specific binding
$B_0$: maximum binding

The compound or a salt thereof obtainable by the screening method or by the screening kit of the present invention is the compound selected from the test compounds described above, such as peptides, proteins, non-peptide compounds, synthetic compounds, fermentation products, cell extracts, plant extracts, animal tissue extracts, plasma, etc. and the compound that accelerates or inhibits the functions (e.g., a cell stimulating activity, a somatostatin secretion regulating activity, etc.) of the polypeptide of the present invention.
As the salts of the compound, there may be employed similar salts to those of the polypeptide of the present invention described above.
When the compound or salts thereof obtainable by the screening method or the screening kit of the present invention are used as the therapeutic and prophylactic agents described above, a conventional means may be applied to making pharmaceutical preparations. For example, the compound or its salts may be prepared into tablets, capsules, elixirs, microcapsules, sterile solutions, suspensions, etc.
Since the thus obtained preparation is safe and low toxic, it can be administered to human or warm-blooded animal (e.g., mouse, rat, rabbit, sheep, swine, bovine, horse, chicken, cat, dog, monkey, etc.).
The dose of the compound or salts thereof varies depending on activity, target disease, subject to be administered, method for administration, etc.; for example, in oral administration of the compound that accelerates the functions of the polypeptide of the present invention, the dose is normally about 0.1 to about 100 mg, preferably about 1.0 to about 50 mg, more preferably about 1.0 to about 20 mg per day for adult (as 60 kg body weight). In parenteral administration, the single dose varies depending on subject to be administered, target disease, etc. but it is advantageous to administer, for example, the compound that accelerates the functions of the polypeptide of the present invention intravenously at a daily dose of about 0.01 to about 30 mg, preferably about 0.1 to about 20 mg, more preferably about 0.1 to about 10 mg for adult (as 60 kg body weight). For other animal species, the corresponding dose as converted per 60 kg weight can be administered.

Turning to the compound that inhibits the functions of the polypeptide of the present invention when it is orally administered, the dose is normally about 0.1 to about 100 mg, preferably about 1.0 to about 50 mg, more preferably about 1.0 to about 20 mg per day for adult (as 60 kg body weight). In parenteral administration, the single dose varies depending on subject to be administered, target disease, etc. When the compound that inhibits the functions of the polypeptide of the present invention is administered to adult (as 60 kg body weight) generally in the form of injection, it is advantageous to administer the compound intravenously at a daily dose of about 0.01 to about 30 mg, preferably about 0.1 to about 20 mg, more preferably about 0.1 to about 10 mg. For other animal species, the corresponding dose as converted per 60 kg weight can be administered.

(3) Quantification for the polypeptide of the present invention, its amides or esters, or salts thereof, the partial peptide, its amides or esters, or salts thereof, and the receptor protein of the present invention or salts thereof, or the partial peptide, its amides or esters, or salts thereof:

The antibody to the polypeptide of the present invention or the receptor protein of the present invention (hereinafter sometimes merely referred to as the antibody of the present invention) is capable of specifically recognizing the polypeptide of the present invention or the receptor protein of the present invention and thus, can be used for a quantification of the polypeptide of the present invention or the receptor protein of the present invention in a test sample fluid, in particular, for a quantification by sandwich immunoassay.

That is, the present invention provides:

(i) a method for quantification of the polypeptide of the present invention or the receptor protein of the present invention in a test sample fluid, which comprises competitively reacting the antibody of the present invention, a test sample fluid and the labeled polypeptide of the present invention or the labeled receptor protein of the present invention, and measuring the ratio of the labeled polypeptide of the present invention or the labeled receptor protein of the present invention bound to said antibody; and,

(ii) a method for quantification of the polypeptide of the present invention or the receptor protein of the present invention in a test sample fluid, which comprises reacting the test sample fluid simultaneously or continuously with the antibody of the present invention immobilized on a carrier and a labeled antibody of the present invention, and then measuring the activity of the labeling agent on the insoluble carrier.

In the method (ii) for quantification described above, it is preferred that one antibody is capable of recognizing the N-terminal region of the polypeptide of the present invention or the receptor protein of the present invention, while another antibody is capable of recognizing the C-terminal region of the polypeptide of the present invention or the receptor protein of the present invention.

The monoclonal antibody to the polypeptide of the present invention or the receptor protein of the present invention may be used to assay the polypeptide of the present invention or the receptor protein of the present invention. Moreover, the polypeptide of the present invention or the receptor protein of the present invention can be detected by means of a tissue staining as well. For these purposes, the antibody molecule per se may be used or $F(ab')_2$, Fab' or Fab fractions of the antibody molecule may also be used.

There is no particular limitation for the assaying method using the antibody to the polypeptide of the present invention or the receptor protein of the present invention; any method may be used so far as it relates to a method in which the amount of antibody, antigen or antibody-antigen complex can be detected by a chemical or a physical means, depending on or corresponding to the amount of antigen (e.g., the amount of the polypeptide) in a test sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be described later, is particularly preferred.

Examples of the labeling agent used in the assay method using the labeling substance are radioisotopes, enzymes, fluorescent substances and luminescent substances, etc. Examples of the radioisotope are [$^{125}$I], [$^{131}$I], [$^3$H] , [$^{14}$C], etc. Preferred examples of the enzyme are those that are stable and have a high specific activity, which include β-galactosidase, β-glucosidase, alkaline phosphatase, peroxidase and malate dehydrogenase. Examples of the fluorescent substance are fluorescamine, fluorescein isothiocyanate, etc. Examples of the luminescent substance are luminol, a luminol derivative, luciferin, lucigenin, etc. Furthermore, the biotin-avidin system may also be used for binding of an antibody or antigen to a labeling agent.

In the immobilization of antigens or antibodies, physical adsorption may be used. Alternatively, chemical binding that is conventionally used for immobilization of proteins or enzymes may be used as well. Examples of the carrier include insoluble polysaccharides such as agarose, dextran and cellulose; synthetic resins such as polystyrene, polyacrylamide and silicone; glass; etc.

In the sandwich method, a test sample fluid is reacted with an immobilized monoclonal antibody of the present invention (first reaction), then reacted with another labeled monoclonal antibody of the present invention (second reaction) and the activity of the labeling agent on the insoluble carrier is assayed, whereby the amount of the polypeptide of the present invention or the receptor protein of the present invention in the test sample fluid can be quantified. The first and second reactions may be carried out in a reversed order, simultaneously or sequentially with an interval. The type of the labeling agent and the method for immobilization may be the same as those described hereinabove. In the immunoassay by the sandwich method, it is not always necessary that the antibody used for the labeled antibody and for the solid phase should be one type or one species but a mixture of two or more antibodies may also be used for the purpose of improving the measurement sensitivity, etc.

In the method for assaying the polypeptide of the present invention or the receptor protein of the present invention by the sandwich method according to the present invention, preferred monoclonal antibodies of the present invention used for the first and the second reactions are antibodies, which binding sites to the polypeptide of the present invention or the receptor protein of the present invention are different from one another. Thus, the antibodies used in the first and the second reactions are those wherein, when the antibody used in the second reaction recognizes the C-terminal region of the polypeptide of the present invention or the receptor protein, the antibody recognizing the site other than the C-terminal regions, e.g., recognizing the N-terminal region, is preferably used in the first reaction.

The monoclonal antibody of the present invention may be used in an assay system other than the sandwich method, such as a competitive method, an immunometric method and a nephrometry.

In the competitive method, an antigen in a test sample fluid and a labeled antigen are competitively reacted with an antibody, then the unreacted labeled antigen (F) and the labeled antigen bound to the antibody (B) are separated (i.e., B/F separation) and the labeled amount of either B or F is measured to determine the amount of the antigen in the test sample fluid. In the reactions for such a method, there are a liquid phase method in which a soluble antibody is used as the antibody and the B/F separation is effected by polyethylene glycol while a second antibody to the antibody is used, and a solid phase method in which an immobilized antibody is used as the first antibody or a soluble antibody is used as the first antibody while an immobilized antibody is used as the second antibody.

In the immunometric method, an antigen in a test sample fluid and an immobilized antigen are competitively reacted with a given amount of a labeled antibody followed by separating the solid phase from the liquid phase; or an antigen in a test sample fluid and an excess amount of labeled antibody are reacted, then an immobilized antigen is added to bind an unreacted labeled antibody to the solid phase and the solid phase is separated from the liquid phase. Thereafter, the labeled amount of any of the phases is measured to determine the antigen amount in the test sample fluid.

In the nephrometry, the amount of insoluble sediment, which is produced as a result of the antigen-antibody reaction in a gel or in a solution, is measured. Even when the amount of an antigen in a test sample fluid is small and only a small amount of the sediment is obtained, a laser nephrometry utilizing laser scattering can be suitably used.

In applying each of those immunoassays to the assay method for the present invention, any special conditions or operations are not required to set forth. The assay system for the polypeptide of the present invention may be constructed in addition to conditions or operations conventionally used for each of the methods, taking the technical consideration of one skilled in the art into account consideration. For the details of such conventional technical means, a variety of reviews, reference books, etc. may be referred to (for example, Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immuochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)) ; ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.)

As described above, the polypeptide of the present invention or the receptor protein of the present invention can be quantified with high sensitivity, using the antibody of the present invention.

Furthermore, when a decrease or increase in level of the polypeptide of the present invention or the receptor protein of the present invention is detected by quantifying the level of the polypeptide of the present invention or the receptor protein using the antibody of the present invention, it can be diagnosed that the following diseases are involved or it is highly likely to suffer from these disease in the future. Examples of such diseases are hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer, rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet' s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc.

Where a decrease or increase in the level of the polypeptide of the present invention or the receptor protein of the present invention is detected, it can be diagnosed as well that a disease such as macular edema cystoid or the like is involved or it is highly likely to suffer from such a disease in the future.

Moreover, since the polypeptide of the present invention, the receptor protein of the present invention and the DNA of the present invention are associated with secretion control of somatostatin, it can be diagnosed that the following diseases are involved or there is a high possibility to suffer from these diseases in the future, when a decreased or increased level of the polypeptide of the present invention or the receptor protein of the present invention is detected. Examples of these diseases are:

(1) acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor;

(2) insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) obesity, bulimia, etc. caused by improving hyperinsulinism or the suppression of appetite;

(4) acute pancreatitis, chronic pancreatitis, pancreatic/intestinal fistula, hemorrhagic ulcer, peptic ulcer, gastritis, hyperchylia, reflux esophagitis;

(5) various conditions accompanied by Helicobacter pylori bacterial infections (e.g., an agent for suppressing accentuated gastrin secretion, etc.);

(6) oversecretion of amylase accompanied by endoscopic cholangio pancreatography and for the postoperative treatment in pancreas surgery;

(7) diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro-endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia;

(8) Dumping syndrome, hypersensitive colitis, Crohn's disease, inflammatory bowel disease;

(9) tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-$\alpha$, $\beta$ and $\gamma$, interleukin-2, etc.);

(10) hypertrophic cardiomyopathy, arteriosclerosis, valvular disease, myocardial infarction (especially myocardial infarction after percutaneous transluminal angioplasty) or regeneration of blood vessels;

(11) hemorrhage in esophagal venous cancer, cirrhosis or peripheral vessel disease;

(12) disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.);

(13) dementia (e.g., Alzheimer's disease, Alzheimer's senile dementia, vascular/multiple dementia, etc.), schizophrenia, epilepsy, depression, general anxiety disorder, sleeping disorder, multiple sclerosis;

(14) eye disease (e.g., glaucoma, etc.);

(15) acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet s disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis;

(16) burn, wound, alopecia;

(17) chronic or acute pains (pains accompanied by, e.g., postoperative pain, inflammatory pain, toothache, bone disease (e.g., arthritis, rheumatoid, osteoporosis, etc.)).

The antibody of the present invention can be employed for detecting the polypeptide of the present invention or the receptor protein of the present invention which may be present in a test sample fluid such as a body fluid, a tissue, etc. The antibody can also be used for preparation of an antibody column for purification of the polypeptide of the present invention or the receptor protein of the present invention, detection of the receptor protein of the present invention in the fractions upon purification, and analysis of the behavior of the polypeptide of the present invention or the receptor protein of the present invention in the cells under investigation.

(4) Gene diagnostic agent

By using the DNA of the present invention, e.g., as a probe, an abnormality (gene abnormality) of the DNA or mRNA coding for the polypeptide of the present invention or the receptor protein of the present invention in human or warm-blooded animal (e.g., rat, mouse, guy pig, rabbit, chicken, sheep, swine, bovine, horse, cat, dog, monkey, etc.) can be detected. Therefore, the DNA of the present invention is useful as a gene diagnostic agent for the damage to the DNA or mRNA, its mutation, or its decreased expression, or increased expression or overexpression of the DNA or mRNA.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

In case that decreased expression or overexpression is detected by, e.g., the Northern hybridization, it can be diagnosed that the following diseases are involved or it is highly likely to suffer from these disease in the future. Examples of such diseases are hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer, rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet' s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc.

In case that a decreased expression or overexpression is detected by the Northern hybridization, it can also be diagnosed that a disease such as macular edema cystoid or the like is involved or it is highly likely to suffer from such a disease in the future.

In addition, since the polypeptide of the present invention, the receptor protein of the present invention and the DNA of the present invention are associated with secretion control of somatostatin, the decrease in expression or overexpression detected by the Northern hybridization results in such a diagnosis that the following diseases are involved or there is a high possibility to suffer from these diseases in the future. Examples of the diseases are:

(1) acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor;

(2) insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) obesity, bulimia, etc. caused by improving hyperinsulinism or the suppression of appetite;

(4) acute pancreatitis, chronic pancreatitis, pancreatic/intestinal fistula, hemorrhagic ulcer, peptic ulcer, gastritis, hyperchylia, reflux esophagitis;

(5) various conditions accompanied by Helicobacter pylori bacterial infections (e.g., an agent for suppressing accentuated gastrin secretion, etc.);

(6) oversecretion of amylase accompanied by endoscopic cholangio pancreatography and for the postoperative treatment in pancreas surgery;

(7) diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro-endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia;

(8) Dumping syndrome, hypersensitive colitis, Crohn's disease, inflammatory bowel disease;

(9) tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-$\alpha$, $\beta$ and $\gamma$, interleukin-2, etc.);

(10) hypertrophic cardiomyopathy, arteriosclerosis, valvular disease, myocardial infarction (especially myocardial infarction after percutaneous trnsluminal angioplasty) or regeneration of blood vessels;

(11) hemorrhage in esophagal venous cancer, cirrhosis or peripheral vessel disease;

(12) disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.);

(13) dementia (e.g., Alzheimer's disease, Alzheimer's senile dementia, vascular/multiple dementia, etc.), schizophrenia, epilepsy, depression, general anxiety disorder, sleeping disorder, multiple sclerosis;

(14) eye disease (e.g., glaucoma, etc.);

(15) acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet' s disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis;

(16) burn, wound, alopecia;

(17) chronic or acute pains (pains accompanied by, e.g., postoperative pain, inflammatory pain, toothache, bone disease (e.g., arthritis, rheumatoid, osteoporosis, etc.)).

(5) Pharmaceutical composition comprising antisense DNA

Antisense DNA that binds to the DNA of the present invention complemenarily to inhibit expression of the DNA can be used as the agent for the treatment/prevention of diseases such as hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer, rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet' s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc.

The antisense DNA that binds to the DNA of the present invention and can inhibit expression of the DNA can also be used as the therapeutic/prophylactic agent for macular edema cystoid.

In addition, since the polypeptide of the present invention or the receptor protein of the present invention are associated with secretion control of somatostatin, the antisense DNA that binds to the DNA of the present invention and can inhibit expression of the DNA are useful as:

(1) therapeutic agents for tumors such as acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor, etc.;

(2) therapeutic agents for insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) agents for improving hyperinsulinism or for the treatment of obesity, bulimia, etc. caused by the suppression of appetite;

(4) therapeutic agents for acute pancreatitis, chronic pancreatitis, pancreatic/intestinal fistula, hemorrhagic ulcer, peptic ulcer, gastritis, hyperchylia, reflux esophagitis, etc.;

(5) agents for alleviating various conditions accompanied by Helicobacter pylori bacterial infections (e.g., an agent for suppressing accentuated gastrin secretion, etc.);

(6) agents for suppressing secretion of amylase accompanied by endoscopic cholangio pancreatography and for the postoperative treatment in pancreas surgery;

(7) agents for the treatment of diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro-endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia, etc.;

(8) agents for the treatment of Dumping syndrome, hypersensitive colitis, Crohn's disease, inflammatory bowel disease, etc.;

(9) agents for the treatment of tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-a, β and γ, interleukin-2, etc.);

(10) agents for the prevention and treatment of hypertrophic cardiomyopathy, arteriosclerosis, valvular disease, myocardial infarction (especially myocardial infarction after percutaneous transluminal angioplasty) or regeneration of blood vessels;

(11) agents for the treatment of hemorrhage in esophagal venous cancer, cirrhosis or peripheral vessel disease;

(12) agents for the treatment of disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.), etc.;

(13) agents for the treatment of, for example, dementia (e.g., Alzheimer's disease, Alzheimer's senile dementia, vascular/multiple dementia, etc.), schizophrenia, epilepsy, depression, general anxiety disorder, sleeping disorder, multiple sclerosis, etc.;

(14) agents for the treatment of eye disease (e.g., glaucoma, etc.), etc.;

(15) agents for the prevention and treatment of acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet' s disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis, etc.

(16) The antisense DNA can also be used for healing organ transplantation, burn, wound, alopecia, etc.

(17) The antisense DNA is also useful as analgesics for suppression or alleviation of chronic or acute pains (pains accompanied by, e.g., postoperative pain, inflammatory pain, toothache, bone disease (e.g., arthritis, rheumatoid, osteoporosis, etc.)).

In the case that the antisense DNA described above is used as the therapeutic/prophylactic agent, the therapeutic/prophylactic agents for various diseases described above comprising the DNA of the present invention apply similarly to the antisense DNA.

For example, when the antisense DNA is used, the antisense DNA is administered directly, or the antisense DNA is inserted into an appropriate vector such as retrovirus vector, adenovirus vector, adenovirus-associated virus vector, etc. followed by treating in a conventional manner. The antisense DNA may be administered as it stands, or with a physiologically acceptable carrier to assist its uptake by gene gun or through a catheter such as a catheter with a hydrogel.

In addition, the antisense DNA may also be employed as an oligonucleotide probe for diagnosis to examine the presence of the DNA of the present invention in tissues or cells and states of its expression.

(6) Pharmaceutical composition comprising the antibody of the present invention

The antibody of the present invention which possesses the effect to neutralize the activities of the polypeptide of the present invention or the receptor peptide of the present invention can be used as drugs for the treatment/prevention of diseases such as hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer/rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet' s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc.

In addition, the antibody of the present invention having the effect of neutralizing the polypeptide of the present invention or the receptor protein of the present invention can also be used as the therapeutic/prophylactic agent for macular edema cystoid. Moreover, since the polypeptide of the present invention or the receptor protein of the present invention are associated with secretion control of somatostatin, the antibody of the present invention having the effect of neutralizing the polypeptide of the present invention or the receptor protein of the present invention are useful as:

(1) therapeutic agents for tumors such as acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor, etc.;

(2) therapeutic agents for insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) agents for improving hyperinsulinism or for the treatment of obesity, bulimia, etc. caused by the suppression of appetite;

(4) therapeutic agents for acute pancreatitis, chronic pancreatitis, pancreatic/intestinal fistula, hemorrhagic ulcer, peptic ulcer, gastritis, hyperchylia, reflux esophagitis, etc.;

(5) agents for alleviating various conditions accompanied by Helicobacter pylori bacterial infections (e.g., an agent for suppressing accentuated gastrin secretion, etc.);

(6) agents for suppressing secretion of amylase accompanied by endoscopic cholangio pancreatography and for the postoperative treatment in pancreas surgery;

(7) agents for the treatment of diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro-endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia, etc.;

(8) agents for the treatment of Dumping syndrome, hypersensitive colitis, Crohn's disease, inflammatory bowel disease, etc.;

(9) agents for the treatment of tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer; ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-$\alpha$, $\beta$ and $\gamma$, interleukin-2, etc.);

(10) agents for the prevention and treatment of hypertrophic cardiomyopathy, arteriosclerosis, valvular disease, myocardial infarction (especially myocardial infarction after percutaneous transluminal angioplasty) or regeneration of blood vessels;

(11) agents for the treatment of hemorrhage in esophagal venous cancer, cirrhosis or peripheral vessel disease;

(12) agents for the treatment of disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.), etc.;

(13) agents for the treatment of, for example, dementia (e.g., Alzheimer's disease, Alzheimer's senile dementia, vascular/multiple dementia, etc.), schizophrenia, epilepsy, depression, general anxiety disorder, sleeping disorder, multiple sclerosis, etc.;

(14) agents for the treatment of eye disease (e.g., glaucoma, etc.), etc.;

(15) agents for the prevention and treatment of acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet' s disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis, etc.

(16) Furthermore, the antibody of the present invention is also used for healing organ transplantation, burn, wound, alopecia, etc.

(17) The antibody of the present invention is also useful as analgesics for suppression or alleviation of chronic or acute pains (pains accompanied by, e.g., postoperative pain, inflammatory pain, toothache, bone disease (e.g., arthritis, rheumatoid, osteoporosis, etc.)).

The pharmaceutical agent comprising the antibody of the present invention for the treatment and prevention of the aforesaid diseases may be administered orally or parenterally to human or mammal (e.g., rat, rabbit, sheep, swine, bovine, cat, dog, monkey, etc.) as a liquid preparation in its original form, or as a pharmaceutical composition in an appropriate drug form. The dose varies depending on subject to be administered, target disease, conditions, route for administration, etc.; for example, when used for the treatment and prevention of adult patient with neuropathy, the antibody of the present invention is intravenously administered normally in the dose of about 0.01 mg to about 20 mg/kg body weight, preferably about 1.0 to about 10 mg/kg body weigh, and more preferably about 0.1 to about 5 mg per day once to about 5 times a day, preferably once to about 3 times. In parenteral administration in other route and in oral administration, a dose similar to those given above can be administered. Where conditions are serious, the dose may be increased depending on the conditions.

The antibody of the present invention may be administered in itself or as an appropriate pharmaceutical composition. The pharmaceutical composition used for the administration described above contains a pharmacologically acceptable carrier with the aforesaid compounds or salts thereof, a diluent or excipient. Such a composition is provided in the preparation suitable for oral or parenteral administration.

That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration that can be used are injections, suppositories, etc. and the injections include the form of intravenous, subcutaneous, transcutaneous, intramuscular and drip injections. Such injections are prepared by publicly known methods, e.g., by dissolving, suspending or emulsifying the aforesaid antibody or its salts in a sterile aqueous or oily liquid medium. For the aqueous medium for injection, for example, physiological saline and isotonic solutions containing glucose and other adjuvant, etc. are used. Appropriate dissolution aids, for example, alcohol (e.g. ethanol), polyalcohol (e.g. propylene glycol, polyethylene glycol), nonionic surfactant (e.g. polysorbate 80™, HCO-50 (polyoxyethylene (50 mol) adduct of hydrogenated castor oil)) may be used in combination. For the oily solution, for example, sesame oil, soybean oil and the like are used, and dissolution aids such as benzyl benzoate and benzyl alcohol may be used in combination. The thus-prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of 5 to 500 mg per unit dosage form, 5 to 100 mg especially for injections and 10 to 250 mg for other preparations.

Each composition described above may further contain other active components unless formulation with the antibody causes any adverse interaction.

(7) DNA transgenic animal

The present invention provides a non-human mammal bearing DNA encoding the polypeptide of the present invention or the receptor protein of the present invention, which is exogenous (hereinafter abbreviated as the exogenous DNA of the present invention) or its variant DNA (sometimes simply referred to as the exogenous variant DNA of the present invention).

Thus, the present invention provides:

(1) a non-human mammal bearing the exogenous DNA or its variant DNA;
(2) the mammal according to (1), wherein the non-human mammal is a rodent;
(3) the mammal according to (2), wherein the rodent is mouse or rat; and,
(4) a recombinant vector bearing the exogenous DNA of the present invention or its variant DNA and capable of expressing in a mammal.

The non-human mammal bearing the exogenous DNA of the present invention or its variant DNA (hereinafter simply referred to as the DNA transgenic animal of the present invention) can be created by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method etc. In addition, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to create the transgenic animal of the present invention.

Examples of the non-human mammal that can be used include bovine, swine, sheep, goat, rabbits, dogs, cats, guinea pigs, hamsters, rats, mice and the like. Above all, preferred are rodents, especially mice (e.g., C57B1/6 strain, DBA2 strain, etc. for a pure line and for a cross line, B6C3F$_1$ strain, BDF$_1$ strain B6D2F$_1$ strain, BALB/c strain, ICR strain, etc.) or rats (Wistar, SD, etc.), since they are relatively short in ontogeny and life cycle from a standpoint of creating model animals for human disease.

"Mammals" in a recombinant vector that can be expressed in the mammals include the aforesaid non-human mammals and human.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal polypeptide or receptor protein of the present invention and exemplified by DNA that expresses a polypeptide for suppressing the functions of the normal polypeptide or receptor protein of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the polypeptide of the present invention, there are *Escherichia coli*-derived plasmids, *Bacillus subtilis-derived* plasmids, yeast-derived plasmids, bacteriophages such as λ phage, retroviruses such as Moloney leukemia virus, etc., and animal viruses such as vaccinia virus, baculovirus, etc. Of these vectors, *Escherichia coli*-derived plasmids, *Bacillus subtilis-derived* plasmids, or yeast-derived plasmids, etc. are preferably used.

Examples of these promoters for regulating the DNA expression include (1) promoters for DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), polypeptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle, α actin, preproencephalin A, vasopressin, etc. Among them, cytomegalovirus promoters, human polypeptide elongation factor 1α (EF-1α) promoters, human and chicken β actin promoters etc., which protein can highly express in the whole body are preferred.

It is preferred that the vectors described above have a sequence for terminating the transcription of the desired messenger RNA in the DNA transgenic animal (generally termed terminator); for example, a sequence of each DNA derived from viruses and various mammals. SV40 terminator of the simian virus, etc. are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal polypeptide or receptor protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using complementary DNA prepared by a publicly known method from RNA of liver, kidney, thyroid cell or fibroblast origin as a starting material. In addition, an exogenous abnormal DNA can be obtained using complementary DNA prepared by a publicly known method from RNA of human fibroblast origin as a starting material. Alternatively, the translational region for a normal polypeptide translational region obtained by the cell or tissue described above can be made variant by point mutagenesis.

The translational region can be prepared by a conventional DNA engineering technique in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

By obtaining a homozygotic animal having the transfected DNA in both of homologous chromosomes and mating a male and female of the animal, all offspring can be passaged to retain the DNA.

In a non-human mammal bearing the normal DNA of the present invention, the normal DNA of the present invention has expressed to a high level, and may eventually develop the function inactive type inadaptability of the polypeptide of the present invention or the receptor protein of the present invention by accelerating the functions of endogenous normal DNA. Therefore, the animal can be utilized as a pathologic model animal for such a disease. Specifically, using the normal DNA transgenic animal of the present invention, it is possible to elucidate the mechanism of the function inactive type inadaptability of the polypeptide or the receptor protein of the present invention and the pathological mechanism of the disease associated with the receptor protein of the present invention and to determine how to treat the disease.

Further, since a mammal transfected the exogenous normal DNA of the present invention exhibits an increasing symptom of the polypeptide or the receptor protein of the present invention librated, the animal is usable for screening of treatment agent for the disease associated with the polypeptide or the receptor protein of the present invention.

On the other hand, non-human mammal having the exogenous abnormal DNA of the present invention can be passaged under normal breeding conditions as the DNA-bearing animal by confirming the stable retaining of the exogenous DNA via crossing. Further, the exogenous DNA to be subjected can be utilized as a starting material by inserting the DNA into the plasmid described above. The DNA construct with promoter can be prepared with conventional DNA engineering techniques. The transfection of the abnormal DNA of the present invention at the fertilized egg cell stage is preserved to be present in all of the germinal and somatic cells of the mammals to be subjected. The fact that the abnormal DNA of the present invention is present in the germinal cells of the animal after DNA transfection means that all of the offspring of the prepared animal have the abnormal DNA of the present invention in all of the germinal and somatic cells. Such an offspring passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bled to have the DNA.

Since non-human mammal having the abnormal DNA of the present invention may express the abnormal DNA of the present invention at a high level, the animal may be the function inactivation type inadaptability of the polypeptide or the receptor protein of the present invention by inhibiting the function of the endogenous normal DNA and can be utilized as its disease model animal. For example, using the abnormal DNA-transferred animal of the present invention, it is possible to elucidate the mechanism of inadaptability of the polypeptide or the receptor protein of the present invention and to perform to study a method for treatment of this disease.

More specifically, the transgenic animal of the present invention expressing the abnormal DNA of the present invention to a high level is also expected to serve as an experimental model for the elucidation of the mechanism of the functional inhibition (dominant negative effect) of normal polypeptide by the abnormal polypeptide of the present invention in the function inactive type inadaptability of the polypeptide of the present invention or the receptor protein of the present invention.

A mammal bearing the abnormal exogenous DNA of the present invention is also expected to serve for screening a candidate drug for the treatment of the function inactive type inadaptability of the polypeptide of the present invention or the receptor protein of the present invention, since the polypeptide of the present invention or the receptor protein of the present invention is increased in such an animal in its free form. Other potential applications of two kinds of the transgenic animals described above include:

(1) use as a cell source for tissue culture;
(2) elucidation of the relation to a polypeptide that is specifically expressed or activated by the polypeptide of the present invention or the receptor protein of the present invention, by direct analysis of DNA or RNA in tissue of the DNA transgenic animal of the present invention or by analysis of the polypeptide tissue expressed by the DNA;
(3) research in the function of cells derived from tissues that are cultured usually only with difficulty, using cells of tissue bearing the DNA cultured by a standard tissue culture technique;
(4) screening for a drug that enhances the functions of cells using the cells described in (3) above; and,
(5) isolation and purification of the variant polypeptide of the present invention and preparation of an antibody thereto.

Furthermore, clinical conditions of a disease associated wit the polypeptide of the present invention or the receptor protein of the present invention, including the function inactive type inadaptability of the polypeptide of the polypeptide of the present invention or the receptor protein of the present invention can be determined using the DNA transgenic animal of the present invention. In addition, pathological findings on each organ in a disease model associated with the polypeptide of the present invention or the receptor protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve as identification of cells capable of producing the polypeptide of the present invention or the receptor protein of the present invention, and as studies on association with apoptosis, differentiation or propagation or on the mechanism of signal transduction in these properties to inspect any abnormality therein. Thus the DNA transgenic animal of the present invention can provide an effective research material for the polypeptide of the present invention or the receptor protein of the present invention and for elucidating the function and effect thereof.

To develop a therapeutic drug for the treatment of diseases associated with the polypeptide of the present invention or the receptor protein of the present invention, including the function inactive type inadaptability of the polypeptide of the present invention or the receptor protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the polypeptide of the present invention or the receptor protein of the present invention, using the DNA transgenic animal of the present invention or a vector capable of expressing the exogenous DNA of the present invention.

(8) Knockout animal

The present invention provides a non-human mammal embryonic stem cell bearing the DNA of the present invention inactivated and a non-human mammal deficient in expressing the DNA of the present invention.

Thus, the present invention provides:

(1) a non-human embryonic stem cell in which the DNA of the present invention is inactivated;
(2) an embryonic stem cell according to (1), wherein the DNA is inactivated by introducing a reporter gene (e.g., β-galactosidase gene derived from *Escherichia coli);*
(3) an embryonic stem cell according to (1), which is resistant to neomycin;
(4) an embryonic stem cell according to (1), wherein the non-human mammal is a rodent;
(5) an embryonic stem cell according to (4), wherein the rodent is mouse;
(6) a non-human mammal deficient in expressing the DNA of the present invention, wherein the DNA of the present invention is inactivated;
(7) a non-human mammal according to (5), wherein the DNA is inactivated by inserting a reporter gene (e.g., β-galactosidase derived from *Escherichia coli*) therein and the reporter gene is capable of being expressed under control of a promoter for the DNA of the present invention;
(8) a non-human mammal according to (6), which is a rodent;
(9) a non-human mammal according to (8), wherein the rodent is mouse; and,
(10) a method for screening a compound that accelerates or inhibits the promoter activity for the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the polypeptide of the present invention or the receptor protein of the present invention (hereinafter sometimes referred to as the knockout DNA of the present invention) by substantially inactivating the activities of the polypeptide of the present invention or the receptor protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the subject animal by, e.g., homologous recombination, a DNA sequence which terminates gene transcription (e.g., polyA additional signal, etc.) in the intron between exons to, thus inhibit the synthesis of complete messenger RNA and eventually destroy the gene (hereinafter simply referred to as targeting vector). The thus-obtained ES cells to Southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may be originally established in accordance with a modification of the known method by Evans and Kaufman supra. For example, in the case of mouse ES cells, currently it is common practice to use ES cells of the 129 strain. However, since their immunological background is obscure, the C57BL/6 mouse or the BDF1 mouse (F1 hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF1 mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is also desirable that sexes be identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1-10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on fleshly prepared feeder cells. This passage is normally conducted every 1 to 3 days; it is desirable that cells be observed at passage and cells found to be morphologically abnormal in culture, if any, be abandoned.

Where ES cells are allowed to reach a high density in mono-layers or to form cell aggregates in suspension under appropriate conditions, they will spontaneously differentiate to various cell types, for example, pariental and visceral muscles, cardiac muscle or the like (M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985). The cells deficient in expression of the DNA of the present invention, which are obtainable from the differentiated ES cells of the present invention are useful for studying the functions of the polypeptide of the present invention or the receptor protein of the present invention cytologically or molecular biologically.

The non-human mammal deficient in expression of the DNA of the present invention can be identified from a normal animal by measuring the mRNA amount in the subject animal by a publicly known method, and indirectly comparing the degrees of expression.

As the non-human mammal, the same examples supra apply.

With respect to the non-human mammal deficient in expression of the DNA of the present invention, the DNA of the present invention can be made knockout by transfecting a targeting vector, prepared as described above, to non-human mammal embryonic stem cells or oocytes thereof, and conducting homologous recombination in which a targeting vector DNA sequence, wherein the DNA of the present invention is inactivated by the transfection, is replaced with the DNA of the present invention on a chromosome of a non-human mammal embryonic stem cell or embryo thereof.

The knockout cells with the DNA of the present invention disrupted can be identified by Southern hybridization analysis with a DNA fragment on or near the DNA of the present invention as a probe, or by PCR analysis using a DNA sequence on the targeting vector and another DNA sequence which is not included in the targeting vector as primers. When non-human mammalian embryonic stem cells are used, a cell line wherein the DNA of the present invention is inactivated by homologous recombination is cloned; the resulting cloned cell line is injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the peptide of the present invention. The individuals deficient in homozygous expression of the polypeptide of the present invention or the receptor protein of the present invention can be obtained from offspring of the intercross between the heterozygotes.

When an oocyte or egg cell is used, a DNA solution may be injected, e.g., to the prenucleus by microinjection thereby to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals in which the DNA of the present invention is rendered knockout permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell in which the DNA of the present invention is inactivated is very useful for preparing a non-human mammal deficient in expression of the DNA of the present invention.

Since the non-human mammal in which the DNA of the present invention is inactivated lacks various biological activities derived from the polypeptide of the present invention or the receptor protein of the present invention, such an animal can be a disease model suspected of inactivated biological activities of the polypeptide of the present invention or the receptor protein of the present invention and thus, offers an effective study to investigate causes for and therapy for these diseases.

(8a) Method for screening of compounds having therapeutic/prophylactic effects for diseases caused by deficiency, damages, etc. of the DNA of the present invention

The non-human mammal deficient in expression of the DNA of the present invention can be employed for screening of compounds having therapeutic/prophylactic effects for diseases caused by deficiency, damages, etc. of the DNA of the present invention.

That is, the present invention provides a method for screening of a compound having therapeutic/prophylactic effects for diseases caused by deficiency, damages, etc. of the DNA of the present invention, which comprises administering a test compound to the non-human mammal deficient in expression of the DNA of the present invention and observing and measuring a change occurred in the animal.

As the non-human mammal deficient in expression of the DNA of the present invention which can be employed for the screening method, the same examples as given hereinabove apply.

Examples of the test compounds include peptides, proteins, non-peptide compounds, synthetic compounds, fermentation products, cell extracts, vegetable extracts, animal tissue extracts, blood plasma and the like and these compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an index to assess the therapeutic/prophylactic effects of the test compound.

For treating an animal to be test with a test compound, for example, oral administration, intravenous injection, etc. are applied and the treatment is appropriately selected depending upon conditions of the test animal, properties of the test compound, etc. Further, an amount of administration for a test compound can be selected depending on the administration route, nature of the test compound and the like.

For example, the non-human mammal deficient in expression of the DNA of the present invention is subjected to a sugar loading treatment, a test compound is administered before or after the sugar loading treatment and, blood sugar level, body weight change, etc. of the animal is measured with passage of time, in the case of screening a compound having a therapeutic/prophylactic effect for diseases such as hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer, rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet' s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc.; or a compound having a therapeutic and prophylactic effect for macular edema cystoid; and furthermore, a compound useful as a therapeutic and prophylactic effect as:

(1) therapeutic agents for tumors such as acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor, etc.;

(2) therapeutic agents for insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) agents for improving hyperinsulinism or for the treatment of obesity, bulimia, etc. caused by the suppression of appetite;

(4) therapeutic agents for acute pancreatitis, chronic pancreatitis, pancreatic/intestinal fistula, hemorrhagic ulcer, peptic ulcer, gastritis, hyperchylia, reflux esophagitis, etc.;

(5) agents for alleviating various conditions accompanied by Helicobacter pylori bacterial infections (e.g., an agent for suppressing accentuated gastrin secretion, etc.);

(6) agents for suppressing secretion of amylase accompanied by endoscopic cholangio pancreatography and for the postoperative treatment in pancreas surgery;

(7) agents for the treatment of diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro-endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia, etc.;

(8) agents for the treatment of Dumping syndrome, hypersensitive colitis, Crohn's disease, inflammatory bowel disease, etc.;

(9) agents for the treatment of tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-$\alpha$, $\beta$ and $\gamma$, interleukin-2, etc.);

(10) agents for the prevention and treatment of hypertrophic cardiomyopathy, arteriosclerosis, valvular disease, myocardial infarction (especially myocardial infarction after percutaneous transluminal angioplasty) or regeneration of blood vessels;

(11) agents for the treatment of hemorrhage in esophagal venous cancer, cirrhosis or peripheral vessel disease;

(12) agents for the treatment of disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.), etc.;

(13) agents for the treatment of, for example, dementia (e.g., Alzheimer's disease, Alzheimer's senile dementia, vascular/multiple dementia, etc.), schizophrenia, epilepsy, depression, general anxiety disorder, sleeping disorder, multiple sclerosis, etc.;

(14) agents for the treatment of eye disease (e.g., glaucoma, etc.), etc.;

(15) agents for the prevention and treatment of acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet' s disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis, etc.;

(16) further use in healing organ transplantation, burn, wound, alopecia, etc.;

(17) analgesics for suppression or alleviation of chronic or acute pains (pains accompanied by, e.g., postoperative pain, inflammatory pain, toothache, bone disease (e.g., arthritis, rheumatoid, osteoporosis, etc.)).

In the screening method *supra,* when a test compound is administered to an animal to be tested and found to reduce the blood sugar level of the animal to at least about 10%, preferably at least about 30% and more preferably at least about 50%, the test compound can be selected to be a compound having a therapeutic and prophylactic effect for the diseases *supra.*

The compound obtained using the above screening method is a compound selected from the test compounds described above and exhibits a therapeutic and prophylactic effect for the diseases caused by deficiencies, damages, etc. of the polypeptide of the present invention or the receptor protein of the present invention. Therefore, the compound can be employed as a safe and low toxic drug for the treatment and prevention of these diseases. Furthermore, compounds derived from such a compound obtained by the screening *supra* can be likewise employed.

The compound obtained by the screening above may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical composition comprising the compound obtained by the above screening method or salts thereof may be manufactured in a manner similar to the method for preparing the composition comprising the polypeptide of the present invention described hereinabove.

Since the pharmaceutical composition thus obtained is safe and low toxic, it can be administered to human and another mammal (e.g., rat, mouse, guinea pig, rabbit, sheep, swine, bovine, horse, cat, dog, monkey, etc.).

Although the amount of the compound or its salt to be administered varies depending upon particular disease, subject to be administered, route of administration, etc. in general, for oral administration to an adult (as 60 kg body weight), the compound is administered in an amount of about 0.1 mg/day to about 100 mg/day, preferably about 1.0 mg/day to about 50 mg/day, more preferably about 1.0 mg to about 20 mg. For parenteral administration to an adult (as 60 kg body weight), it is advantageous to administer the composition in the form of an injectable preparation in an amount of about 0.01 mg/day to about 30 mg/day, preferably about 0.1 mg/day to about 20 mg/day, more preferably about 0.1 mg/day to about 10 mg/day, though the single dosage varies depending upon particular subject, particular disease, etc. As for other animals, the composition can be administered in the above amount with converting it into that for the body weight of 60 kg.

(8b) Method for screening a compound that accelerates or inhibits the activities of a promoter to the DNA of the present invention

The present invention provides a method for screening a compound that accelerates or inhibits the activities of a promoter to the DNA of the present invention or salts thereof, which comprises administering a test compound to a non-human mammal deficient in expression of the DNA of the present invention and detecting expression of the reporter gene.

In the screening method *supra,* the non-human mammal deficient in expression of the DNA of the present invention is selected from the aforesaid non-human mammal deficient in expression of the DNA of the present invention, as an animal in which the DNA of the present invention is inactivated by introducing a reporter gene and the reporter gene is expressed under control of a promoter to the DNA of the present invention.

The same examples of the test compound apply to specific compounds used for the screening.

As the reporter gene, the same specific examples apply to this screening method. Preferably employed are β-galactosidase (lacZ), soluble alkaline phosphatase gene, luciferase gene and the like.

Since a reporter gene is present under control of a promoter to the DNA of the present invention in the non-human mammal deficient in expression of the DNA of the present invention wherein the DNA of the present invention is substituted with the reporter gene, the activity of the promoter can be detected by tracing expression of a substance encoded by the reporter gene.

When a part of the DNA region encoding the polypeptide of the present invention or the receptor protein of the present invention is substituted with, e.g., β-galactosidase gene (lacZ) derived from *Escherichia coli*, β-galactosidase is expressed in a tissue where the polypeptide of the present invention or the receptor protein of the present invention should originally be expressed, instead of the polypeptide or receptor protein of the present invention. Thus, the state of expression of the polypeptide or the receptor protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the polypeptide of the present invention or the receptor protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method *supra* are compounds that are selected from the test compounds described above and that accelerate or inhibit the promoter activity to the DNA of the present invention.

The compound obtained by the screening method above may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Since the compounds or salts thereof that accelerate or inhibit the promoter activity to the DNA of the present invention can accelerate or inhibit the expression of the polypeptide of the present invention or the receptor protein of the present invention or can accelerate or inhibit the functions of the polypeptide of the present invention or the receptor protein of the present invention, they are useful as safe and low toxic drugs for the treatment/prevention of diseases such as hypertension, autoimmune disease, heart failure, cataract, glaucoma, acute bacterial meningitis, acute myocardial infarction, acute pancreatitis, acute viral encephalitis, adult respiratory distress syndrome, alcoholic hepatitis, Alzheimer's. disease, asthma, arteriosclerosis, atopic dermatitis, bacterial pneumonia, bladder cancer, fracture, breast cancer, bulimia, polyphagia, burn healing, uterine cervical cancer, chronic lymphocytic leukemia, chronic myelogenous leukemia, chronic pancreatitis, liver cirrhosis, cancer of the colon and rectum (colon cancer, rectal cancer), Crohn's disease, dementia, diabetic complications, diabetic nephropathy, diabetic neuropathy, diabetic retinopathy, gastritis, Helicobacter pylori bacterial infectious disease, hepatic insufficiency, hepatitis A, hepatitis B, hepatitis C, hepatitis, herpes simplex virus infectious disease, varicellazoster virus infectious disease, Hodgkin's disease, AIDS infectious disease, human papilloma virus infectious disease, hypercalcemia, hypercholesterolemia, hyperglyceridemia, hyperlipemia, infectious disease, influenza infectious disease, insulin dependent diabetes mellitus (type I), invasive staphylococcal infectious disease, malignant melanoma, cancer metastasis, multiple myeloma, allergic rhinitis, nephritis, non-Hodgkin's lymphoma, insulin-independent diabetes mellitus (type II), non-small cell lung cancer, organ transplantation, arthrosteitis, osteomalacia, osteopenia, osteoporosis, ovarian cancer, Behcet' s disease of bone, peptic ulcer, peripheral vessel disease, prostatic cancer, reflux esophagitis, renal insufficiency, rheumatoid arthritis, schizophrenia, sepsis, septic shock, severe systemic fungal infectious disease, small cell lung cancer, spinal injury, stomach cancer, systemic lupus erythematosus, transient cerebral ischemia, tuberculosis, cardiac valve failure, vascular/multiple infarction dementia, wound healing, insomnia, arthritis, pituitary hormone secretion disorder, pollakiuria, uremia, neurodegenerative disease, etc.

In addition, the compounds or salts thereof that accelerate or inhibit the promoter activity to the DNA of the present invention can also be used as a safe and low toxic therapeutic/prophylactic agent for macular edema cystoid.

Moreover, the compounds or salts thereof that accelerate or inhibit the promoter activity to the DNA of the present invention are useful as safe and low toxic therapeutic/prophylactic agents for diseases, which are specifically given below:

(1) therapeutic agents for tumors such as acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid tumor, etc.;

(2) therapeutic agents for insulin-dependent or insulin-independent diabetes mellitus, or various diseases associated with the diabetes, i.e., diabetic complications (e.g., diabetic retinopathy, diabetic nephropathy, diabetic neuropathy, Down's syndrome, orthostatic hypotension, etc.);

(3) agents for improving hyperinsulinism or for the treatment of obesity, bulimia, etc. caused by the suppression of appetite;

(4) therapeutic agents for acute pancreatitis, chronic pancreatitis, pancreatic/intestinal fistula, hemorrhagic ulcer, peptic ulcer, gastritis, hyperchylia, reflux esophagitis, etc.;

(5) agents for alleviating various conditions accompanied by Helicobacter pylori bacterial infections (e.g., an agent for suppressing accentuated gastrin secretion, etc.);

(6) agents for suppressing secretion of amylase accompanied by endoscopic cholangio pancreatography and for the postoperative treatment in pancreas surgery;

(7) agents for the treatment of diarrhea caused by reduced absorption or accentuated secretion in small intestine or abnormal motility of digestive tract (Short bowel syndrome, etc.), diarrhea caused by drugs in chemotherapy of cancer, etc., diarrhea caused by congenital small intestine atrophy, diarrhea caused by neuro-endocrinal tumor such as VIP-producing tumor, etc., diarrhea caused by AIDS, diarrhea caused by graft-versus-host reaction accompanied by spinal transplant, etc., diarrhea caused by diabetes mellitus, diarrhea caused by blocking nervous plexus in the abdominal cavity, diarrhea caused by systemic screlosis, diarrhea caused by eosinophilia, etc.;

(8) agents for the treatment of Dumping syndrome, hypersensitive colitis, Crohn's disease, inflammatory bowel disease, etc.;

(9) agents for the treatment of tumor or cancer (e.g., thyroid cancer, colon cancer, breast cancer, prostatic cancer, small cell lung cancer, non-small cell lung cancer, pancreatic cancer, gastric cancer, bile duct cancer, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (e.g., leukemia/chronic lymphoid leukemia of basophil leukocyte, chronic myeloid leukemia, Hodgkin's disease, non-Hodgkin's lymphoma, etc.), these agents may also be used alone or in combination with other carcinostatic agents (e.g., tamoxifen, LHRH agonist, LHRH antagonist, interferon-$\alpha$, $\beta$ and $\gamma$, interleukin-2, etc.);

(10) agents for the prevention and treatment of hypertrophic cardiomyopathy, arteriosclerosis, valvular disease, myocardial infarction (especially myocardial infarction after percutaneous transluminal angioplasty) or regeneration of blood vessels;

(11) agents for the treatment of hemorrhage in esophagal venous cancer, cirrhosis or peripheral vessel disease;

(12) agents for the treatment of disease accompanied by regulation of secretion of physiologically active substances acting on the immune system, such as systemic or regional inflammation (e.g., multiple arthritis, rheumatoid arthritis, psoriasis, sunburn, eczema, allergy (e.g., asthma, atopic dermatitis, allergic rhinitis, etc.), etc.);

(13) agents for the treatment of, for example, dementia (e.g., Alzheimer's disease, Alzheimer's senile dementia, vascular/multiple dementia, etc.), schizophrenia, epilepsy, depression, general anxiety disorder, sleeping disorder, multiple sclerosis, etc.;

(14) agents for the treatment of eye disease (e.g., glaucoma, etc.), etc.;

(15) agents for the prevention and treatment of acute bacterial meningitis, acute viral encephalitis, adult respiratory distress syndrome, bacterial pneumonia, severe systemic fungal infectious disease, tuberculosis, spinal injury, bone fracture, hepatic insufficiency, pneumonia, alcoholic hepatitis, hepatitis A, hepatitis B, hepatitis C, AIDS infectious disease, human papilloma virus infectious disease, influenza infectious disease, cancer metastasis, multiple myeloma, osteomalacia, osteoporosis, Behcet' s disease of bone, nephritis, renal insufficiency, sepsis, septic shock, hypercalcemia, hypercholesterolemia, hyperglyceridemia, hyperlipemia, systemic lupus erythematosus, transient cerebral ischemia, alcoholic hepatitis, etc.;

(16) further use in healing organ transplantation, burn, wound, alopecia, etc.;

(17) analgesics for suppression or alleviation of chronic or acute pains (pains accompanied by, e.g., postoperative pain, inflammatory pain, toothache, bone disease (e.g., arthritis, rheumatoid, osteoporosis, etc.)). In addition, compound derived from the compounds obtained by the screening above may be likewise employed.

A pharmaceutical composition comprising the compounds or salts thereof obtained by the screening method *supra* may be manufactured in a manner similar to the method for preparing the composition comprising the polypeptide of the present invention described hereinabove.

Since the pharmaceutical composition thus obtained is safe and low toxic, it can be administered to human or another mammal (e.g., rat, mouse, guinea pig, rabbit, sheep, swine, bovine, horse, cat, dog, monkey, etc.).

The dose of the compound or salts thereof varies depending on target disease, subject to be administered, method for administration, etc.; for example, in oral administration of the compound that accelerates the promoter activity to the DNA of the present invention, the dose is normally about 0.1 to about 100 mg, preferably about 1.0 to about 50 mg, more preferably about 1.0 to about 20 mg per day for adult (as 60 kg body weight). In parenteral administration, the single dose varies depending on subject to be administered, target disease, etc. but it is advantageous to administer, for example, the compound that accelerates the functions of the polypeptide of the present invention intravenously at a daily dose of about 0.01 to about 30 mg, preferably about 0.1 to about 20 mg, more preferably about 0.1 to about 10 mg for adult (as 60 kg body weight). For other animal species, the corresponding dose as converted per 60 kg weight can be administered.

Turning to the compound that inhibits the promoter activity to the DNA of the present invention when it is orally administered, the dose is normally about 0.1 to about 100 mg, preferably about 1.0 to about 50 mg, more preferably about 1.0 to about 20 mg per day for adult (as 60 kg body weight). In parenteral administration, the single dose varies depending on subject to be administered, target disease, etc. When the compound that inhibits the promoter activity to the DNA of the present invention is administered to an adult (as 60 kg body weight) generally in the form of injection, it is advantageous to administer the compound intravenously at a daily dose of about 0.01 to about 30 mg, preferably about 0.1 to about 20 mg, more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that accelerates or inhibits the activity of a promoter to the DNA of the present invention and can greatly contribute to the elucidation of causes for various diseases suspected of deficiency in expression of the DNA of the present invention and for the development of prophylactic/therapeutic agent for these diseases.

Furthermore, a so-called transgenic animal (gene transferred animal) can be prepared by using DNA containing a promoter region of the polypeptide of the present invention or the receptor protein of the present invention, ligating genes encoding various proteins downstream and injecting the same into oocyte of an animal. It is then possible to synthesize the polypeptide or protein therein specifically and study its activity in vivo. When an appropriate reporter gene is ligated to the promoter site above and a cell line that express the gene is established, the resulting system can be utilized for exploring a low molecular compound having the action of specifically promoting or inhibiting the in vivo productivity of the polypeptide of the present invention or the receptor protein of the present invention, per se.

(9) Identification of receptor to the polypeptide of the present invention

The receptor to the polypeptide of the present invention can be identified as follows. Most receptors for the physiologically active peptides are of seven-transmembrane type and presently many orphan receptors, which ligands are unknown, are reported. Thus, specific receptors can be identified by expressing these orphan receptors in appropriate cells such as CHO cells, HEK293 cells, etc. and adding the polypeptide of the present invention to the expressed receptors to examine if such a cell stimulating activity as inducing a specific signal transduction is exhibited. Furthermore, a gene encoding the receptor can be isolated by inserting genome or cDNA library into appropriate animal cells and adding thereto a radioisotope-labeled polypeptide of the present invention to examine its binding.

Since a gene encoding the physiologically active peptide is characterized often by repeating a sequence motif of the peptide, the present invention further provides a method for identification of an unknown physiologically active peptide or its amides or esters, or salts thereof, by utilizing the characteristic property and also provides a physiologically active peptide obtained by the method, its amides or esters, or salts thereof.

Specific examples of the sequence motif possessed by the physiologically active peptide are RFG (R/K) sequence or RSG (R/K) sequence or RLG (R/K) sequence which is characteristic of the polypeptide of the present invention bearing an RF amide, RS amide or RL amide structure, and a base sequence encoding the amino acid sequence. The DNA sequence capable of encoding such a short amino acid sequence appears accidentally with a considerably high frequency also in those other than the DNA sequence of the physiologically active peptide. By exploring a sequence characterized by repeating such a sequence, DNA encoding a physiologically active peptide can be discovered in a high probability.

More specifically, the desired gene can be obtained by retrieval of database using as a probe the RFG(R/K) sequence or RSG(-R/K) sequence or RLG(K/R) sequence or a sequence containing the amino acid sequence and a sequence containing the base sequence encoding the same. Examples of the probe include:

```
RFGK: 5'-(C/A)G(A/C/G/T)TT(T/C)GG(A/C/G/T)AA(A/G)-3'
(SEQ ID NO:20)


RFGR: 5'-(C/A)G(A/C/G/T)TT(T/C)GG(A/C/G/T)G(A/C/G/T)-3'
(SEQ ID NO:21)


RsGK: 5'-(C/A)G(A/C/G/T)(A/T)(C/G)
(A/C/G/T)GG(A/C/G/T)AA(A/G)-3' (SEQ ID NO:22)


RSGR: 5'-(C/A)G(A/C/G/T)(A/T)(C/G)
(A/C/G/T)(A/C)G(A/C/G/T)AA(A/G)-3' (SEQ ID NO:23)


RLGK: 5'-(C/A)G(A/C/G/T)(T/C)T(A/C/G/T)AA(A/G)-3' (SEQ
ID NO:24)


RLGR: 5'-
(C/A)G(A/C/G/T)(T/C)T(A/C/G/T)GG(A/C/G/T)(A/C)G(A/C/G/T
)-3' (SEQ ID NO:25)
```

and the like, as the DNA sequence corresponding to RFG(K/R), RSG(K/R) and RLG(K/R).

The desired gene may also be obtained by screening cDNA or genomic library using the sequence motif above. Moreover, mRNA of the desired gene is purified by using the probes *supra* as in a gene trapper to acquire cDNA from the mRNA purified. Further by using other sequence motif (an amino acid sequence repeatedly encoded by the gene or a base sequence encoding the amino acid sequence), these probes may also be used for identification of a physiologically active peptide having other than the RF amide, RS amide or RL amide structure.

The peptide having the RF amide, RS amide or RL amide structure possesses a common structure of RF amide, RS amide or RL amide at the C-terminal region of the peptide. It is thus possible to explore a peptide having an unknown RF amide, RS amide or RL amide structure, using an antibody containing the RF amide, RS amide or RL amide structure. Most receptors to the peptide having the RF amide, RS amide or RL amide structure are of seven-transmembrane type. Therefore, a ligand to orphan receptor can be determined by using an anti-RF amide antibody, anti-RS amide antibody or anti-RL amide antibody, wherein a condensed or fractionated animal tissue extract is added to cells capable of expressing an orphan receptor with the ligand being not determined. Since many peptides that contain a common structure other than those having the RF amide, RS amide or RL amide structure are present, this method is applicable also to peptides other than those having the RF amide, RS amide or RL amide structure.

(10) Determination of a ligand (agonist) to the receptor protein of the present invention

The receptor protein of the present invention is useful as a reagent for searching and determining a ligand (agonist) to the receptor protein of the present invention and salts thereof.

That is, the present invention provides a method for determining a ligand to the receptor protein of the present invention, which comprises bringing the receptor protein of the present invention in contact with a test compound.

Examples of compounds to be tested include publicly known ligands (e.g., angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotrienes, pancreastatin, prostaglandins, thromboxane, adenosine, adrenaline, $\alpha$ and $\beta$-chemokines (e.g., IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamin, neurotensin, TRH, pancreatic polypeptide, galanin, etc.) as well as other substances, for example, tissue extracts and cell culture supernatants from human and mammals (e.g., mice, rats, swine, bovine, sheep, monkeys, etc.). For example, the tissue extract or cell culture supernatant is added to the receptor protein of the present invention and fractionated while assaying the cell stimulating activities to finally give a single ligand.

In more detail, the method for determining a ligand of the present invention comprises determining compounds (e.g., peptides, proteins, non-peptide compounds, synthetic compounds, fermentation products, etc.) or salts thereof that bind to the receptor protein of the present invention to provide cell stimulating activities (e.g., the activities that accelerate or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.), using the receptor of the present invention, or using the constructed recombinant receptor protein expression system in the receptor binding assay.

The method for determining a ligand of the present invention is characterized, for example, by measurement of the amount of the test compound bound to the receptor protein of the present invention or the cell-stimulating activities, etc., when the test compound is brought in contact with the receptor protein of the present invention.

More specifically, the present invention provides the following:

(1) A method for determining a ligand to the receptor protein of the present invention which comprises bringing a labeled test compound in contact with the receptor protein of the present invention and measuring the amount of the labeled test compound bound to the receptor protein;

(2) A method for determining a ligand to the receptor protein of the present invention which comprises bringing a labeled test compound in contact with cells containing the receptor protein of the present invention or with a membrane fraction of the cells and measuring the amount of the labeled test compound bound to the cells or the membrane fraction;

(3) A method for determining a ligand to the receptor protein of the present invention which comprises culturing a transformant containing the DNA encoding the receptor protein of the present invention, bringing a labeled test compound in contact with the receptor protein expressed on the cell membrane by said culturing, and measuring the amount of the labeled test compound bound to the expressed receptor protein;

(4) A method for determining a ligand to the receptor protein of the present invention which comprises bringing a test compound in contact with cells containing the receptor protein of the present invention and measuring the receptor protein-mediated cell stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.); and,

(5) A method for determining a ligand to the receptor protein of the present invention which comprises culturing a transformant containing DNA encoding the receptor protein of the present invention, bringing a labeled test compound in contact with the receptor protein expressed on the cell membrane by said culturing, and measuring the receptor protein-mediated cell stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.).

In particular, it is preferred to perform the methods (1) to (3) described above, thereby to confirm that a test compound can bind to the receptor protein of the present invention, followed by the methods (4) and (5) described above.

Any protein exemplified to be usable as the receptor protein of the present invention can be used for determining ligands. However, the receptor protein that is abundantly expressed using animal cells is appropriate.

The receptor protein of the present invention can be manufactured by the method for expression described above, preferably by expressing DNA encoding the receptor protein in mammalian or insect cells. DNA fragments encoding the desired portion of the protein include, but are not limited to, complementary DNA. For example, gene fragments or synthetic DNA may also be used. For introducing a DNA fragment encoding the receptor protein of the present invention into host animal cells and efficiently expressing the same, it is preferred to insert the DNA fragment downstream the polyhedrin promoter of nuclear polyhedrosis virus (NPV), which is a baculovirus having insect hosts, an SV40-derived promoter, a retrovirus promoter, a metallothionein promoter, a human heat shock promoter, a cytomegalovirus promoter, an SR $\alpha$ promoter or the like. The amount and quality of the receptor expressed can be determined by a publicly known method. For example, this determination can be made by the method described in the literature (Nambi, P. et al., J. Biol. Chem., Vol. 267, pp. 19555-19559 (1992)).

Accordingly, the subject containing the receptor protein in the method for determining the ligand may be the receptor protein purified by publicly known method, cells containing the receptor protein or membrane fraction of such cells.

Where cells containing the receptor protein of the present invention are used in the method of the present invention for determination of ligands, the cells may be fixed using glutaraldehyde, formalin etc. The fixation can be made by a publicly known method.

The cells containing the receptor protein of the present invention are host cells that have expressed the receptor protein of the present invention, which host cells include Escherichia coli, Bacillus subtilis, yeast, insect cells, animal cells and the like.

The cell membrane fraction is a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The amount of the receptor protein in the cells containing the receptor protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (1) through (3) for determination of a ligand to the receptor protein of the present invention, an appropriate receptor fraction and a labeled test compound are required.

The receptor protein fraction is preferably a fraction of naturally occurring receptor protein or a recombinant receptor fraction having an activity equivalent to that of the natural protein. Herein, the term "equivalent activity" is intended to mean a ligand binding activity, a signal transduction activity or the like that is equivalent to that possessed by naturally occurring receptor proteins.

Preferred examples of labeled test compounds include angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amylin, bradykinin, CGRP (calcitonin gene-related peptide), leukotrienes, pancreastatin, prostaglandins, thromboxane, adenosine, adrenaline, $\alpha$ and $\beta$-chemokines (e.g., IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamin, neurotensin, TRH, pancreatic polypeptide, galanin, etc.), which are labeled with [$^3$H], [$^{125}$I], [$^{14}$C], [$^{35}$S], etc.

More specifically, the ligand to the receptor protein of the present invention is determined by the following procedures. First, a standard receptor preparation is prepared by suspending cells containing the receptor protein of the present invention or the membrane fraction thereof in a buffer appropriate for use in the determination method. Any buffer can be used so long as it does not interfere with ligand-receptor binding, such buffers including a phosphate buffer or a Tris-HCl buffer having pH of 4 to 10 (preferably pH of 6 to 8). For the purpose of minimizing non-specific binding, a surfactant such as CHAPS, Tween-80™ (manufactured by Kao-Atlas Inc.), digitonin or deoxycholate, and various proteins such as bovine serum albumin or gelatin, may optionally be added to the buffer. Further for the purpose of suppressing the degradation of the receptor or ligand by a protease, a protease inhibitor such as PMSF, leupeptin, E-64 (manufactured by Peptide Institute, Inc.) and pepstatin may also be added. A given amount (5,000 to 500,000 cpm) of the test compound labeled with [$^3$H], [$^{125}$I], [$^{14}$C], [$^{35}$S] or the like is added to 0.01 ml to 10 ml of the receptor solution. To determine the amount of non-specific binding (NSB), a reaction tube containing an unlabeled test compound in a large excess is also provided. The reaction is carried out at approximately 0 to 50°C, preferably about 4 to 37°C for about 20 minutes to about 24 hours, preferably about 30 minutes to 3 hours. After completion of the reaction, the reaction mixture is filtrated through glass fiber filter paper, etc. and washed with an appropriate volume of the same buffer. The residual radioactivity in the glass fiber filter paper is then measured by means of a liquid scintillation counter or $\gamma$-counter. A test compound exceeding 0 cpm in count obtained by subtracting nonspecific binding (NSB) from the total binding (B) (B minus NSB) may be selected as a ligand (agonist) to the receptor protein of the present invention.

The method (4) or (5) above for determination of a ligand to the receptor protein of the present invention can be performed as follows. The receptor protein-mediated cell-stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.) may be determined by a publicly known method, or using an assay kit commercially available. Specifically, cells containing the receptor protein are first cultured on a multi-well plate, etc. Prior to the ligand determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance for the cell-stimulating activity (e.g., arachidonic acid) due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production can then be detected.

The kit of the present invention for determination of the ligand that binds to the receptor protein of the present invention comprises the receptor protein of the present invention, cells containing the receptor protein of the present invention, or the membrane fraction of the cells containing the receptor protein of the present invention.

Examples of the ligand determination kit of the present invention are given below.

1. Reagents for determining ligands

(1) Buffers for assay and washing

Hanks' Balanced Salt Solution (manufactured by Gibco Co.) supplemented with 0.05% bovine serum albumin (Sigma Co.).

The solution is sterilized by filtration through a 0.45 μm filter and stored at 4°C. Alternatively, the solution may be prepared at use.

(2) Standard G protein receptor protein

CHO cells on which the receptor protein of the present invention has been expressed are subjected to passage culture in a 12-well plate in a density of $5 \times 10^5$ cells/well followed by culturing at 37°C under 5% $CO_2$ and 95% air for two days.

(3) Labeled test compounds

Compounds labeled with commercially available [3H], [125I], [14C], [35S], etc. or compounds labeled by appropriate methods.

An aqueous solution of the compound is stored at 4°C or -20°C. The solution is diluted to 1 μM with an assay buffer at use. A sparingly water-soluble test compound is dissolved in dimethylformamide, DMSO, methanol, etc.

(4) Non-labeled compounds

A non-labeled form of the same compound as the labeled compound is prepared in a concentration 100 to 1,000-fold higher than that of the labeled compound.

2. Method for assay

(1) CHO cells expressing the receptor protein of the present invention are cultured in a 12-well culture plate. After washing twice with 1 ml of an assay buffer, 490 μl of the assay buffer is added to each well.

(2) After 5 μl of the labeled test compound is added, the resulting mixture is incubated at room temperature for an hour. To determine the non-specific binding, 5 μl of the non-labeled compound is added to the system.

(3) The reaction mixture is removed and the wells are washed 3 times with 1 ml of washing buffer. The labeled test compound bound to the cells is dissolved in 0.2N NaOH-1% SDS and then mixed with 4 ml of liquid scintillator A (manufactured by Wako Pure Chemical Industries, Ltd.).

(4) The radioactivity is measured using a liquid scintillation counter (manufactured by Beckman Co.).

The ligands that bind to the receptor protein of the present invention include substances specifically present in the brain, pituitary gland and pancreas. Examples of such ligands are angiotensin, bombesin, canavinoid, cholecystokinin, glutamine, serotonin, melatonin, neuropeptide Y, opioids, purines, vasopressin, oxytocin, PACAP, secretin, glucagon, calcitonin, adrenomedulin, somatostatin, GHRH, CRF, ACTH, GRP, PTH, VIP (vasoactive intestinal peptide), somatostatin, dopamine, motilin, amylin, bradykinin, CGRP (calcitonin gene-related peptide), leukotriens, pancreastatin, prostaglandins, thromboxane, adenosine, adrenaline, $\alpha$ and $\beta$-chemokines (e.g. IL-8, GRO$\alpha$, GRO$\beta$, GRO$\gamma$, NAP-2, ENA-78, PF4, IP10, GCP-2, MCP-1. HC14, MCP-3, I-309, MIP1$\alpha$, MIP-1$\beta$, RANTES, etc.), endothelin, enterogastrin, histamine, neurotensin, TRH, pancreatic polypeptide, galanin, etc.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
cDNA : complementary deoxyribonucleic acid
A : adenine
T : thymine
G : guanine
C : cytosine
I : inosine
R : adenine (A) or guanine (G)
Y : thymine (T) or cytosine (C)
M : adenine (A) or cytosine (C)
K : guanine (G) or thymine (T)
S : guanine (G) or cytosine (C)
W : adenine (A) or thymine (T)
B : guanine (G), guanine (G) or thymine (T)
D : adenine (A), guanine (G) or thymine (T)
V : adenine (A), guanine (G) or cytosine (C)
N : adenine (A), guanine (G), cytosine (C) or thymine (T), or unknown or other base
RNA : ribonucleic acid
mRNA : : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
BHA : benzhydrylamine
pMBHA: p-methyobenzhydrylamine
Tos p-toluenesulfonyl
Bzl : benzyl
Bom : benzyloxymethyl
Boc t-butyloxycarbonyl
DCM : dichloromethane
HOBt : 1-hydroxybenztriazole
DCC : N,N'-dicyclohexylcarbodiimide
TFA : trifluoroacetic acid
DIEA : diisopropylethylamine
Gly : glycine
Ala : alanine
Val : valine
Leu : leucine
Ile : isoleucine
Ser : serine
Thr : threonine

Cys  : cysteine
Met :  methionine
Glu  glutamic acid
Asp  aspartic acid
Lys  lysine
Arg :  arginine
His  histidine
Phe :  phenylalanine
Tyr :  tyrosine
Trp :  tryptophan
Pro :  proline
Asn :  asparagine
Gln :  glutamine
pGlu :  pyroglutamic acid

The sequence identification numbers in the sequence listing of the specification indicates the following sequence, respectively.

[SEQ ID NO:1]

This shows the amino acid sequence of the polypeptide (human type) of the present invention, obtained in Example 1 which will be later described.

[SEQ ID NO:2]

This shows the base sequence of DNA encoding the polypeptide of the present invention shown by SEQ ID NO:1.

[SEQ ID NO:3]

This shows the base sequence of primer F5 used in Example 1 later described.

[SEQ ID NO:4]

This shows the base sequence of primer F6 used in Example 1 later described.

[SEQ ID NO:5]

This shows the base sequence of primer F1 used in Example 1 later described.

[SEQ ID NO:6]

This shows the base sequence of primer R5 used in Example 1 later described.

[SEQ ID NO:7]

This shows the base sequence of primer hR1 used in Example 3 later described.

[SEQ ID NO:8]

This shows the amino acid sequence of the polypeptide (human type) of the present invention obtained in Example 3 later described.

[SEQ ID NO:9]

This shows the base sequence of DNA encoding the polypeptide of the present invention represented by SEQ ID NO:8.

[SEQ ID NO:10]

This shows the base sequence of primer bF6 used in Example 4 later described.

[SEQ ID NO:11]

This shows the base sequence of primer bF7 used in Example 4 later described.

[SEQ ID NO:12]

This shows the base sequence of primer bR6 used in Example 4 later described.

[SEQ ID NO:13]

This shows the base sequence of primer bR7 used in Example 4 later described.

[SEQ ID NO:14]

This shows the amino acid sequence of the polypeptide (bovine type) obtained in Example4, which will be later described.

[SEQ ID NO:15]

This shows the base sequence of the DNA encoding the polypeptide of the present invention shown by SEQ ID NO:14.

[SEQ ID NO:16]

This shows the base sequence of primer rLPR1 used in Example 5, which will be later described.

[SEQ ID NO:17]

This shows the base sequence of primer rLPF1 employed in Example 5, which will be later described.

[SEQ ID NO:18]

This shows the amino acid sequence of the polypeptide (rat type) of the present invention obtained in Example 5, which will be later described (before cloning).

[SEQ ID NO:19]

This shows the base sequence of the DNA encoding the polypeptide of the present invention shown by SEQ ID NO:18.

[SEQ ID NO:20]

This shows the base sequence encoding RFGK sequence.

[SEQ ID NO:21]

This shows the base sequence encoding RFGR sequence.

[SEQ ID NO:22]

This shows the base sequence encoding RSGK sequence.

[SEQ ID NO:23]

This shows the base sequence encoding RSGR sequence.

[SEQ ID NO:24]

This shows the base sequence encoding RLGK sequence.

[SEQ ID NO:25]

This shows the base sequence encoding RLGR sequence.

[SEQ ID NO:26]

This shows the base sequence of primer FF2 used in Example 6, which will be later described.

[SEQ ID NO:27]

This shows the base sequence of primer rR4 used in Example 6, which will be later described.

[SEQ ID NO:28]

This shows the base sequence of primer mF1 used in Example 6, which will be later described.

[SEQ ID NO:29]

This shows the base sequence of primer mF3 used in Example 6, which will be later described.

[SEQ ID NO:30]

This shows the base sequence of primer mR1 used in Example 6, which will be later described.

[SEQ ID NO:31]

This shows the base sequence of primer moF used in Example 6, which will be later described.

[SEQ ID NO:32]

This shows the base sequence of primer moR used in Example 6, which will be later described.

[SEQ ID NO:33]

This shows the amino acid sequence of the polypeptide (mouse type) of the present invention obtained in Example 6, which will be later described.

[SEQ ID NO:34]

This shows the base sequence of the DNA encoding the polypeptide of the present invention bearing the amino acid sequence shown by SEQ ID NO:33.

[SEQ ID NO:35]

This shows the base sequence of primer 1 used for cloning the cDNA encoding the rat cerebellum-derived novel G protein-coupled receptor protein rOT7T022L obtained in Example 7, which will be later described.

[SEQ ID NO:36]

This shows the base sequence of primer 2 used for cloning the cDNA encoding the rat cerebellum-derived novel G protein-coupled receptor protein rOT7T022L obtained in Example 7, which will be later described.

[SEQ ID NO:37]

This shows the amino acid sequence of the rat cerebellum-derived novel G protein-coupled receptor protein rOT7T022L obtained in Example 7, which will be later described.

[SEQ ID NO:38]

This shows the base sequence of the cDNA encoding the rat cerebellum-derived novel G protein-coupled receptor protein rOT7T022L obtained in Example 7, which will be later described.

[SEQ ID NO:39]

This shows the amino acid sequence of the peptide obtained in Example 7 (3), which will be later described.

[SEQ ID NO:40]

This shows the amino acid sequence of the peptide obtained in Example 7 (4), which will be later described.

[SEQ ID NO:41]

This shows the amino acid sequence of the peptide obtained in Example 7 (5), which will be later described.

[SEQ ID NO:42]

This shows the base sequence encoding the peptide bearing the amino acid sequence of the 81st (Met) to 92nd (Phe) in the amino acid sequence shown by SEQ ID NO:1.

[SEQ ID NO:43]

This shows the base sequence encoding the peptide bearing the amino acid sequence of the 101st (Ser) to 112nd (Ser) in the amino acid sequence shown by SEQ ID NO:1.

[SEQ ID NO:44]

This shows the base sequence encoding the peptide bearing the amino acid sequence of the 124th (Val) to 131st (Phe) in the amino acid sequence shown by SEQ ID NO:1.

[SEQ ID NO:45]

This shows the base sequence encoding the peptide bearing the amino acid sequence of the 1st (Met) to 92nd (Phe) in the amino acid sequence shown by SEQ ID NO:1.

[SEQ ID NO:46]

This shows the base sequence encoding the peptide bearing the amino acid sequence of the 1st (Met) to 112nd (Ser) in the amino acid sequence shown by SEQ ID NO:1.

[SEQ ID NO:47]

This shows the base sequence encoding the peptide bearing the amino acid sequence of the 1st (Met) to 131st (Phe) in the amino acid sequence shown by SEQ ID NO:1.

[SEQ ID NO:48]

This shows the base sequence of primer ratF2 used in Example 5.

[SEQ ID NO:49]

This shows the base sequence of primer ratR used in Example 5.

[SEQ ID NO:50]

This shows the amino acid sequence of the polypeptide (rat type) of the present invention obtained in Example 5, which will be later described (after cloning).

[SEQ ID NO:51]

This shows the base sequence of the DNA encoding the polypeptide of the present invention bearing the amino acid sequence shown by SEQ ID NO:50.

[SEQ ID NO:52]

This shows the base sequence of primer bFF used in Example 9.

[SEQ ID NO:53]

This shows the base sequence of primer bFR used in Example 9.

[SEQ ID NO:54]

This shows the amino acid sequence coding the protein (polypeptide) represented by h0T7T022 obtained Example 11.

[SEQ ID NO:55]

This shows the base sequence of the DNA encoding the protein (polypeptide) represented by h0T7T022 bearing the amino acid sequence shown by SEQ ID NO:54.

[SEQ ID NO:56]

This shows the base sequence of the DNA encoding the protein (polypeptide) represented by h0T7T022 bearing the amino acid sequence shown by SEQ ID NO:54.

[SEQ ID NO:57]

This shows the base sequence of primer 1 used in Example 11.

[SEQ ID NO:58]

This shows the base sequence of primer 2 used in Example 11.

*Escherichia coli* transformant JM109/p hRF1 obtained in Example 2 later described was on deposit with the Ministry of International Trade and Industry, Agency of Industrial Science and Technology, National Institute of Bioscience and Human Technology (NIBH) as the Accession Number FERM BP-6702 on April 14, 1999 and with Institute for Fermentation, Osaka (IFO) as the Accession Number IFO 16265 on March 5, 1999.

*Escherichia coli* transformant DH10B/pAK-rOT022L obtained in Example 7 later described was on deposit with the Ministry of International Trade and Industry, Agency of Industrial Science and Technology, National Institute of Bioscience and Human Technology (NIBH) as the Accession Number FERM BP-6558 on November 2, 1998 and with Institute for Fermentation, Osaka (IFO) as the Accession Number IFO 16211 on October 16, 1998.

*Escherichia coli* transformant JM109/pbRF2 obtained in Example 9 later described was on deposit with the Ministry of International Trade and Industry, Agency of Industrial Science and Technology, National Institute of Bioscience and Human Technology (NIBH) as the Accession Number FERM BP-6811 on August 2, 1999 and with Institute for Fermentation, Osaka (IFO) as the Accession Number IFO 16288 on June 18, 1999.

*Escherichia coli* transformant JM109/phRF2 obtained in Example 8 later described was on deposit with the Ministry of International Trade and Industry, Agency of Industrial Science and Technology, National Institute of Bioscience and Human Technology (NIBH) as the Accession Number FERM BP-6812 on August 2, 1999 and with Institute for Fermentation, Osaka (IFO) as the Accession Number IFO 16289 on June 18, 1999.

*Escherichia coli* transformant JM109/pmLP4 obtained in Example 6 later described was on deposit with the Ministry of International Trade and Industry, Agency of Industrial Science and Technology, National Institute of Bioscience and Human Technology (NIBH) as the Accession Number FERM BP-6813 on August 2, 1999 and with Institute for Fermentation, Osaka (IFO) as the Accession Number IFO 16290 on June 18, 1999.

*Escherichia coli* transformant JM109/prLPL6 obtained in Example 5 later described was on deposit with the Ministry of International Trade and Industry, Agency of Industrial Science and Technology, National Institute of Bioscience and Human Technology (NIBH) as the Accession Number FERM BP-6814 on August 2, 1999 and with Institute for Fermentation, Osaka (IFO) as the Accession Number IFO 16291 on June 18, 1999.

*Escherichia coli* transformant DH5 α/pCR2.1 - h0T022T obtained in Example 11 later described was on deposit with the Ministry of International Trade and Industry, Agency of Industrial Science and Technology, National Institute of Bioscience and Human Technology (NIBH) as the Accession Number FERM BP - 6930 on November 8, 1999 and with Institute for Fermentation, Osaka (IFO) as the Accession Number IFO 16330 on October 27, 1999.

*Escherichia coli* transformant DH5 α/pCR2.1-h0T022G obtained in Example 11 later described was on deposit with the Ministry of International Trade and Industry, Agency of Industrial Science and Technology, National Institute of Bioscience and Human Technology (NIBH) as the Accession Number FERM BP-6931 on November 8, 1999 and with Institute for Fermentation, Osaka (IFO) as the Accession Number IFO 16331 on October 27, 1999.

EXAMPLES

The present invention is described in detail below with reference to Examples, but not intended to limit the scope of the present invention thereto. The gene manipulation procedures using *Escherichia coli* were performed according to the methods described in the Molecular Cloning.

Example 1 Synthesis of cDNA from human fetal brain poly(A)$^+$RNA fraction and amplification of physiologically active peptide cDNA by RT-PCR

Oligo dT primer (Gibco BRL Inc.) was added as a primer to 1μg of human fetal brain poly(A)$^+$RNA fraction available from Clonetech and cDNA was synthesized with reverse transcriptase from Moloney murine leukemia virus (Gibco BRL Inc.) using a buffer attached thereto. After completion of the reaction, the product was extracted with phenol : chloroform (1:1) and the extract was precipitated with ethanol. The precipitate was dissolved in 30μl of TE. Using a 1μl aliquot of the thus prepared cDNA as a template, amplification was performed by PCR using the following two primers (F5 and F6).

```
F5: 5'-GGGCTGCACATAGAGACTTAATTTTAG-3' (SEQ ID NO:3)
```

```
F6: 5'-CTAGACCACCTCTATATAACTGCCCAT-3' (SEQ ID NO:4)
```

The reaction solution was composed of 20 pM each of the synthetic DNA primers (F5 and F6), 0.25 mM dNTPs, 0.5μl of Ex Taq DNA polymerase and 5μ l of a buffer attached to the enzyme, which were mixed together to make the total volume of the reaction solution 50μl. Using Thermal Cycler (Perkin-Elmer Co.) for amplification, one cycle was set to include 98°C for 10 seconds, 63°C for 20 seconds and 72°C for 40 seconds. This cycle was repeated 40 times in total.

Using a 1μl aliquot of the PCR product as a template, the following two primers (F1 and R5) were amplified by nested PCR.

```
F1: 5'-GCACATAGAGACTTAATTTTAGATTTAGAC-3' (SEQ ID NO:5)
```

```
R5: 5'-CATGCACTTTGACTGGTTTCCAGGTAT-3' (SEQ ID NO:6)
```

The reaction solution was composed of 20 pM each of the synthetic DNA primers (F1 and R5), 0.25 mM dNTPs, 0.5 μl of Ex Taq DNA polymerase and 5 μl of a buffer attached to the enzyme, which were mixed together to make the total volume of the reaction solution 50μl. Using Thermal Cycler (Perkin-Elmer Co.) for amplification, one cycle was set to include 98°C for 10 seconds, 60°C for 20 seconds and 72°C for 40 seconds. This cycle was repeated 40 times in total. The amplification product was confirmed by 1.2% agarose electrophoresis and ethidium bromide staining.

Example 2 Subcloning of the PCR products into plasmid vectors and selection of novel physiologically active peptide candidate clone by decoding base sequence of the inserted cDNA region

The PCR products obtained after the PCR procedure in Example 1 were separated by using a 1.2% agarose gel. After DNA fragments were proven to be amplified to the desired size, the DNAs were recovered using Quiagen PCR purification kit (Quiagen). According to the protocol attached to TA Cloning Kit (Invitrogen Co.), the recovered DNAs were subcloned to plasmid vector pCR™2.1. The recombinant vectors were introduced into *Escherichia coli* JM109 competent cells (Takara Shuzo Co., Ltd.) for transformation. Then, the resulting transformant clones bearing a cDNA-inserted fragment were selected in an LB agar culture medium supplemented with ampicillin, IPTG and X-gal. Only transformant clones that showed white color were picked up with a sterilized toothpick to obtain transformant *Escherichia coli* JM109/phRF1.

After the individual clones were cultured overnight in an LB culture medium containing ampicillin, the clones were treated with an automated plasmid extracting machine (Kurabo Co., Ltd.) to prepare plasmid DNAs. An aliquot of the DNAs thus prepared was cleaved by EcoRI to confirm the size of the cDNA fragment inserted. An aliquot of the remaining DNAs was further treated with RNase, extracted with phenol/chloroform followed by concentrating the aliquot through ethanol precipitation. Sequencing was carried out by using DyeDeoxy Terminator Cycle Sequencing Kit (ABI Inc.), the DNAs were decoded using an automated fluorescent sequencer. The data of the base sequences obtained were read by DNASIS (Hitachi System Engineering Co., Ltd.). The base sequence determined is shown in FIG. 1.

The base sequence thus determined was subjected to homology retrieval and sequence analysis based on FIG. 1. The results reveal that the novel physiologically active peptide was encoded by the cDNA fragment inserted in the plasmid of the transformant *Escherichia coli* JM109/phRF1.

Example 3 Acquisition of splicing variant of the physiologically active peptide cDNA from human fetal brain cDNA

Using as a template 1 ml of the human fetal brain cDNA prepared in Example 1, amplification was performed by PCR using the following two primers (F5 and hR1).

```
F5:   5'-GGGCTGCACATAGAGACTTAATTTTAG-3'  (SEQ ID NO:3)


hR1:  5'-CAGCTTTAGGGACAGGCTCCAGGTTTC-3'  (SEQ ID NO:7)
```

The reaction solution was composed of 20 pM each of the synthetic DNA primers (F5 and hR1), 0.25 mM dNTPs, 0.5 ml of Ex Taq DNA polymerase and a buffer attached to the enzyme, which were mixed together to make the total volume of the reaction solution 50 ml. Using Thermal Cycler (Perkin-Elmer Co.) for amplification, one cycle was set to include 98°C for 10 seconds, 65°C for 20 seconds and 72°C for 20 seconds. This cycle was repeated 40 times in total. The amplification product was confirmed by 1.2% agarose electrophoresis and ethidium bromide staining. After the PCR product was proven to be amplified, the reaction product was purified using QUIA Quick PCR Purification Kit (Quiagen), followed by sequencing. The sequencing reaction was conducted using BigDye Deoxy Terminator Cycle Sequence Kit (ABI Inc.). The DNAs were decoded using an automated fluorescent sequencer (ABI377). The data of the base sequences obtained were read by DNASIS (Hitachi System Engineering Co., Ltd.). As a result, cDNA with the 3' terminus different from the cDNA obtained in Example 2 was obtained. The cDNA thus obtained in this Example was found to be a splicing variant of the cDNA obtained in Example 2. The base sequence determined (SEQ ID NO: 9) and the deduced amino acid sequence (SEQ ID NO:8) are shown in FIG. 3.

Example 4 Acquisition of physiologically active peptide cDNA from bovine hypothalamus poly(A)+RNA

Bovine type physiologically active peptide cDNA was obtained from bovine hypothalamus poly(A)+RNA using Marathon cDNA Amplification Kit (Clontech). Using as a template bovine hypothalamus cDNA prepared in accordance with the manual attached to the Kit, the following four primers (bF6, bF7, bR6 and bR7) were synthesized and employed in combination with two primers AP1 and AP2 attached to the Kit to effect amplification by PCR.

```
bF6:  5'-GCCTAGAGGAGATCTAGGCTGGGAGGA-3' (SEQ ID NO:10)


bF7:  5'-GGGAGGAACATGGAAGAAGAAAGGAGC-3' (SEQ ID NO:11)


bR6:  5'-GATGGTGAATGCATGGACTGCTGGAGC-3' (SEQ ID NO:12)


bR7:  5'-TTCCTCCCAAATCTCAGTGGCAGGTTG-3' (SEQ ID NO:13)
```

For amplification of the 5' terminus (N-terminal region), a first PCR was carried out using the synthetic primers (bR6 and AP1). The reaction solution composed of 20 pM each of the synthetic DNA primers, 0.25 mM dNTPs, 0.5 ml of Klen Taq DNA polymerase and a buffer attached to the enzyme was made the total volume of the reaction solution 25 ml. Using Thermal Cycler (Perkin-Elmer Co.) for amplification, one cycle was set to include 98°C for 10 seconds and 72°C for 2 minutes, which cycle was repeated 5 times, further cycle was set to include 98°C for 10 seconds and 70°C for 2 minutes, which cycle was repeated 5 times, and another cycle set to include 98°C for 10 seconds and 68°C for 2 minutes and 30 seconds, which cycle was repeated 25 times. Then, the reaction solution of the first PCR was diluted to 10-fold, 1 ml of the aliquot was used as a template to perform a second PCR using (bF7 and AP2) as primers. The reaction solution composed of 20 pM each of the primers, 0.25 mM dNTPs, 0.5 ml of Klen Taq DNA polymerase and a buffer attached to the enzyme was made the total volume of the reaction solution 25 ml. Using a Thermal Cycler (Perkin-Elmer Co.) for amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds and 68°C for 2 minutes and 30 seconds, which cycle was repeated 35 times.

For amplification of the 3' terminus (C-terminal region), a first PCR was carried out using the synthetic primers (bF6 and AP1). The reaction solution composed of 20 pM each of the primers, 0.25 mM dNTPs, 0.5 ml of Klen Taq DNA polymerase and a buffer attached to the enzyme was made the total volume of the reaction solution 25 ml. Using Thermal Cycler (Perkin-Elmer Co.) for amplification, one cycle was set to include 98°C for 10 seconds and 72°C for 2 minutes, which cycle was repeated 5 times, and another cycle set to include 98°C for 10 seconds and 70°C for 2 minutes, which cycle was repeated 5 times and a further cycle set to include 98°C for 10 seconds and 68°C for 2 minutes and 30 seconds, which cycle was repeated 25 times. Then, the reaction solution of the first PCR was diluted to 10-fold, 1 ml of the aliquot was used as a template to perform a second PCR using (bF7 and AP2) as primers. The reaction solution composed of 20 pM each of the primers, 0.25 mM dNTPs, 0.5 ml of Klen Taq DNA polymerase and a buffer attached to the enzyme was made the total volume of the reaction solution 25 ml. Using Thermal Cycler (Perkin-Elmer Co.) for amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds and 68°C for 2 minutes and 30 seconds, which cycle was repeated 35 times. The amplification products at the 5' and 3' termini were confirmed by 1.2% agarose gel electrophoresis and ethidium bromide staining, respectively. After the PCR product was confirmed to be amplified, the reaction product was purified using QIA quick PCR purification Kit (Quiagen), followed by sequencing. The sequencing reaction was conducted using BigDye Deoxy Terminator Cycle Sequence Kit (ABI). The DNAs were decoded using an automated fluorescent sequencer (ABI377).

The data of the base sequences obtained were read by DNASIS (Hitachi System Engineering Co., Ltd.). The base sequence determined (SEQ ID NO:15) and the deduced amino acid sequence (SEQ ID NO:14) are shown in FIG. 4.

Example 5 Acquisition of physiologically active peptide cDNA from rat brain poly(A)$^+$RNA

Rat type physiologically active peptide cDNA was obtained from rat brain poly(A)$^+$RNA using Marathon cDNA Amplification Kit (Clontech). Using as a template rat brain cDNA prepared in accordance with the manual attached to the Kit, the following two primers were synthesized and employed in combination with two primers AP1 and AP2 attached to the Kit to effect amplification by PCR.

```
rLPR1: 5'-CCCTGGGGCTTCTTCTGTCTTCTATGT-3' (SEQ ID
NO:16)
```

```
rLPF1: 5'-AGCGATTCATTTTATTGACTTTAGCA-3' (SEQ ID NO:17)
```

For amplification of the 5' terminus (N-terminal region), a first PCR was carried out using the primer set of rLPR1 and AP1. The reaction solution composed of 20 pM each of the primers, 0.1 mM dNTPs, 0.25 ml of Klen Taq DNA polymerase was made the total volume of the reaction solution 25 ml with a buffer attached to the enzyme. Using Thermal Cycler (Perkin-Elmer Co.) for amplification, one cycle was set to include 98°C for 10 seconds and 72°C for 2 minutes, which cycle was repeated 5 times, another cycle set to include 98°C for 10 seconds and 70°C for 2 minutes, which cycle was repeated 5 times and then a further cycle set to include 98°C for 10 seconds and 68°C for 2 minutes and 30 seconds, which cycle was repeated 25 times. Then, a second PCR was performed using the first PCR solution as a template, the first set of primers and the same compositions of he reaction solution. For amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds (68°C for 2 minutes and 30 seconds), which cycle was repeated 38 times.

For amplification of the 3' terminus (C-terminal region), a first PCR was carried out using the primer set of rLPF1 and AP1. The composition of the reaction solution was the same as that for amplification of the 5'-terminus (N-terminal region). For amplification, one cycle was set to include 98°C for 10 seconds and 72°C for 2 minutes, which cycle was repeated 5 times, another cycle set to include 98°C for 10 seconds and 72°C for 2 minutes, which cycle was repeated 5 times and a further cycle set to include 98°C for 10 seconds, 65°C for 20 seconds and 72°C for 2 minutes, which cycle was repeated 25 times. Then, the reaction solution of the first PCR was used as a template to perform a second PCR using rLPF1 and AP2 primers. The composition of the reaction solution was the same as that for the first PCR. Using Thermal Cycler (Perkin-Elmer Co.) for amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds, 65°C for 20 seconds and 72°C for 2 minutes, which cycle was repeated 38 times. The amplification products at the 5' and 3' termini were confirmed by 1.2% agarose gel electrophoresis and ethidium bromide staining, respectively. The PCR product band was purified using QIA quick Gel Extrication Kit (Quiagen), followed by sequencing. The sequencing was conducted in a manner similar to Example 3. The base sequence determined (SEQ ID NO:19) and the deduced amino acid sequence (SEQ ID NO:18) are shown in FIG. 5. Based on the sequences, two primers were synthesized around the initiation and termination codons.

```
ratF2: 5'-AATGGAAATTATTTCATCAAAGCGATTCAT-3' (SEQ ID
NO:48)
```

```
ratR: 5'-CACCTATACTGACAGGAATGATGGCTCTCC-3' (SEQ ID
NO:49)
```

Using as a template cDNA that was synthesized from rat hypothalamus poly(A)$^+$ RNA using AMV reverse transferase (Takara Shuzo Co., Ltd.) and random 9mer (Takara Shuzo Co., Ltd.), PCR was carried out by repeating 33 times a cycle set to include 98°C for 10 seconds and 68°C for 40 seconds. Using the reaction solution as a template, PCR was carried out by repeating 38 times a cycle set to include 98°C for 10 seconds and 68°C for 1 minute to obtain the PCR product of about 690 bp. The PCR product was inserted to cloning vector pCR2.1 TOPO following the instructions attached to TA cloning kit (Invitrogen Inc.), which was then introduced into *Escherichia coli* JM109 to obtain transformant E. coli JM109/prLPL6. The base sequence was determined in a manner similar to Example 3 (SEQ ID NO:51), from which the amino acid sequence (SEQ ID NO:50) was deduced.

Example 6 Acquisition of mouse type physiologically active peptide cDNA from mouse brain poly(A)$^+$RNA by the Marathon PCR method and confirmation of its sequence

To acquire mouse type physiologically active peptide cDNA from mouse brain poly(A)$^+$RNA, firstly 1μg of mouse brain poly(A)$^+$RNA was reacted with Superscript II RNase H-reverse transcriptase (Gibco BRL) at 42°C for an hour in the presence of 2.5 pmols of oligo d(T) primer (Takara Shuzo Co., Ltd.), 0.5 mM dNTPs and 10 mM DTT to synthesize cDNA. Using the cDNA as a template, PCR was carried out using the following primers and Klen Taq DNA polymerase (Clontech), while repeating 39 times a cycle set to include 98°C for 10 seconds, 56°C for 20 seconds and 72°C for 25 seconds.

```
FF2: 5'-GACTTAATTTTAGATTTAGACAAAATGGAA-3' (SEQ ID
NO:26)


rR4: 5'-TTCTCCCAAACCTTTGGGGCAGGTT-3' (SEQ ID NO:27)
```

Further using the same primer set, PCR was carried out by repeating 39 times a cycle set to include 98°C for 10 seconds, 60°C for 20 seconds and 72°C for 25 seconds. The amplification product was confirmed by 1.2% agarose gel electrophoresis and ethidium bromide staining. The band was purified using QIA quick Gel Extrication Kit (Quiagen), followed by sequencing in a manner similar to Example 3. To obtain the 5' and 3' terminal sequences of the mouse type physiologically active peptide cDNA fragment, cDNA was synthesized from 1 μg of mouse brain poly(A)$^+$ RNA in a manner similar to Example 5, using Marathon cDNA Amplification Kit (Clontech) to use the cDNA as a template. The following three primers were synthesized and used in combination with AP1 primer attached to the kit for PCR.

```
mF1: 5'-ACAGCAAAGAAGGTGACGGAAAATACTC-3' (SEQ ID NO:28)


mF3: 5'-ATAGATGAGAAAAGAAGCCCCGCAGCAC-3' (SEQ ID NO:29)


mR1: 5'-GTGCTGCGGGGCTTCTTTTCTCATCTAT-3' (SEQ ID NO:30)
```

For amplification of the 5' terminus, a first PCR was carried out using the primer set of mR1 and AP1. For amplification of the 3' terminus (C-terminal region), a first PCR was carried out using. the primer set of mF1 and AP1. The reaction solution composed of 200 pM each of the primers, 0.1 mM dNTP, 0.25 ml of Klen Taq DNA polymerase was made the total volume of the reaction solution 25 ml with a buffer attached to the enzyme. For amplification, one cycle was set to include 98°C for 10 seconds and 72°C for 2 minutes, which cycle was repeated 5 times, another cycle set to include 98°C for 10 seconds and 70°C for 2 minutes, which cycle was repeated 5 times and a further cycle set to include 98°C for 10 seconds and 68°C for 2 minutes and 30 seconds, which cycle was repeated 25 times. Then, the reaction solution of the first PCR was used as a template to perform a second PCR. Amplification at the 5' terminus was performed using the same primer set as in the first PCR and for amplification at the 3' terminus, the same composition of the reaction solution as in the first PCR was prepared, using the primer set of mF3 and AP1. PCR was carried out by repeating 5 times a cycle set to include 98°C for 10 seconds and 72°C for 2 minutes, 5 times another cycle set to include 98°C for 10 seconds and 70°C for 2 minutes, and then 38 times a further cycle set to 98°C for 10 seconds and 68°C for 2 minutes and 30 seconds.
The amplification products at the 5' and 3' termini were confirmed by 1.2% agarose gel electrophoresis and ethidium bromide staining, respectively. The PCR product band was purified using QIA quick Gel Extrication Kit (Quiagen), followed by sequencing. The sequencing was conducted in a manner similar to Example 3.
Based on the sequences, two primers were further synthesized.

moF: 5'-TTTAGACTTAGACGAAATGGA-3' (SEQ ID NO:31)

moR: 5'-GCTCCGTAGCCTCTTGAAGTC-3' (SEQ ID NO:32)

Using as a template the above-described cDNA that was synthesized from mouse brain poly(A)$^+$ RNA using Superscript II RNase H-reverse, PCR was carried out to amplify a fragment containing mouse physiologically active peptide full-length cDNA. The reaction was carried out using Klen Taq DNA polymerase (Clontech), by repeating 35 times a cycle set to include 98°C for 10 seconds, 56°C for 20 seconds and 72°C for 15 seconds. The amplification product of about 600 bp was confirmed by 2% agarose electrophoresis and ethidium bromide staining. The band was purified using QIA quick Gel Extrication Kit (Quiagen), subcloned to cloning vector pCR2.1-TOPO (TOPO TA cloning kit, Invitrogen Inc.) and then introduced into *Escherichia* coli JM109 to obtain transformant E. coli JM109/pmLP4. The base sequence was determined in a manner similar to Example 3. The base sequence thus determined (SEQ ID NO: 34) and the deduced amino acid sequence (SEQ ID NO:33) therefrom are shown in FIG. 7.

Example 7

(1) Cloning of the cDNA encoding the rat cerebellum-derived G protein-coupled receptor protein and determination of the base sequence

Using rat cerebellum-derived cDNA as a template and two primers, namely, primer 1 (SEQ ID NO :35) and primer 2 (SEQ ID NO :36), PCR was carried out. The reaction solution in the above reaction comprised of 1/10 volume of the cDNA, 1/50 volume of Advantage cDNA Polymerase Mix (CLONTEC Inc.), 0.2 μM of primer 1 (SEQ ID NO :35), 0.2 μM of primer 2 (SEQ ID NO :36), 200 μM dNTPs and a buffer attached to the enzyme to make the final volume 50 μl. The PCR was carried out by cycles of (1) 94°C for 2 minutes, (2) then a cycle set to include 94°C for 30 seconds followed by 72°C for 2 minutes, which was repeated 3 times, (3) a cycle set to include 94°C for 30 seconds followed by 68°C for 2 minutes, which was repeated 3 times, (4) a cycle set to include 94°C for 30 seconds followed by 64°C for 30 seconds and 68°C for 2 minutes, which was repeated 30 times, and (5) finally, extension reaction at 68°C for 8 minutes. After completion of the PCR reaction, the product was subcloned to plasmid vector pCR2.1 (Invitrogen Inc.) following the instructions attached to the TA cloning kit (Invitrogen Inc.), which was then introduced into *Escherichia coli* DH5α, and the clones containing the cDNA were selected on LB agar plates containing ampicillin. The sequence of each clone was analyzed to give the cDNA sequence (SEQ ID NO :38) encoding the novel G protein-coupled receptor protein. The novel G protein-coupled receptor protein containing the amino acid sequence (SEQ ID NO :1) deduced therefrom was designated rOT7T022L.

Plasmid pAK-rOT7T022L in which the cDNA (SEQ ID NO:38) encoding the rat cerebellum-derived G protein-coupled receptor protein rOT7T022L of the present invention was subcloned was introduced into *Escherichia coli* DH10B according to a publicly known method to give transformant *Escherichia coli* DH10B/pAK-rOT7T022L.

(2) Establishment of G protein-coupled receptor protein rOT7T022L-expressing CHO cell

CHOdhfr⁻ cells of 1 x 10$^6$ were inoculated on Petri's dish of a 10 mm diameter for tissue culture followed by incubation for 24 hours. Using 20 μg of rOT7T022L-expressing vector pAK-rOT7T022L obtained (1), DNA-liposome complex was formed by the liposome method using a gene transfer kit (Gene Transfer, Nippon Gene Co.). After a fresh medium was exchanged for the medium, the DNA-liposome complex was added to the medium and incubated overnight. The medium was replaced with a fresh medium and further incubation was performed for one day followed by incubation for 2 days for transformant selection. The cells in the Petri's dish were recovered by treatment with trypsin-EDTA. By culturing again in a dilute cell density, the ratio of transformants was increased thereby to obtain stable clone of cell line CHO-rOT7T022L capable of expressing rOT7T022L in a high level.

(3) Synthesis of Met-Pro-His-Ser-Phe-Ala-Asn-Leu-Pro-Leu-Arg-Phe-NH$_2$ (SEQ ID NO:39)

Commercially available p-methyl BHA resin, 0.5 mmole, (manufactured by Applied Biosystems, now Perkin-Elmer Inc.) was charged in a reaction tank of peptide synthesizer (430A manufactured by Applied Biosystems). After swelling with DCM, first amino acid Boc-Phe was activated with the HOBt/DCC method and then introduced into p-methyl BHA resin. The resin was treated with 50% TFA/DCM to remove Boc, wherein the amino group was liberated and neutralized with DIEA. Next amino acid Boc-Arg(Tos) was condensed to the amino group by the HOBt/DCC method. Ninhydrin test was conducted to examine if any unreacted amino group was present. After it was confirmed that the reaction was completed, Boc-Leu, Boc-Pro, Boc-Leu, Boc-Asn, Boc-Ala, Boc-Phe, Boc-Ser(Bzl), Boc-His(Bom), Boc-Pro and Boc-Met were introduced in this order. The resin in which all amino acids of the sequence were introduced was treated with 50% TFA/DCM to remove the Boc groups on the resin. Thereafter the resin was dried to give 0.73 g of Met-Pro-His(Bom)-Ser(Bzl) - Phe-Ala-Asn-Leu-Pro-Leu-Arg (Tos)-Phe-pMBHA-resin.

In a Teflon-made hydrogen fluoride reactor the resin, 0.25 g, was reacted in 15 ml of hydrogen fluoride together with 5.1 g of p-cresol at 0°C for 60 minutes. After removing the hydrogen fluoride by distillation in vacuum, 100 ml of diethyl ether was added to the residue, stirred and filtrated through a glass filter followed by drying. The dried product was suspended in 50 ml of 50% acetic acid aqueous solution and stirred. After the peptide was extracted, it was separated from the resin and concentrated to about 5 ml in vacuum. The concentrate was applied to a column of Sephadex G-25 (2 x 90 cm) and developed with 50% acetic acid aqueous solution. Main fractions were collected and lyophilized. Next, the crudely purified peptide was dissolved in 1.5 ml of 5% thioglycolic acid/50% acetic acid. The solution was kept at 50°C for 12 hours to reduce the Met-oxidized peptide. The peptide was applied to a reversed phase column filled up with LiChroprep (trade name) RP-18 (manufactured by MERCK Inc.) followed by repeating purification with gradient elution using 0.1% aqueous TFA and 33% acetonitrile aqueous solution containing 0.1% TFA. Fractions eluted at the acetonitrile concentration of about 27% were collected and lyophilized to give 26 mg of white powders.

Mass spectrum (M+H)$^+$ 1428.7 (calcd. 1428.8)

Elution time on HPLC: 18.0 mins.

Column conditions:

Column: Wakosil (trademark) 5C18 (4.6 x 100 mm)
Eluant: linear density gradient elution (25 mins.) with solution A to solution B, using solution A (5% aqueous acetonitrile solution containing 0.1% TFA) and solution B (55% aqueous acetonitrile solution containing 0.1% TFA)
Flow rate: 1.0 ml/min.

(4) Synthesis of Val-Pro-Asn-Leu-Pro-Gln-Arg-Phe-NH$_2$ (SEQ ID NO:40)

As in Example 7 (3) described above, Boc-Phe, Boc-Arg(Tos), Boc-Gln, Boc-Pro, Boc-Leu, Boc-Asn, Boc-Pro and Boc-Val were condensed in this order to give 0.43 g of Boc-Val-Pro-Asn-Leu-Pro-Gln-Arg(Tos)-Phe-pMBHA-resin. In a manner similar to the above, 0.22 g of the resin was treated with hydrogen fluoride and purified by column chromatography to give 46 mg of the product as white powders.

Mass spectrum (M+H)$^+$ 969.5 (calcd. 969.6)

Elution time on HPLC: 11.8 mins.

Column conditions:

Column: Wakosil (trademark) 5C18 (4.6 x 100 mm)
Eluant: linear density gradient elution (25 mins.) with solution A to solution B, using solution A (5% aqueous acetonitrile solution containing 0.1% TFA) and solution B (55% aqueous acetonitrile solution containing 0.1% TFA)
Flow rate: 1.0 ml/min.

(5) Synthesis of Ser-Ala-Gly-Ala-Thr-Ala-Asn-Leu-Pro-Arg-Ser-NH$_2$ (SEQ ID NO:41)

As in Example 7 (3) described above, Boc-Ser(Bzl), Boc-Arg(Tos), Boc-Leu, Boc-Pro, Boc-Leu, Boc-Asn, Boc-Ala, Boc-Thr(Bzl), Boc-Ala, Boc-Gly, Boc-Ala and Boc-Ser(Bzl) were condensed in this order to give 0.62 g of Boc-Ser(Bzl)-Ala-Gly-Ala-Thr(Bzl)-Ala-Asn-Leu-Pro-Leu-Arg(Tos)-Ser(Bzl)-pMBHA-resin. In a manner similar to the above, 0.23 g of the resin was treated with hydrogen fluoride and purified by column chromatography to give 71 mg of the product as white powders.

Mass spectrum (M+H)$^+$ 1156.4 (calcd. 1156.6)

Elution time on HPLC: 11.8 mins.

Column conditions:

Column: Wakosil (trademark) 5C18 (4.6 x 100 mm)
Eluant: linear density gradient elution (25 mins.) with solution A to solution B, using solution A (5% aqueous acetonitrile solution containing 0.1% TFA) and solution B (55% aqueous acetonitrile solution containing 0.1% TFA)
Flow rate: 1.0 ml/min.

(6) Reaction of rOT7T022L (SEQ ID NO:37) and peptide MPHSFANLPLRFamide (SEQ ID NO:39) and peptide VPNLPQRFamide (SEQ ID NO:40) with site sensor

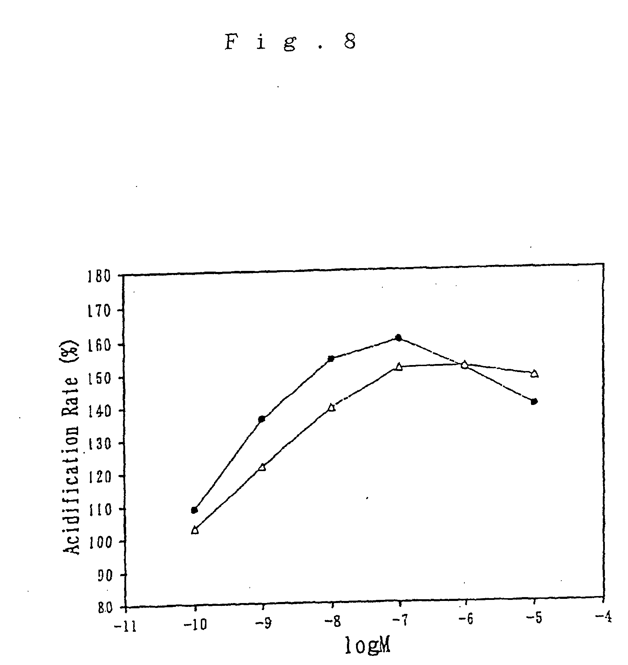

The rOT7T022L receptor-expressing CHO cells obtained in Example 7 (2) above were inoculated on capsules for site sensor in 2.7 x 10$^5$ cells/capsule. After incubation overnight, the site sensor was mounted to a work station for the site sensor. A medium for assay (low buffered RMPI1640 medium supplemented with 0.1% bovine serum albumin), that was set in the flow path of the site sensor, was supplied to the cells in a pump cycle of ON (80 seconds) and OFF (40 seconds). A rate of change in extracellular cells from 8 to 30 seconds after the pump stopped was calculated as an acidification rate. A change of the acidification rate with passage of time was monitored; when stable reading was obtained, the flow path was switched to expose each peptide to the cells for 7 minutes and 2 seconds. In the acidification rate of each well, the data for 3 cycles immediately before the peptide exposure was made 100% for standardization. Comparison of cell reactions reveals that rOT7T022L-expressing CHO cells strongly showed dose-dependent reaction with peptide MPHSFANLPLRFamide (SEQ ID NO:39) and peptide VPNLPQRFamide (SEQ ID NO:40) (FIG. 8).

Example 8 Construction of transformant bearing splicing variant cDNA for human novel physiologically active peptide candidate

The reaction product obtained after PCR in Example 3 *supra* was separated using 1.2% agarose gel. After DNA fragments were proven to be amplified to the desired size, the DNAs were recovered using Quigen PCR purification kit (Quiagen). According to the protocol attached to TA Cloning Kit (Invitrogen Co.), the recovered DNAs were subcloned to plasmid vector pCR™2.1. The recombinant vectors were introduced into *Escherichia coli* JM109 competent cells (Takara Shuzo Co.) for transformation. Then, the resulting clones bearing the cDNA-inserted fragment were selected in an LB agar culture medium supplemented with ampicillin, IPTG and X-gal. Only transformant clones that showed white color were picked up with a sterilized toothpick. Each clone was cultured overnight in an LB culture medium supplemented with ampicillin and plasmid DNA was prepared using an automated plasmid extracting machine (Kurabo Co., Ltd.). An aliquot of the DNAs thus prepared was cleaved by EcoRI to confirm the size of the cDNA fragment inserted. An aliquot of the remaining DNAs was further treated with RNase, extracted with phenol/chloroform followed by concentrating the aliquot through ethanol precipitation. The reaction for sequencing was carried out by using DyeDeoxy Terminator Cycle Sequencing Kit (ABI Inc.), the DNAs were decoded using an automated fluorescent sequencer to obtain transformant *Escherichia coli* JM109/phRF2.

Example 9 Construction of transformant bovine novel physiologically active peptide cDNA

Using as a template 1 ml of the bovine hypothalamus cDNA prepared in Example 4, amplification was performed by PCR using the following two primers (bFF and bFR).

```
bFF: 5'-TTCTAGATTTTGGACAAAATGGAAATT-3' (SEQ ID NO:52)


bFR: 5'-CGTCTTTAGGGACAGGCTCCAGATTTC-3' (SEQ ID NO:53)
```

The reaction solution was composed of 20 pM each of the synthetic primers (bFF and bFR), 0.25 mM dNTPs, 0.5 ml of Ex Taq DNA polymerase and a buffer attached to the enzyme, which were mixed together to make the total volume 50 ml. Using Thermal Cycler (Perkin-Elmer Co.) for amplification, one cycle was set to include 98°C for 10 seconds, 65°C for 20 seconds and 72°C for 20 seconds, which cycle was repeated 40 times. The amplification product was confirmed by 1.2% agarose electrophoresis and ethidium bromide staining. The reaction product obtained after PCR in Example 3 was separated using 1.2% agarose gel. After the DNA fragments were proven to be amplified to the desired size, the DNAs were recovered using Quigen PCR Purification Kit (Quiagen). According to the protocol attached to TA Cloning Kit (Invitrogen Co.), the recovered DNAs were subcloned to plasmid vector pCR™2.1. The recombinant DNA was introduced into *Escherichia coli* JM109 competent cells (Takara Shuzo Co., Ltd.) for transformation. Then, the resulting clones bearing a cDNA-inserted fragment were selected in an LB agar culture medium supplemented with ampicillin, IPTG and X-gal. Only clones that showed white color were picked up with a sterilized toothpick. Each clone was cultured overnight in an LB culture medium supplemented with ampicillin and plasmid DNA was prepared using an automated plasmid extracting machine (Kurabo Co., Ltd.). An aliquot of the DNAs thus prepared was cleaved by EcoRI to confirm the size of the cDNA fragment inserted. The DNAs prepared were further treated with RNase, extracted with phenol/chloroform and the extract was concentrated by ethanol precipitation. The reaction for sequencing was carried out by using DyeDeoxy Terminator Cycle Sequencing Kit (ABI Inc.), the DNAs were decoded using an automated fluorescent sequencer to obtain transformant *Escherichia coli* JM109/pbRF2.

Example 10 Activity of suppressing CAMP production of peptide MPHSFANLPLRFamide (SEQ ID NO:39) and peptide VPNLPQRFamide (SEQ ID NO:40) for rOT7T022L(SEQ ID NO:37)-expressing CHO cells

It was confirmed by the site sensor experiment of Example 7 (6) that peptide MPHSFANLPLRFamide (SEQ ID NO:39) and peptide VPNLPQRFamide (SEQ ID NO:40) synthesized in Example 7 (3) and (4) specifically reacted with the rOT7T022L receptor. Next, the cAMP production suppression activity of the peptides for the rOT7T022L-expressing CHO cells were evaluated.

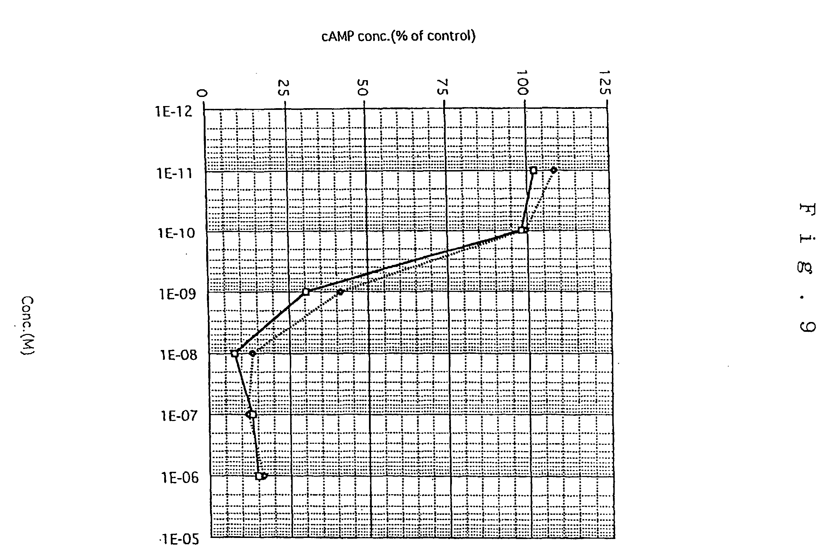

The rOT7T022L-expressing CHO cells obtained in Example 7 (2) above was inoculated in a 24-well plate in a concentration of $1.0 \times 10^5$ cells/well, followed by incubation at 37°C for 2 days. After the cells were washed with Hanks' buffer (HBSS) supplemented with 0.05% BSA and 0.2 mM IBMX, the system was allowed to stand at 37°C for 30 minutes in the same buffer. Thirty minutes after, an assay buffer was prepared by adding the cells to Hanks' buffer supplemented with $10^{-6}$ M Forskolin and at the same time, the peptides described above were added thereto in various concentrations. Incubation was performed at 37°C for 30 minutes. According to the method given in cAMP EIA Kit (Amersham Inc.), the CAMP level in the cells of each well was measured 30 minutes after. As shown in FIG. 9, peptide MPHSFANLPLRFamide (SEQ ID NO:39) and peptide VPNLPQRFamide (SEQ ID NO:40) showed a potent effect of CAMP production suppression on rOT7T022L receptor-expressing CHO cells at $IC_{50}$ of 0.5 nM and 0.7 nM, respectively, indicating that the peptide concentrations were very low.

Example 11 Cloning of the cDNA encoding human hypothalamus G protein-coupled receptor protein and determination of its base sequence

Using human hypothalamus cDNA (CLONTECH Inc.) as a template and two primers: primer 1, 5'-GTCGACATGG AGGGGGAGCC CTCCCAGCCT C-3' (SEQ ID NO :57) and primer 2, 5'-ACTAGTTCAG ATATCCCAGG CTGGAATGG-3' (SEQ ID NO :58), PCR was carried out. The reaction solution in the above reaction comprised of 1/10 volume of the cDNA, which was used as a template, 1/50 volume of Advantage cDNA Polymerase Mix (CLONTECH Inc.), 0.2 µM of primer 1 (SEQ ID NO :57), 0.2 µM of primer 2 (SEQ ID NO :58), 200 µM dNTPs, 4% dimethylsulfoxide and a buffer attached to the enzyme to make the final volume 25 µl. The PCR was carried out by (1) a cycle of 94°C for 2 minutes, (2) then a cycle set to include 94°C for 20 seconds followed by 72°C for 1 minute and 30 seconds, which was repeated 3 times, (3) a cycle set to include 94°C for 20 seconds followed by 67°C for 1 minute and 30 seconds, which was repeated 3 times, (4) a cycle set to include 94°C for 20 seconds followed by 62°C for 20 seconds and 68'C for 1 minute and 30 seconds, which was repeated 38 times, and (5) finally, extension reaction at 68°C for 7 minutes. After completion of the PCR reaction, the reaction product was subcloned to plasmid vector pCR2.1 (Invitrogen Inc.) following the instructions attached to the TA cloning kit (Invitrogen Inc.), which was then introduced into *Escherichia coli* DH5α, and the clones carrying the cDNA were selected in an LB agar medium containing ampicillin. The sequence of each clone was analyzed to give the cDNA sequences (SEQ ID NO :55 and SEQ ID NO:56) encoding the novel G protein-coupled receptor protein. The two sequences are different by one base in the 597th residue but the deduced amino acid sequences are the same (SEQ ID NO:57). Novel G protein-coupled receptor protein containing the amino acid sequence was designated hOT7T022. The two transformants were named *Escherichia coli* DH5 α/ pCR2.1-hOT022T (containing cDNA shown by SEQ ID NO:55) and *Escherichia coli* DH5 α/pCR2.1-hOT022G (containing cDNA shown by SEQ ID NO:56).

Industrial Applicability

The polypeptide, receptor protein, etc. of the present invention exhibits, e.g., a nerve cell stimulating activity and thus can be employed as a pharmaceutical composition for the treatment of neuropathy. The polypeptide or receptor protein of the present invention is useful as a reagent for screening a compound that accelerates or inhibits the activities of the polypeptide or receptor protein of the present invention, or its salts. These compounds obtained by the screening are expected-to be useful as an agent for the treatment/prevention of neuropathy. Furthermore, antibodies to the polypeptide or receptor protein of the present invention can recognize the polypeptide or receptor protein of the present invention specifically and can be used for quantification of the polypeptide or receptor protein of the present invention in a test sample fluid.

**Claims**

1. A polypeptide containing the same or substantially the same amino acid sequence as the amino acid sequence represented by SEQ ID NO:1, its amide or ester, or a salt thereof.

2. A polypeptide or its amide or ester, or a salt thereof, according to claim 1, wherein substantially the same amino acid sequence is represented by SEQ ID NO:8, SEQ ID NO:14, SEQ ID NO:18, SEQ ID NO:33 or SEQ ID NO:50.

3. A partial peptide of the polypeptide according to claim 1, or its amide or ester, or a salt thereof.

4. A partial peptide or its amide or ester, or a salt thereof, according to claim 3, comprising amino acid residues 81 (Met) to 92 (Phe) of SEQ ID NO:1.

5. A partial peptide or its amide or ester, or a salt thereof, according to claim 3, comprising amino acid residues 101 (Ser) to 112 (Ser) of SEQ ID NO:1.

6. A partial peptide or its amide or ester, or a salt thereof, according to claim 3, comprising amino acid residues 124 (Val) to 131 (Phe) of SEQ ID NO:1.

7. An amide of the partial peptide of the polypeptide according to claim 1, or a salt thereof.

8. A DNA containing a DNA bearing a base sequence encoding the polypeptide of claim 1.

9. A DNA according to claim 8 having the base sequence represented by SEQ ID NO:2, SEQ ID NO:9, SEQ ID NO: 15, SEQ ID NO:19, SEQ ID NO:34 or SEQ ID NO:51.

10. A DNA containing a DNA encoding the partial peptide of claim 3.

11. A DNA according to claim 10, comprising bases 241 to 276 of the base sequence represented by SEQ ID NO:2.

12. A DNA according to claim 10, comprising bases 301 to 336 of the base sequence represented by SEQ ID NO:2.

13. A DNA according to claim 10, comprising bases 370 to 393 of the base sequence represented by SEQ ID NO:2.

14. A recombinant vector containing the DNA of claim 8 or claim 10.

15. A transformant transformed with the recombinant vector of claim 14.

16. A method for manufacturing the polypeptide or its amide or ester, or a salt thereof, according to claim 1 or the partial peptide or its amide or ester, or a salt thereof, according to claim 3, which comprises culturing said transformant of claim 15 and producing and accumulating the polypeptide of claim 1 or the partial peptide of claim 3.

17. An antibody to the polypeptide or its amide or ester, or a salt thereof, according to claim 1 or the partial peptide or its amide or ester, or a salt thereof according to claim 3.

18. A diagnostic composition comprising the DNA according to claim 8 or claim 10 or the antibody according to claim 17.

19. An antisense DNA having a complementary or substantially complementary base sequence to the DNA according to claim 8 or claim 10 and capable of suppressing expression of said DNA.

20. A composition comprising the polypeptide or its amide or ester, or a salt thereof, according to claim 1 or the partial peptide, or its amide or ester, or a salt thereof, according to claim 3.

21. A pharmaceutical composition comprising the polypeptide or its amide or ester, or a salt thereof, according to claim 1 or the partial peptide or its amide or ester, or a salt thereof, according to claim 3.

22. A method for screening a compound that accelerates or inhibits the activity of the polypeptide or its amide or ester, or a salt thereof, according to claim 1 or the partial peptide or its amide or ester, or a salt thereof, according to claim 3, which comprises using the polypeptide or its amide or ester, or a salt thereof, according to claim 1 or the partial peptide or its amide or ester, or a salt thereof, according to claim 3.

23. A method for screening according to claim 22, wherein the polypeptide or its amide or ester, or a salt thereof, according to claim 1 or the partial peptide or its amide or ester, or a salt thereof, according to claim 3 and a protein containing the same or substantially the same amino acid sequence as the amino acid sequence represented by SEQ ID NO:37, or a salt thereof, or the partial peptide or its amide or ester, or a salt thereof, are employed.

24. A kit for screening a compound that accelerates or inhibits the activity of the polypeptide or its amide or ester, or a salt thereof, according to claim 1 or the partial peptide or its amide or ester, or a salt thereof, according to claim 3, comprising the polypeptide or its amide or ester, or a salt thereof, according to claim 1, or the partial peptide or its amide or ester, or a salt thereof, according to claim 3.

25. A kit for screening according to claim 24, comprising the polypeptide or its amide or ester, or a salt thereof, according to claim 1 or the partial peptide or its amide or ester, or a salt thereof, according to claim 3 and a protein containing the same or substantially the same amino acid sequence as the amino acid sequence represented by SEQ ID NO: 37 or the partial peptide or its amide or ester, or a salt thereof.

26. A compound that accelerates or inhibits the polypeptide, or its amide or ester, or a salt thereof, according to claim 1 or the partial peptide, or its amide or ester, or a salt thereof, according to claim 3, which is obtainable using the screening method according to claim 22 or the screening kit according to claim 24.

27. A pharmaceutical composition comprising a compound that accelerates or inhibits the polypeptide, or its amide or ester, or a salt thereof, according to claim 1 or the partial peptide, or its amide or ester, or a salt thereof, according to claim 3, which is obtainable using the screening method according to claim 22 or the screening kit according to claim 24.

28. A protein or a salt thereof containing the same or substantially the same amino acid sequence as the amino acid sequence represented by SEQ ID NO:37.

29. A protein or a salt thereof according to claim 28, wherein substantially the same amino acid sequence as the amino acid sequence represented by SEQ ID NO:37 is the amino acid sequence represented by SEQ ID NO:54.

30. A partial peptide or its amide or ester, or a salt thereof, according to claim 28.

31. A DNA containing a DNA having a base sequence encoding the protein according to claim 28 or the partial peptide according to claim 30.

32. A DNA according to claim 31 having the base sequence represented by SEQ ID NO:38, SEQ ID NO:55 or SEQ ID NO:56.

33. A recombinant vector containing the DNA according to claim 31.

34. A transformant transformed with the recombinant vector according to claim 33.

35. A method for manufacturing the protein or a salt thereof according to claim 28 or the partial peptide or its amide or ester, or a salt thereof, according to claim 30, which comprises culturing the transformant according to claim 34 and producing and accumulating the protein according to claim 28 or the partial peptide according to claim 30.

36. An antibody to the protein or a salt thereof according to claim 28 or the partial peptide or its amide or ester, or a salt thereof, according to claim 30.

37. A diagnostic composition comprising the DNA according to claim 31 or the antibody according to claim 36.

38. A ligand to the protein or a salt thereof according to claim 28, which is obtainable by using the protein or a salt thereof according to claim 28 or the partial peptide or its amide or ester or, a salt thereof, according to claim 30.

39. A method for determination of a ligand to the protein or a salt thereof according to claim 28, **characterized by** using the protein or a salt thereof according to claim 28 or the partial peptide or its amide or ester, or a salt thereof, according to claim 30.

40. A method for screening a compound that alters the binding property between a ligand and the protein or a salt thereof according to claim 28, which comprises using the protein or a salt thereof according to claim 28 or the partial peptide or its amide or ester, or a salt thereof, according to claim 30.

41. A kit for screening a compound that alters the binding property between a ligand and the protein or a salt thereof according to claim 28, comprising the protein or a salt thereof according to claim 28 or the partial peptide or its amide or ester, or a salt thereof, according to claim 30.

42. A compound that alters the binding property between a ligand and the protein or a salt thereof according to claim 28, which is obtainable by using the screening method according to claim 40 or the screening kit according to claim 41.

43. A pharmaceutical composition comprising a compound that alters the binding property between a ligand and the protein or a salt thereof according to claim 28, which is obtainable by using the screening method according to claim 40 or the screening kit according to claim 41.

44. A method for quantifying the protein or a salt thereof according to claim 28, which comprises using the antibody of claim 36.

# F i g . 1

```
            9           18          27          36          45          54
5'  ATG GAA ATT ATT TCA TCA AAA CTA TTC ATT TTA TTG ACT TTA GCC ACT TCA AGC
    --- --- --- --- --- --- --- --- --- --- --- --- --- --- --- --- --- ---
    Met Glu Ile Ile Ser Ser Lys Leu Phe Ile Leu Leu Thr Leu Ala Thr Ser Ser

            63          72          81          90          99          108
    TTG TTA ACA TCA AAC ATT TTT TGT GCA GAT GAA TTA GTG ATG TCC AAT CTT CAC
    --- --- --- --- --- --- --- --- --- --- --- --- --- --- --- --- --- ---
    Leu Leu Thr Ser Asn Ile Phe Cys Ala Asp Glu Leu Val Met Ser Asn Leu His

            117         126         135         144         153         162
    AGC AAA GAA AAT TAT GAC AAA TAT TCT GAG CCT AGA GGA TAC CCA AAA GGG GAA
    --- --- --- --- --- --- --- --- --- --- --- --- --- --- --- --- --- ---
    Ser Lys Glu Asn Tyr Asp Lys Tyr Ser Glu Pro Arg Gly Tyr Pro Lys Gly Glu

            171         180         189         198         207         216
    AGA AGC CTC AAT TTT GAG GAA TTA AAA GAT TGG GGA CCA AAA AAT GTT ATT AAG
    --- --- --- --- --- --- --- --- --- --- --- --- --- --- --- --- --- ---
    Arg Ser Leu Asn Phe Glu Glu Leu Lys Asp Trp Gly Pro Lys Asn Val Ile Lys

            225         234         243         252         261         270
    ATG AGT ACA CCT GCA GTC AAT AAA ATG CCA CAC TCC TTC GCC AAC TTG CCA TTG
    --- --- --- --- --- --- --- --- --- --- --- --- --- --- --- --- --- ---
    Met Ser Thr Pro Ala Val Asn Lys Met Pro His Ser Phe Ala Asn Leu Pro Leu

            279         288         297         306         315         324
    AGA TTT GGG AGG AAC GTT CAA GAA GAA AGA AGT GCT GGA GCA ACA GCC AAC CTG
    --- --- --- --- --- --- --- --- --- --- --- --- --- --- --- --- --- ---
    Arg Phe Gly Arg Asn Val Gln Glu Glu Arg Ser Ala Gly Ala Thr Ala Asn Leu

            333         342         351         360         369         378
    CCT CTG AGA TCT GGA AGA AAT ATG GAG GTG AGC CTC GTG AGA CGT GTT CCT AAC
    --- --- --- --- --- --- --- --- --- --- --- --- --- --- --- --- --- ---
    Pro Leu Arg Ser Gly Arg Asn Met Glu Val Ser Leu Val Arg Arg Val Pro Asn

            387         396         405         414         423         432
    CTG CCC CAA AGG TTT GGG AGA ACA ACA ACA GCC AAA AGT GTC TGC AGG ATG CTG
    --- --- --- --- --- --- --- --- --- --- --- --- --- --- --- --- --- ---
    Leu Pro Gln Arg Phe Gly Arg Thr Thr Thr Ala Lys Ser Val Cys Arg Met Leu

            441         450         459         468         477         486
    AGT GAT TTG TGT CAA GGA TCC ATG CAT TCA CCA TGT GCC AAT GAC TTA TTT TAC
    --- --- --- --- --- --- --- --- --- --- --- --- --- --- --- --- --- ---
    Ser Asp Leu Cys Gln Gly Ser Met His Ser Pro Cys Ala Asn Asp Leu Phe Tyr

            495         504         513         522         531         540
    TCC ATG ACC TGC CAG CAC CAA GAA ATC CAG AAT CCC GAT CAA AAA CAG TCA AGG
    --- --- --- --- --- --- --- --- --- --- --- --- --- --- --- --- --- ---
    Ser Met Thr Cys Gln His Gln Glu Ile Gln Asn Pro Asp Gln Lys Gln Ser Arg


    TAA 3'
    ---
    ***
```

69

Fig. 2

# F i g . 3

```
           9            18            27            36           45            54
5'  ATG GAA ATT ATT TCA TCA AAA CTA TTC ATT TTA TTG ACT TTA GCC ACT TCA AGC
    --- --- --- --- --- --- --- --- --- --- --- --- --- --- --- --- --- ---
    Met Glu Ile Ile Ser Ser Lys Leu Phe Ile Leu Leu Thr Leu Ala Thr Ser Ser

           63           72            81            90           99           108
    TTG TTA ACA TCA AAC ATT TTT TGT GCA GAT GAA TTA GTG ATG TCC AAT CTT CAC
    --- --- --- --- --- --- --- --- --- --- --- --- --- --- --- --- --- ---
    Leu Leu Thr Ser Asn Ile Phe Cys Ala Asp Glu Leu Val Met Ser Asn Leu His

          117          126           135           144          153           162
    AGC AAA GAA AAT TAT GAC AAA TAT TCT GAG CCT AGA GGA TAC CCA AAA GGG GAA
    --- --- --- --- --- --- --- --- --- --- --- --- --- --- --- --- --- ---
    Ser Lys Glu Asn Tyr Asp Lys Tyr Ser Glu Pro Arg Gly Tyr Pro Lys Gly Glu

          171          180           189           198          207           216
    AGA AGC CTC AAT TTT GAG GAA TTA AAA GAT TGG GGA CCA AAA AAT GTT ATT AAG
    --- --- --- --- --- --- --- --- --- --- --- --- --- --- --- --- --- ---
    Arg Ser Leu Asn Phe Glu Glu Leu Lys Asp Trp Gly Pro Lys Asn Val Ile Lys

          225          234           243           252          261           270
    ATG AGT ACA CCT GCA GTC AAT AAA ATG CCA CAC TCC TTC GCC AAC TTG CCA TTG
    --- --- --- --- --- --- --- --- --- --- --- --- --- --- --- --- --- ---
    Met Ser Thr Pro Ala Val Asn Lys Met Pro His Ser Phe Ala Asn Leu Pro Leu

          279          288           297           306          315           324
    AGA TTT GGG AGG AAC GTT CAA GAA GAA AGA AGT GCT GGA GCA ACA GCC AAC CTG
    --- --- --- --- --- --- --- --- --- --- --- --- --- --- --- --- --- ---
    Arg Phe Gly Arg Asn Val Gln Glu Glu Arg Ser Ala Gly Ala Thr Ala Asn Leu

          333          342           351           360          369           378
    CCT CTG AGA TCT GGA AGA AAT ATG GAG GTG AGC CTC GTG AGA CGT GTT CCT AAC
    --- --- --- --- --- --- --- --- --- --- --- --- --- --- --- --- --- ---
    Pro Leu Arg Ser Gly Arg Asn Met Glu Val Ser Leu Val Arg Arg Val Pro Asn

          387          396           405           414          423           432
    CTG CCC CAA AGG TTT GGG AGA ACA ACA ACA GCC AAA AGT GTC TGC AGG ATG CTG
    --- --- --- --- --- --- --- --- --- --- --- --- --- --- --- --- --- ---
    Leu Pro Gln Arg Phe Gly Arg Thr Thr Thr Ala Lys Ser Val Cys Arg Met Leu

          441          450           459           468          477           486
    AGT GAT TTG TGT CAA GGA TCC ATG CAT TCA CCA TGT GCC AAT GAC TTA TTT TAC
    --- --- --- --- --- --- --- --- --- --- --- --- --- --- --- --- --- ---
    Ser Asp Leu Cys Gln Gly Ser Met His Ser Pro Cys Ala Asn Asp Leu Phe Tyr

          495          504           513           522          531           540
    TCC ATG ACC TGC CAG CAC CAA GAA ATC CAG AAT CCC GAT CAA AAA CAG TCA AGG
    --- --- --- --- --- --- --- --- --- --- --- --- --- --- --- --- --- ---
    Ser Met Thr Cys Gln His Gln Glu Ile Gln Asn Pro Asp Gln Lys Gln Ser Arg

          549          558           567           576          585
    AGA CTG CTA TTC AAG AAA ATA GAT GAT GCA GAA TTG AAA CAA GAA AAA TAA 3'
    --- --- --- --- --- --- --- --- --- --- --- --- --- --- --- --- ---
    Arg Leu Leu Phe Lys Lys Ile Asp Asp Ala Glu Leu Lys Gln Glu Lys ***
```

# F i g . 4

```
              9              18              27              36              45              54
5'  ATG GAA ATT ATT TCA TTA AAA CGA TTC ATT TTA TTG ATG TTA GCC ACT TCA AGC
    --- --- --- --- --- --- --- --- --- --- --- --- --- --- --- --- --- ---
    Met Glu Ile Ile Ser Leu Lys Arg Phe Ile Leu Leu Met Leu Ala Thr Ser Ser

              63              72              81              90              99             108
    TTG TTA ACA TCA AAC ATC TTC TGC ACA GAC GAA TCA AGG ATG CCC AAT CTT TAC
    --- --- --- --- --- --- --- --- --- --- --- --- --- --- --- --- --- ---
    Leu Leu Thr Ser Asn Ile Phe Cys Thr Asp Glu Ser Arg Met Pro Asn Leu Tyr

             117             126             135             144             153             162
    AGC AAA AAG AAT TAT GAC AAA TAT TCC GAG CCT AGA GGA GAT CTA GGC TGG GAG
    --- --- --- --- --- --- --- --- --- --- --- --- --- --- --- --- --- ---
    Ser Lys Lys Asn Tyr Asp Lys Tyr Ser Glu Pro Arg Gly Asp Leu Gly Trp Glu

             171             180             189             198             207             216
    AAA GAA AGA AGT CTT ACT TTT GAA GAA GTA AAA GAT TGG GCT CCA AAA ATT AAG
    --- --- --- --- --- --- --- --- --- --- --- --- --- --- --- --- --- ---
    Lys Glu Arg Ser Leu Thr Phe Glu Glu Val Lys Asp Trp Ala Pro Lys Ile Lys

             225             234             243             252             261             270
    ATG AAT AAA CCT GTA GTC AAC AAA ATG CCA CCT TCT GCA GCC AAC CTG CCA CTG
    --- --- --- --- --- --- --- --- --- --- --- --- --- --- --- --- --- ---
    Met Asn Lys Pro Val Val Asn Lys Met Pro Pro Ser Ala Ala Asn Leu Pro Leu

             279             288             297             306             315             324
    AGA TTT GGG AGG AAC ATG GAA GAA GAA AGG AGC ACT AGG GCG ATG GCC CAC CTG
    --- --- --- --- --- --- --- --- --- --- --- --- --- --- --- --- --- ---
    Arg Phe Gly Arg Asn Met Glu Glu Glu Arg Ser Thr Arg Ala Met Ala His Leu

             333             342             351             360             369             378
    CCT CTG AGA CTC GGA AAA AAT AGA GAG GAC AGC CTC TCC AGA TGG GTC CCA AAT
    --- --- --- --- --- --- --- --- --- --- --- --- --- --- --- --- --- ---
    Pro Leu Arg Leu Gly Lys Asn Arg Glu Asp Ser Leu Ser Arg Trp Val Pro Asn

             387             396             405             414             423             432
    CTG CCC CAG AGG TTT GGA AGA ACA ACA ACA GCC AAA AGC ATT ACC AAG ACC CTG
    --- --- --- --- --- --- --- --- --- --- --- --- --- --- --- --- --- ---
    Leu Pro Gln Arg Phe Gly Arg Thr Thr Thr Ala Lys Ser Ile Thr Lys Thr Leu

             441             450             459             468             477             486
    AGT AAT TTG CTC CAG CAG TCC ATG CAT TCA CCA TCT ACC AAT GGG CTA CTC TAC
    --- --- --- --- --- --- --- --- --- --- --- --- --- --- --- --- --- ---
    Ser Asn Leu Leu Gln Gln Ser Met His Ser Pro Ser Thr Asn Gly Leu Leu Tyr

             495             504             513             522             531             540
    TCC ATG GCC TGC CAG CCC CAA GAA ATC CAG AAT CCT GGT CAA AAG AAC CTA AGG
    --- --- --- --- --- --- --- --- --- --- --- --- --- --- --- --- --- ---
    Ser Met Ala Cys Gln Pro Gln Glu Ile Gln Asn Pro Gly Gln Lys Asn Leu Arg

             549             558             567             576             585
    AGA CGG GGA TTC CAG AAA ATA GAT GAT GCA GAA TTG AAA CAA GAA AAA TAA  3'
    --- --- --- --- --- --- --- --- --- --- --- --- --- --- --- --- ---
    Arg Arg Gly Phe Gln Lys Ile Asp Asp Ala Glu Leu Lys Gln Glu Lys ***
```

# F i g . 5

```
              9          18          27          36          45          54
5' ATG GAA ATT ATT TCA TCA AAG CGA TTC ATT TTA TTG ACT TTA GCA ACT TCA AGC
   --- --- --- --- --- --- --- --- --- --- --- --- --- --- --- --- --- ---
   Met Glu Ile Ile Ser Ser Lys Arg Phe Ile Leu Leu Thr Leu Ala Thr Ser Ser

              63         72          81          90          99         108
   TTC TTA ACT TCA AAC ACC CTT TGT TCA GAT GAA TTA ATG ATG CCC CAT TTT CAC
   --- --- --- --- --- --- --- --- --- --- --- --- --- --- --- --- --- ---
   Phe Leu Thr Ser Asn Thr Leu Cys Ser Asp Glu Leu Met Met Pro His Phe His

              117        126         135         144         153        162
   AGC AAA GAA GGT TAT GGA AAA TAT TAC CAG CTG AGA GGA ATC CCA AAA GGG GTA
   --- --- --- --- --- --- --- --- --- --- --- --- --- --- --- --- --- ---
   Ser Lys Glu Gly Tyr Gly Lys Tyr Tyr Gln Leu Arg Gly Ile Pro Lys Gly Val

              171        180         189         198         207        216
   AAG GAA AGA AGT GTC ACT TTT CAA GAA CTC AAA GAT TGG GGG GCA AAG AAA GAT
   --- --- --- --- --- --- --- --- --- --- --- --- --- --- --- --- --- ---
   Lys Glu Arg Ser Val Thr Phe Gln Glu Leu Lys Asp Trp Gly Ala Lys Lys Asp

              225        234         243         252         261        270
   ATT AAG ATG AGT CCA GCC CCT GCC AAC AAA GTG CCC CAC TCA GCA GCC AAC CTT
   --- --- --- --- --- --- --- --- --- --- --- --- --- --- --- --- --- ---
   Ile Lys Met Ser Pro Ala Pro Ala Asn Lys Val Pro His Ser Ala Ala Asn Leu

              279        288         297         306         315        324
   CCC CTG AGG TTT GGG AGG AAC ATA GAA GAC AGA AGA AGC CCC AGG GCA CGG GCC
   --- --- --- --- --- --- --- --- --- --- --- --- --- --- --- --- --- ---
   Pro Leu Arg Phe Gly Arg Asn Ile Glu Asp Arg Arg Ser Pro Arg Ala Arg Ala

              333        342         351         360         369        378
   AAC ATG GAG GCA GGG ACC ATG AGC CAT TTT CCC AGC CTG CCC CAA AGG TTT GGG
   --- --- --- --- --- --- --- --- --- --- --- --- --- --- --- --- --- ---
   Asn Met Glu Ala Gly Thr Met Ser His Phe Pro Ser Leu Pro Gln Arg Phe Gly

              387        396         405         414         423        432
   AGA ACA ACA GCC AGA CGC ATC ACC AAG ACA CTG GCT GGT TTG CCC CAG AAA TCC
   --- --- --- --- --- --- --- --- --- --- --- --- --- --- --- --- --- ---
   Arg Thr Thr Ala Arg Arg Ile Thr Lys Thr Leu Ala Gly Leu Pro Gln Lys Ser

              441        450         459         468         477        486
   CTG CAC TCC CTG GCC TCC AGT GAA TCG CTC TAT GCC ATG ACC CGC CAG CAT CAA
   --- --- --- --- --- --- --- --- --- --- --- --- --- --- --- --- --- ---
   Leu His Ser Leu Ala Ser Ser Glu Ser Leu Tyr Ala Met Thr Arg Gln His Gln

              495        504         513         522         531        540
   GAA ATT CAG AGT CCT GGT CAA GAG CAA CCT AGG AAA CGG GTG TTC ACG GAA ACA
   --- --- --- --- --- --- --- --- --- --- --- --- --- --- --- --- --- ---
   Glu Ile Gln Ser Pro Gly Gln Glu Gln Pro Arg Lys Arg Val Phe Thr Glu Thr

              549        558         567         576         585        594
   GAT GAT GCA GAA AGG AAA CAA GAA AAA ATA GGA AAC CTC CAG CCA GTC CTT CAA
   --- --- --- --- --- --- --- --- --- --- --- --- --- --- --- --- --- ---
   Asp Asp Ala Glu Arg Lys Gln Glu Lys Ile Gly Asn Leu Gln Pro Val Leu Gln

              603        612
   GGG GCT ATG AAG CTG TGA 3'
   --- --- --- --- --- ---
   Gly Ala Met Lys Leu ***
```

Fig. 6

```
                    10         20         30         40         50
hLPLRF.aa    1  MEIISSKLFI LLTLATSSLL TSNIFCADEI VMSNLHSKEN YDKYSEPRG-   50
bLPLRF.aa    1  MEIISLKRFI LIMLATSSLL TSNIFCIDFS RMPNLYSKKN YDKYSEPRGD   50
rLPLRF.aa    1  MEIISSKRFI LLTLATSSFL TSNTLCSDEI MMPHFHSKEG VGKYVQLRGI   50

                    60         70         80         90        100
hLPLRF.aa   51  --YPKG--ER SINFEELKDW GPKNVIKMST FAVNKMPHSF ANLPLRFGRN  100
bLPLRF.aa   51  LGWEK---ER SLTFEEVKDW APK---IKMNK EVVNKMPHSA ANLPLRFGRN  100
rLPLRF.aa   51  ---PKGVKER SMTFCELKDW GAKKDIKMSP APANKMPHSA ANLPLRFGRN  100

                   110        120        130        140        150
hLPLRF.aa  101  VQEERSAGAT ANLPLRSGRN MEVSLVRRVP NLPQRFGRTT TAKSVCRMLS  150
bLPLRF.aa  101  MEEERSTRAM AHLPLRLGKN REDSLSRWVP NLPQRFGRTT TAKSITKTLS  150
rLPLRF.aa  101  IEDRRSPRAR ANM------- -EAGTMSHFD SLPQRFGRTT -ARRITKTLA  150

                   160        170        180        190        200
hLPLRF.aa  151  DLCCGSMHSD CANLLFYSMT CQHQEIQNPD QKCSRRLLFK KIDDAELKQE  200
bLPLRF.aa  151  NLICCSMHSD STNGLLYSMA CQEQEIQNPG QKNLRRRGFD KIDDAELKQE  200
rLPLRF.aa  151  GLPCKSTHSL ASSESLYAMT RQHQEIQSPG CECFRKRMFT ETDDAERKQE  200

                   210        220        230        240        250
hLPLRF.aa  201  K*--------- ------.... .......... .......... ..........  250
bLPLRF.aa  201  K*--------- ------.... .......... .......... ..........  250
rLPLRF.aa  201  KIGNLQPVLQ GAMKL*.... .......... .......... ..........  250
```

74

# F i g . 7

```
TTTAGACTTAGACGAAATGGAAATTATTTCATTAAAACGATTCATTTTATTGACTGTG          58
                          MetGluIleIleSerLeuLysArgPheIleLeuLeuThrVal     14

GCAACTTCAAGCTTCTTAACATCAAACACCTTCTGTACAGATGAGTTCATGATGCCTCAT        118
AlaThrSerSerPheLeuThrSerAsnThrPheCysThrAspGluPheMetMetProHis         34

TTTCACAGCAAAGAAGGTGACGGAAAATACTCCCAGCTGAGAGGAATCCCAAAAGGGGAA        178
PheHisSerLysGluGlyAspGlyLysTyrSerGlnLeuArgGlyIleProLysGlyGlu         54

AAGGAAAGAAGTGTCAGTTTTCAAGAACTAAAAGATTGGGGGGCAAAGAATGTTATTAAG        238
LysGluArgSerValSerPheGlnGluLeuLysAspTrpGlyAlaLysAsnValIleLys         74

ATGAGTCCAGCCCCTGCCAACAAAGTGCCCCACTCAGCAGCCAACCTGCCCCTGAGATTT        298
MetSerProAlaProAlaAsnLysValProHisSerAlaAlaAsnLeuProLeuArgPhe         94

GGAAGGACCATAGATGAGAAAAGAAGCCCCGCAGCACGGGTCAACATGGAGGCAGGGACC        358
GlyArgThrIleAspGluLysArgSerProAlaAlaArgValAsnMetGluAlaGlyThr       114

AGGAGCCATTTCCCCAGCCTGCCCCAAAGGTTTGGGAGAACAACAGCCAGAAGCCCCAAG        418
ArgSerHisPheProSerLeuProGlnArgPheGlyArgThrThrAlaArgSerProLys       134

ACACCCGCTGATTTGCCACAGAAACCCCTGCACTCACTGGGCTCCAGCGAGTTGCTCTAC        478
ThrProAlaAspLeuProGlnLysProLeuHisSerLeuGlySerSerGluLeuLeuTyr       154

GTCATGATCTGCCAGCACCAAGAAATTCAGAGTCCTGGTGGAAAGCGAACGAGGAGAGGA        538
ValMetIleCysGlnHisGlnGluIleGlnSerProGlyGlyLysArgThrArgArgGly       174

GCGTTTGTGGAAACAGATGATGCAGAAAGGAAACCAGAAAAATAGGAAACCTCGAGCCCG       ,598
AlaPheValGluThrAspAspAlaGluArgLysProGluLys***                       188

ACTTCAAGAGGCTACGGAGC                                                 618
                                                                     188
```

F i g . 8

Fig. 9

Conc.(M)

## INTERNATIONAL SEARCH REPORT

| International application No. |
| --- |
| PCT/JP99/06283 |

**A. CLASSIFICATION OF SUBJECT MATTER**
Int.Cl$^7$   C07K 14/705, C12N 15/12, C12P 21/02, C07K 16/28,
          A61K 39/395, G01N 33/50, C07K 2/00, A61K 38/00

According to International Patent Classification (IPC) or to both national classification and IPC

**B. FIELDS SEARCHED**

Minimum documentation searched (classification system followed by classification symbols)
Int.Cl$^7$   C07K 14/705, C12N 15/12, C12P 21/02, C07K 16/28,
          A61K 39/395, G01N 33/50, C07K 2/00, A61K 38/00

Documentation searched other than minimum documentation to the extent that such documents are included in the fields searched

Electronic data base consulted during the international search (name of data base and, where practicable, search terms used)
SwissProt/PIR/GeneSeq,MEDLINE(STN),
Genbank/EMBL/DDBJ/GeneSeq,WPI(DIALOG),BIOSIS(DIALOG)

**C. DOCUMENTS CONSIDERED TO BE RELEVANT**

| Category* | Citation of document, with indication, where appropriate, of the relevant passages | Relevant to claim No. |
| --- | --- | --- |
| X | JP, 9-238686, A (Takeda Chemical Industries, Ltd.), 16 September, 1997 (16.09.97)   (Family: none) | 1-44 |
| X | WO, 97/24436, A2 (TAKEDA CHEM IND LTD), 10 July, 1997 (10.07.97) & AU, 9712084, A    & JP, 10-146192, A & EP, 870020, A2 | 1-44 |
| X | Daniela Marazziti et al.,"Molecular cloning and chromosomal localization of the mouse Gpr37 gene encoding an orphan G-protein-coupled peptide receptor expressed in brain and testis", Genomics (1998) , Vol.53 ,No.3 , p.315-324 | 1-44 |

☐ Further documents are listed in the continuation of Box C.   ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 17 February, 2000 (17.02.00) | 29 February, 2000 (29.02.00) |

| Name and mailing address of the ISA/ | Authorized officer |
| --- | --- |
| Japanese Patent Office | |
| Facsimile No. | Telephone No. |

Form PCT/ISA/210 (second sheet) (July 1992)